# EUROPÄISCHE PATENTSCHRIFT

(11) **EP 0 892 787 B1**
(45) Veröffentlichungstag und Bekanntmachung des Hinweises auf die Patenterteilung: **11.06.2003**
(21) Anmeldenummer: 97918110.4
(22) Anmeldetag: 04.04.1997
(51) Int. Cl.: C07D 239/52, C07D 239/60, C07D 239/34, C07D 239/70, C07D 405/12, A61K 31/505

(54) **NEUE ALPHA-HYDROXYSÄUREDERIVATE, IHRE HERSTELLUNG UND VERWENDUNG**
NOVEL ALPHA-HYDROXYLIC ACID DERIVATIVES, THEIR PRODUCTION AND USE
NOUVEAUX DERIVES D'ACIDE ALPHA-HYDROXYLIQUE, LEUR PREPARATION ET LEUR UTILISATION

(30) Priorität: 12.04.1996 DE 19614533
(43) Veröffentlichungstag der Anmeldung: 27.01.1999
(73) Patentinhaber: Abbott GmbH & Co. KG, 65205 Wiesbaden (DE)
(72) Erfinder: KLINGE, Dagmar, D-69120 Heidelberg (DE); AMBERG, Wilhelm, D-61381 Friedrichsdorf (DE); BAUMANN, Ernst, D-67373 Dudenhofen (DE); KLING, Andreas, D-68239 Mannheim (DE); RIECHERS, Hartmut, D-67435 Neustadt (DE); UNGER, Liliane, D-67065 Ludwigshafen (DE); RASCHACK, Manfred, D-67256 Weisenheim (DE); HERGENRÖDER, Stefan, D-55128 Mainz (DE); SCHULT, Sabine, D-67346 Speyer (DE)
(74) Vertreter: Grünecker, Kinkeldey, Stockmair & Schwanhäusser Anwaltssozietät
(86) Internationale Anmeldenummer: EP9701688
(87) Internationale Veröffentlichungsnummer: WO97038981

(56) Entgegenhaltungen:
- DE-A- 4 035 758
- DE-A- 19 533 023

## Beschreibung

Die vorliegende Erfindung betrifft neue α-Hydroxysäurederivate, deren Herstellung und Verwendung.

Endothelin ist ein aus 21 Aminosäuren aufgebautes Peptid, das von vaskulärem Endothel synthetisiert und freigesetzt wird. Endothelin existiert in drei Isoformen, ET-1, ET-2 und ET-3. Im Folgenden bezeichnet "Endothelin" oder "ET" eine oder alle Isoformen von Endothelin. Endothelin ist ein potenter Vasokonstriktor und hat einen starken Effekt auf den Gefäßtonus. Es ist bekannt, daß diese Vasokonstriktion von der Bindung von Endothelin an seinen Rezeptor verursacht wird (Nature, 332, 411-415, 1988; FEBS Letters, 231, 440-444, 1988 und Biochem. Biophys. Res. Commun., 154, 868-875, 1988).

Erhöhte oder abormale Freisetzung von Endothelin verursacht eine anhaltende Gefäßkontraktion in peripheren, renalen und zerebralen Blutgefäßen, die zu Krankheiten führen kann. Wie in der Literatur berichtet, ist Endothelin in eine Reihe von Krankheiten involviert; dazu zählen Hypertonie, Myokardinfarkt, Herzversagen, Nierenversagen, pulmonäre Hypertonie, Raynaud-Syndrom, zerebrale Vasospasmen, Atherosklerose, Schlaganfall, benigne Prostatahypertrophie und Asthma (Japan J. Hyperteusion 12, 79 (1989), J. Vascular Med. Biology 2, 207 (1990), J. Am. Med. Association 264, 2868 (1990), Nature 344, 114 (1990), N. Engl. J. Med. 322, 205 (1989). M. Engl. J. Med. 328 1732 (1993), Nephton 66, 373 (1994), Stroke 25, 904 (1994), Nature 365, 759 (1993), J. Mol. Cell. Cardist. 27, A234 (1995), Cancer Research 56, 663 (1996)).

Eine Verbindung mit der Formel A 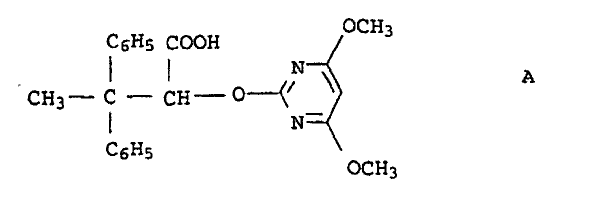 ist in der europäischen Patentanmeldung mit dem Aktenzeichen P 44 36 851.8 genannt (Seite 32, Verbindung I-28). Diese Verbindung ist jedoch nach dem in dieser Patentanmeldung genannten Herstellverfahren nicht herstellbar.

Verbindungen der Formel B, worin R³ beispielsweise Phenyl sein kann und R² und R⁴ für Wasserstoff oder C₁-C₄-Alkyl stehen können, sind in dem europäischen Patent mit der Nummer 0 347 811 B1 als herbizid wirksame Substanzen beschrieben.

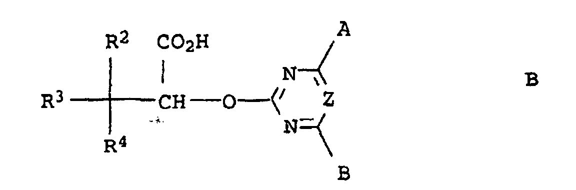

Gegenstand der Erfindung sind die α-Hydroxycarbonsäurederivate der Formel I 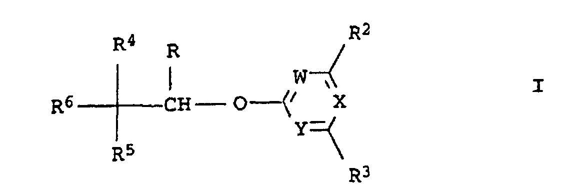 in der R eine Formylgruppe, ein Tetrazol, Nitril, eine Gruppe COOH oder einen zu COOH hydrolysierbaren Rest bedeutet. Beispielsweise steht R für eine Gruppe 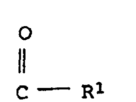 in der R¹ folgende Bedeutung hat:
a) Wasserstoff
b) eine Succinylimidoxygruppe
c) ein über ein Stickstoffatom verknüpfter 5-gliedriger Hetereoaromat wie Pyrrolyl, Pyrazolyl- Imidazolyl und Triazolyl, welcher ein bis zwei Halogenatome oder ein bis zwei C₁-C₄-Alkyl- oder ein bis zwei C₁-C₄-Alkoxygruppen tragen kann;
d) R¹ ferner eine Gruppe 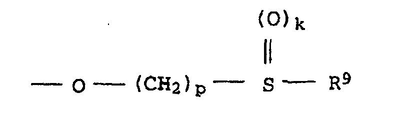 in der k die Werte 0,1 und 2, p die Werte 1,2,3 und 4 annehmen und R⁹ für
   C₁-C₄-Alkyl, C₃-C₇-Cycloalkyl, C₃-C₆-Alkenyl, C₃-C₆-Alkinyl oder gegebenenfalls substituiertes Phenyl steht, das durch einen oder mehrere, z.B. ein bis drei der folgenden Reste substituiert sein kann:
   Halogen, Nitro, Cyano, C₁-C₄-Alkyl, -C₁-C₄-Halogenalkyl, Hydroxy, C₁-C₄-Alkoxy, C₁-C₄-Alkylthio, Mercapto, Amino, C₁-C₄-Alkylamino, C₁-C₄-Dialkylamino;
e) R¹ ferner ein Rest OR¹⁰, worin R¹⁰ bedeutet:
   Wasserstoff, das Kation eines Alkalimetalls wie Lithium, Natrium, Kalium oder das Kation eines Erdalkalimetalls wie Calcium, Magnesium und Barium sowie physiologisch verträgliches Alkylammoniumion oder das Ammoniumion.
   C₃-C₈-Cycloalkyl, wie Cyclopropyl, Cyclobutyl, Cyclopentyl, Cyclohexyl, Cycloheptyl oder Cyclooctyl,
   C₁-C₈-Alkyl, insbesondere C₁-C₄-Alkyl wie Methyl, Ethyl, n-Propyl, iso-Propyl, n-Butyl, iso-Butyl, tert.-Butyl;
   CH₂-Phenyl, das durch einen oder mehrere der folgenden Reste substituiert sein kann: Halogen, Nitro, Cyano, C₁-C₄-Alkyl, C₁-C₄-Halogenalkyl, Hydroxy, C₁-C₄-Alkoxy, Mercapto, C₁-C₄-Alkylthio, Amino, C₁-C₄-Alkylamino, C₁-C₄-Dialkylamino,
   eine C₃-C₆-Alkenyl - oder eine C₃-C₆-Alkinylgruppe, wobei diese Gruppe ihrerseits ein bis fünf Halogenatome tragen können;
   R¹⁰ kann weiterhin ein Phenylrest sein, welcher ein bis fünf Halogenatome und/oder ein bis drei der folgende Reste tragen kann: Nitro, Cyano, C₁-C₄-Alkyl, C₁-C₄-Halogenalkyl, Hydroxy, C₁-C₄-Alkoxy, Mercapto, C₁-C₄-Alkylthio, Amino, C₁-C₄-Alkylamino, C₁-C₄-Dialkylamino;
   ein über ein Stickstoffatom verknüpfter 5-gliedriger Heteroaromat, enthaltend ein bis drei Stickstoffatome, welcher ein bis zwei Halogenatome und/oder ein bis zwei der folgenden Reste tragen kann: C₁-C₄-Alkyl, C₁-C₄-Halogenalkyl, C₁-C₄-Alkoxy, Phenyl, C₁-C₄-Halogenalkoxy und/oder C₁-C₄-Alkylthio. Insbesondere seien genannt: 1-Pyrazolyl, 3-Methyl-1-pyrazolyl, 4-Methyl-1-pyrazolyl, 3,5-Dimethyl-1-pyrazolyl, 3-Phenyl-1-pyrazolyl, 4-Phenyl-1-pyrazolyl, 4-Chlor-1-pyrazolyl, 4-Brom-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl, 3,4-Dichlorimidazol-1-yl;
f) R¹ ferner ein Rest 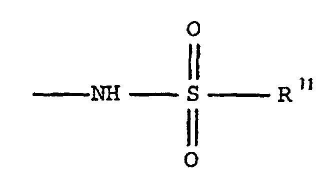 worin R¹¹ bedeutet:
   C₁-C₄-Alkyl, C₃-C₆-Alkenyl, C₃-C₆-Alkinyl, C₃-C₈-Cycloalkyl wie insbesondere vorstehend genannt, wobei diese Reste einen C₁-C₄-Alkoxy-, C₁-C₄-Alkylthio- und/oder einen Phenylrest wie oben genannt tragen können;
   Phenyl, gegebenenfalls substituiert, insbesondere wie vorstehend genannt;
g) R¹ ein Rest 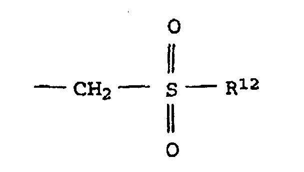 worin R¹² die gleiche Bedeutung hat wie R¹¹;
h) ferner kann R¹ bedeuten 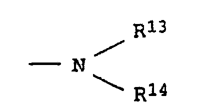 wobei R¹³ und R¹⁴ gleich oder verschieden sein können und folgende Bedeutung haben:
   Wasserstoff, C₁-C₇-Alkyl, C₃-C₇-Cycloalkyl, C₃-C₇-Alkanyl, C₃-C₇-Alkinyl, Benzyl, Phenyl, gegebenenfalls substituiert, wie oben beschrieben,
   oder R¹³ und R¹⁴ bilden gemeinsam eine zu einem Ring geschlossene, optionell substituierte, z.B. durch C₁-C₄-Alkyl substituierte C₄-C₇-Alkylenkette, die ein Heteroatom, z.B. Sauerstoff, Stickstoff oder Schwefel enthalten kann wie -(CH₂)₄-, - (CH₂)₅-, -(CH₂)₆-, -(CH₂)₇-, -(CH₂)₂-O-(CH₂)₂-, - (CH₂)₂-S-(CH₂)₂-, -CH₂-NH-(CH₂)₂-, -(CH₂)₂-NH-(CH₂)₂-.

   Die übrigen Substituenten haben folgende Bedeutung:
   - W: Stickstoff oder C-NO₂, ferner kann W für eine CH-Gruppe stehen, wenn ein oder mehrere der Substituenten R², R³, R¹⁵ und/oder R¹⁶ eine Nitrogruppe bedeuten, oder wenn X und/oder Y Stickstoff bedeuten;
   - R²: Wasserstoff, Halogen, C₁-C₄-Alkyl, C₁-C₄-Halogenalkyl, C₁-C₄-Alkoxy, C₁-C₄-Halogen-alkoxy, Hydroxy, Mercapto, C₁-C₄-Alkylthio, Nitro, Amino, C₁-C₄-Alkylamino oder C₁-C₄-Dialkylamino, Cyano, Phenyl, optional ein- bis dreifach substituiert mit Halogen, Hydroxy, Amino, Mono- oder Dialkyl (C₁-C₃)-amino, C₁-C₃-Alkyl, C₁-C₃-Alkoxy, Mercapto oder C₁-C₃-Alkylthio, Carboxyl, C₁-C₃-Alkylcarboxyl;
   oder
   ein fünf- oder sechsgliedriges Heteroaromat, enthaltend ein bis drei Stickstoffatome und/oder ein Schwefel- oder Sauerstoffatom, welcher ein bis drei Substituenten trägt, wie oben beschrieben;
   Weiterhin kann R² mit dem benachbarten Kohlenstoffatom und X einen 5- oder 6-gliedrigen Alkylen- oder Alkylidenring bilden, worin jeweils ein oder zwei Kohlenstoffatome durch ein Heteroatom wie Stickstoff, Schwefel oder Sauerstoff ersetzt sein kann und der ein- bis dreifach durch folgende Reste substituiert sein kann: Halogen, Nitro, Cyano, Hydroxy, Mercapto, C₁-C₃-Alkyl, C₁-C₃-Halogenalkyl, C₁-C₃-Alkoxy, C₁-C₃-Alkylthio, Amino, C₁-C₃-Alkylamino, C₁-C₃-Dialkylamino;
   - x: Stickstoff oder CR¹⁵, worin R¹⁵ Wasserstoff oder C₁-C₅-Alkyl, optional ein- bis zweimal substituiert mit Hydroxy oder Carboxy;
   C₁-C₆-Alkoxy, C₁-C₆-Alkylthio, Phenyl, Hydroxy, Mercapto, Benzyl, Nitro, Amino, C₁-C₄-Alkylamino, C₁-C₄-Dialkylamino, Cyano oder Carboxy bedeutet;
   ferner kann CR¹⁵ mit R² zu einem 5- oder 6-gliedrigen Ring verknüpft sein, wie oben beschrieben, oder CR¹⁵ kann mit R³ und dessen benachbartem Kohlenstoffatom einen 5- oder 6-gliedrigen Alkylen- oder Alkylidenring bilden, worin jeweils ein oder zwei Kohlenstoffatome durch Stickstoff, Sauerstoff oder Schwefel ersetzt sein kann, der 5- oder 6-gliedrige Ring kann optional ein- bis dreifach mit folgenden Resten substituiert sein:
   Halogen, Nitro, Cyano, Hydroxy, Mercapto, C₁-C₃-Alkyl, C₁-C₃-Halogenalkyl, C₁-C₃-Alkoxy, C₁-C₃-Alkylthio, Amino, C₁-C₃-Alkylamino, C₁-C₃-Dialkylamino oder Carboxy; Stickstoff im 5-Ring kann auch durch eine Formyl- oder Acetylgruppe substituiert sein;
   - R³: kann dieselbe Bedeutung haben wie R², R² und R³ können gleich oder verschieden sein; ferner kann R³ mit dem benachbarten Kohlenstoffatom und mit X einen 5- oder 6-gliedrigen Ring bilden, wie oben beschrieben; ferner kann R³ mit dem benachbarten Kohlenstoffatom und Y zusammen einen 5- oder 6-gliedrigen Alkylen- oder Alkylidenring bilden, worin jeweils ein oder zwei Kohlenstoffatome durch Stickstoff, Sauerstoff oder Schwefel ersetzt sein kann; der 5- oder 6-gliedrige Ring kann optional ein- bis dreifach mit folgenden Resten substituiert sein:
   Halogen, Nitro, Cyano, Hydroxy, Mercapto, C₁-C₃-Alkyl, C₁-C₃-Halogenalkyl, C₁-C₃-Alkoxy, C₁-C₃-Alkylthio, Amino, C₁-C₃-Alkylamino, C₁-C₃-Dialkylamino oder Carboxy; Stickstoff im 5-Ring kann auch durch eine Formyl- oder Acetylgruppe substituiert sein;
   - Y: Stickstoff oder CR¹⁶, worin R¹⁶ Wasserstoff, C₁-C₅-Alkyl, C₁-C₅-Alkoxy, C₁-C₅-Alkylthio, Nitro, Phenyl, Hydroxy, Halogen, Cyano, Amino, C₁-C₄-Alkylamino, C₁-C₄-Dialkylamino, Mercapto oder Carboxy bedeutet oder CR¹⁶ zusammen mit R³ und dessen benachbarten Kohlenstoffatom einen 5- oder 6-gliedrigen Ring bildet, wie oben beschrieben;
   - R⁴: steht für Phenyl, Naphthyl, Dihydro- oder Tetrahydronaphthyl, das durch einen oder mehrere der folgenden Reste substituiert sein kann:
   Halogen, Nitro, Cyano, Hydroxy, C₁-C₄-Alkyl, C₁-C₄-Halogenalkyl, C₁-C₄-Alkoxy, C₁-C₄-Halogenalkoxy, Phenoxy, Phenyl, C₁-C₄-Alkylthio, Amino, C₁-C₄-Alkylamino oder C₁-C₄-Dialkylamino, wobei zwei Reste an benachbarten Kohlenstoffatomen zusammen mit diesen einen über eine Alkylen- oder Alkylidengruppe verbundenen fünf- oder sechsgliedrigen Ring bilden können, bei dem ein oder mehrere Methylen oder Methylidengruppen durch Sauerstoff ersetzt sein können wie zum Beispiel: -(CH₂)₃-, -(CH₂)₄-, -CH=CH-O-, -O-CH₂-O-, -O-(CH₂)₂-O- -CH=CH-CH₂- oder -O-CH=CH-O-;
   beispielsweise kann R⁴ für folgende Reste stehen: 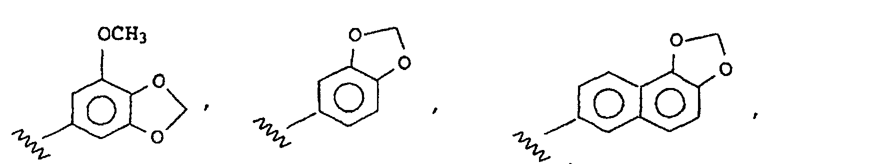 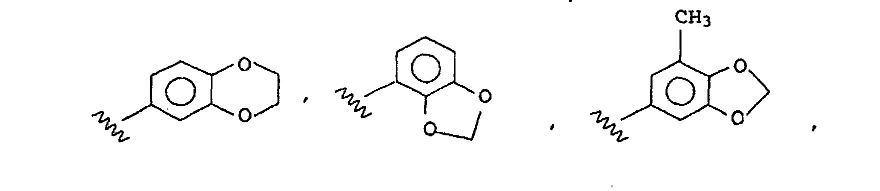 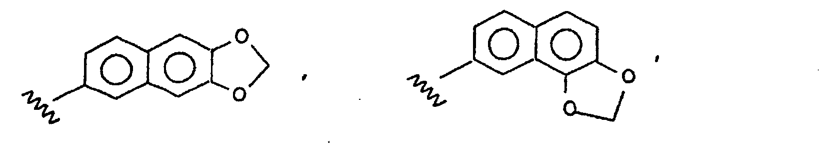 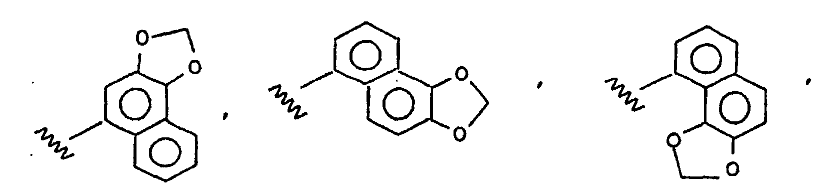
   - R⁵: kann dieselbe Bedeutung haben wie R⁴, wobei R⁴ und R⁵ gleich oder verschieden sein können;
   - R⁶: steht für Wasserstoff, C₁-C₈-Alkyl, C₂-C₈-Alkenyl oder C₃-C₈-Alkinyl, wobei diese Reste jeweils ein- oder mehrfach substituiert sein können durch: Halogen, Nitro, Cyano, C₁-C₄-Alkoxy, Hydroxy, C₁-C₄-Alkylthio, Mercapto,
   C₁-C₄-Halogenalkoxy, Carboxy, C₁-C₄-Alkylcarboxy, C₁-C₄-Alkylcarbonyl, Amino, C₁-C₄-Alkylamino, C₁-C₄-Dialkylamino oder Phenyl, bzw. Naphthyl, das seinerseits ein- oder mehrfach substituiert sein kann durch: Halogen, Nitro, Cyano, Hydroxy, C₁-C₄-Alkoxy, C₁-C₄-Alkyl, C₁-C₄-Halogenalkyl, C₁-C₄-Halogenalkoxy, Mercapto, C₁-C₄-Alkylthio, Amino, C₁-C₄-Alkylamino, C₁-C₄-Dialkylamino oder Phenoxy.
   Ferner steht R⁶ für C₁-C₄-Alkyl, das durch Phenylmethoxy oder Phenoxy substituiert ist, worin die Phenylgruppe ein- oder zweifach durch Halogen, Methyl oder Methoxy substituiert sein kann;
   weiterhin steht R⁶ auch für eine C₁-C₈-Alkyl, C₃-C₈-Alkenyl- oder C₃-C₈-Alkinylkette, die durch einen der folgenden Reste substituiert ist:
   Heteroaryl oder Heteroaryloxy, fünf- oder sechsgliedrig, enthaltend ein bis drei Stickstoffatome und/oder ein Schwefel- oder Sauerstoffatom, welcher ein bis vier Halogenatome und/oder einen bis zwei der folgenden Reste tragen kann:
   C₁-C₄-Alkyl, C₁-C₄-Halogenalkyl, C₁-C₄-Alkoxy, C₁-C₄-Halogenalkoxy, C₁-C₄-Alkylthio, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
   Phenyl, Naphthyl, das seinerseits ein- oder mehrfach substituiert sein kann durch: Halogen, Nitro, Cyano, Hydroxy, C₁-C₄-Alkoxy, C₁-C₄-Alkyl, C₁-C₄-Halogenalkyl, C₁-C₄-Halogenalkoxy, Mercapto, C₁-C₄-Alkylthio, Amino, C₁-C₄-Alkylamino, C₁-C₄-Dialkylamino oder Phenoxy;
   oder einer der folgenden Reste:

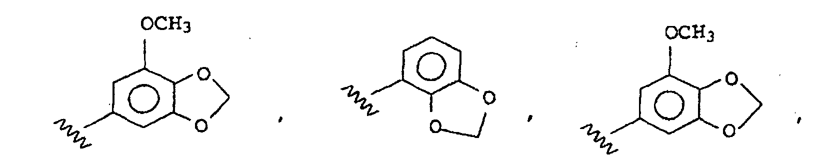

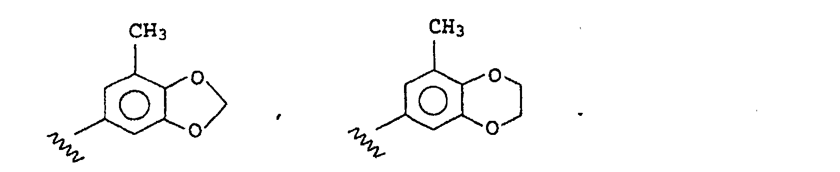

Die Verbindungen und auch die Zwischenprodukte zu ihrer Herstellung II, können ein oder mehrere asymmetrisch substituierte Kohlenstoffatome besitzen. Solche Verbindungen können als reine Enantiomere bzw. reine Diastereomere oder als deren Mischung vorliegen. Bevorzugt ist die Verwendung einer enantiomerenreinen Verbindung als Wirkstoff.

Gegenstand der Erfindung ist weiter die Verwendung der oben genannten Aminosäurederivate zur Herstellung von Arzneimitteln, insbesondere zur Herstellung von Hemmstoffen für Endothelinrezeptoren.

Die Herstellung der erfindungsgemäßen Verbindungen erfolgt durch Umsetzung eines Hydroxysäurederivats II, in der die Substituenten die angegebene Bedeutung haben, mit Verbindungen der allgemeinen Formel III, 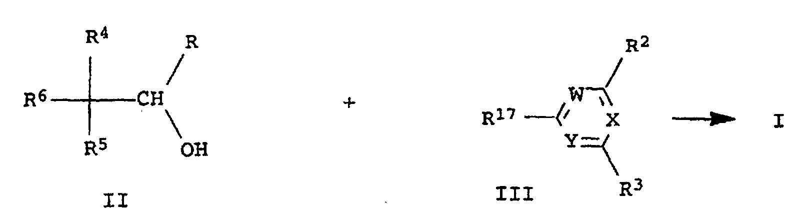 in der R¹⁷ Halogen oder R¹⁸ -SO₂- bedeutet, wobei R¹⁸ C₁-C₄-Alkyl, C₁-C₄-Halogenalkyl oder Phenyl sein kann.

Die Reaktion findet bevorzugt in einem inerten Verdünnungsmittel unter Zusatz einer geeigneten Base, d.h. eine Base, die eine Deprotonierung des Zwischenproduktes II bewirkt, in einem Temperaturbereich von Raumtemperatur bis zum Siedepunkt des Lösungsmittels statt.

Beispiele für solche Lösungsmittel beziehungsweise Verdünnungsmittel sind Wasser, aliphatische, alicyclische und aromatische Kohlenwasserstoffe, die gegebenenfalls chloriert sein können, wie zum Beispiel Hexan, Cyclohexan, Petrolether, Ligroin, Benzol, Toluol, Xylol, Methylenchlorid, Chloroform, Kohlenstofftetrachlorid, Ethylenchlorid und Trichlorethylen, Ether, wie zum Beispiel Diisopropylether, Dibutylether, Propylenoxid, Dioxan und Tetrahydrofuran, Ketone, wie zum Beispiel Aceton, Methylethylketon, Methylisopropylketon und Methylisobutylketon, Nitrile, wie zum Beispiel Acetonitril und Propionitril, Alkohole, wie zum Beispiel Methanol, Ethanol, Isopropanol, Butanol und Ethylenglycol, Ester, wie zum Beispiel Ethylacetat und Amylacetat, Säureamide, wie zum Beispiel Dimethylformamid und Dimethylacetamid, Sulfoxide und Sulfone, wie zum Beispiel Dimethylsulfoxid und Sulfolan, und Basen, wie zum Beispiel Pyridin, N-Methylpyrrolidon, cyclische Harnstoffe wie 1,3-Dimethylimidazolidin-2-on und 1,3-Dimethyl-3,4,5,6-tetrahydro-2(1H)-pyrimidinon. Die Reaktion wird dabei bevorzugt in einem Temperaturbereich zwischen 0°C und dem Siedepunkt des Lösungsmittels bzw. Lösungsmittelgemisches durchgeführt.

Als Base kann ein Alkali- oder Erdalkalimetallhydrid wie Natriumhydrid, Kaliumhydrid oder Calciumhydrid, ein Carbonat wie Alkalimetallcarbonat. z.B. Natrium- oder Kaliumcarbonat, ein Alkalioder Erdalkalimetallhydroxid wie Natrium- oder Kaliumhydroxid, eine metallorganische Verbindung wie Butyllithium oder ein Alkaliamid wie Lithiumdiisopropylamid oder Lithiumamid, oder tertiäre Amine, z.B. Triethylamin, Pyridin, 4-N,N-Dimethylaminopyridin, Imidazol oder Diazabicycloundecan dienen.

Verbindungen der Formel II, soweit sie nicht bekannt sind, sind ebenfalls Gegenstand der Erfindung. Sie können nach bekannter Art hergestellt werden.

Beispielsweisee können Verbindungen der Formel II hergestellt werden, indem ein Nitril der Formel IV durch Alkylierung mit Hilfe einer Base und einer Verbindung R⁶-K in ein Nitril V überführt wird, 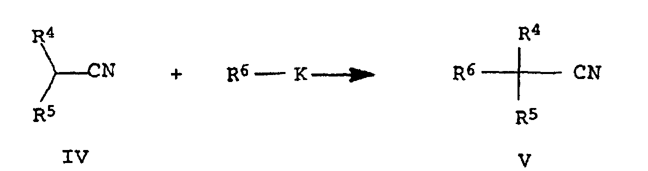 wie beispielsweise im Ca. J. of Chem. 47 (1969) 1587 ff. beschrieben, wobei K für eine Fluchtgruppe wie Halogen, Tosylat, Mesylat oder Triflat steht.

Die Nitrile V werden dann zu Aldehyden VI reduziert, wie in Synth. Comm. 19 (1989), 355 ff. oder J. Am. Chem. Soc. 107 (1985) 4577 ff. beschrieben. Als Reduktionsmittel können Metallhydride wie beispielsweise LiAlH4 oder (n-Bu)₂AlH dienen.

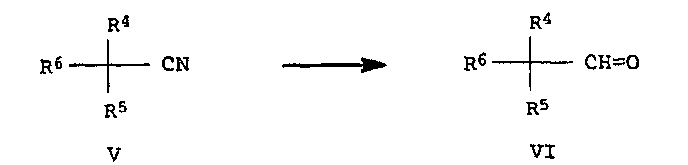

Die Aldehyde VI werden nach bekannten Methoden (wie z.B. in Chem. Pharm. Bull. 37 (1989) 2570-2 beschrieben) in die entsprechenden Cyanhydrine VII überführt: 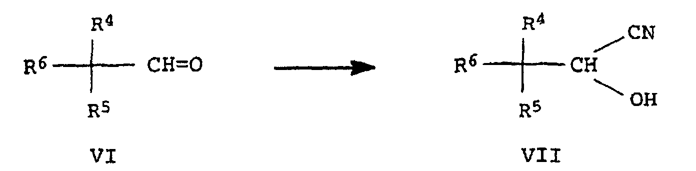 Cyanhydrine VII können hydrolytisch, beispielsweise mit wässriger HCl oder nach Pinner mit HCl-Gas in Alkohol R¹⁰OH in α-Hydroxycarbonsäurederivate II überführt werden, in denen 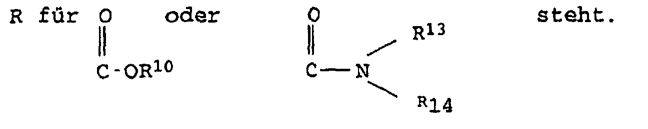 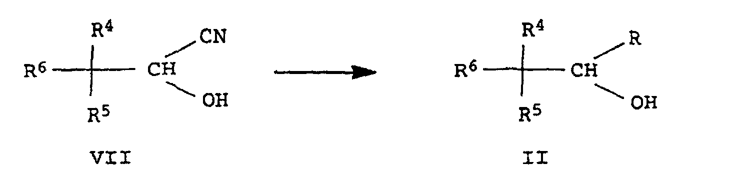 Verbindungen II können ebenfalls hergestellt werden, indem ein Aminosäurederivat VIII nach bekannten Methoden z.B. mit Natriumnitrit und wässriger Schwefelsäure diazotiert und zum Hydroxysäurederivat II hydrolysiert wird, wie z.B. in Synthesis (1987), 479-80 beschrieben.

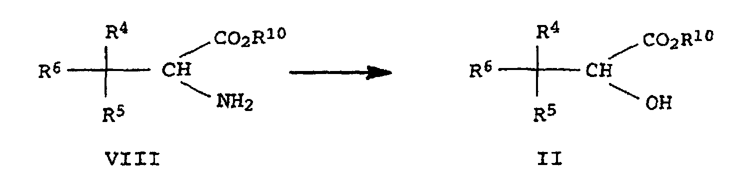

Aminosäurederivate VIII können beispielsweise in einer Strecker-Reaktion aus den Aldehyden VI hergestellt werden, z.B. nach Angew. Chem. Int. Ed. 26 (1987), 557 ff.

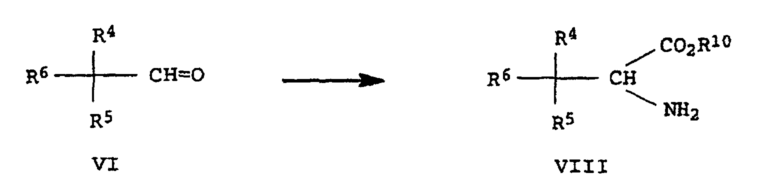

Außerdem können Verbindungen VIII hergestellt werden, indem man eine Verbindung IX mit einer Grignardverbindung X umsetzt und das Produkt XI unter Säureeinwirkung zu VIII hydrolysiert, wie in Liebigs Ann., 1977, 1174-1182 analog beschrieben: 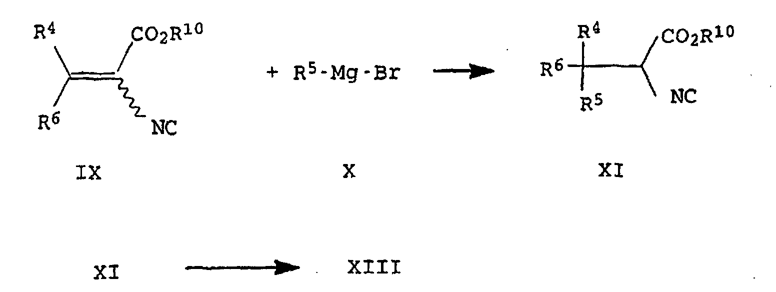 Weiterhin können Verbindungen II durch elektrophile Oxidation von Carbonsäurederivaten XII, z. B. mit Sauerstoff nach Deprotonierung, wie in Tetrahedron Letters 21 (1975), 1731-4, beschrieben, oder mit dem Davis-Reagenz 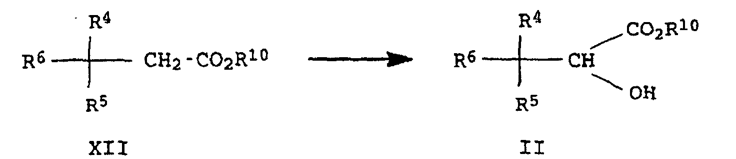 wie in J. Org. Chem. 47 (1982), 1775-77 dargestellt, synthetisiert werden.

Verbindungen XII können hergestellt werden, indem man eine geeignete Phosphonatverbindung XIII mit einer Carbonylverbindung XIV in einer Wittig-Horner-Reaktion zur ungesättigten Verbindung XV umsetzt.

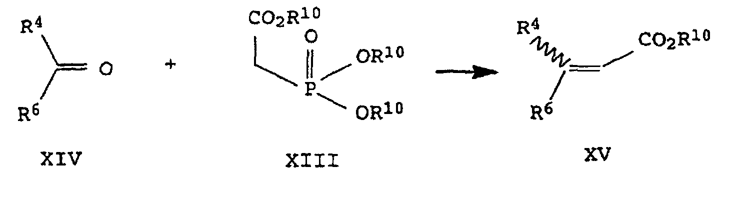

Verbindung XV kann dann nach einer Vorschrift aus Chem. Ber. 1931, 64, 1493 ff mit R⁵-H unter Zuhilfenahme eines Friedel-Crafts-Katalysators wie z.B. Aluminiumtrichlorid zum Carbonsäurederivat XVI umgesetzt werden.

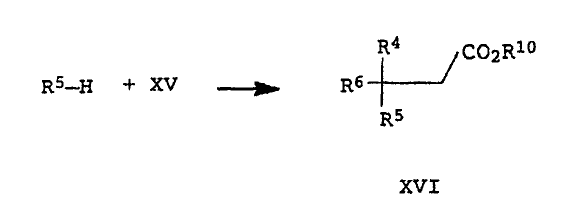

Verbindungen I können auch hergestellt werden, indem Cyanhydrine VII mit Verbindungen III zu Nitrilen XVII umgesetzt werden.

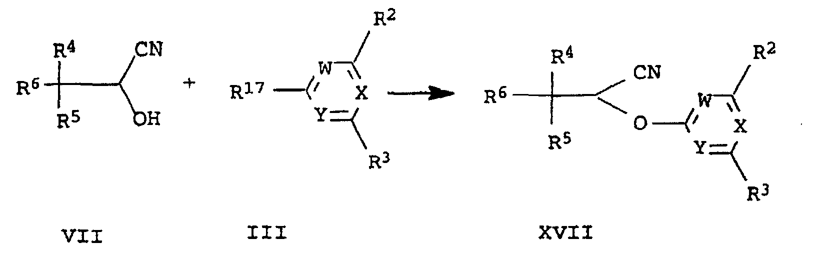

Die Reaktion findet bevorzugt in einem inerten Lösungsmittel unter Zusatz einer geeigneten Base statt, wie vorangehend beschrieben.

Verbindungen XVII lassen sich dann nach bekannter Art, beispielsweise durch Umsetzung mit Säuren wie Salzsäure oder Schwefelsäure, mit oder ohne Zusatz eines Alkohols in Verbindungen vom Typ I überführen.

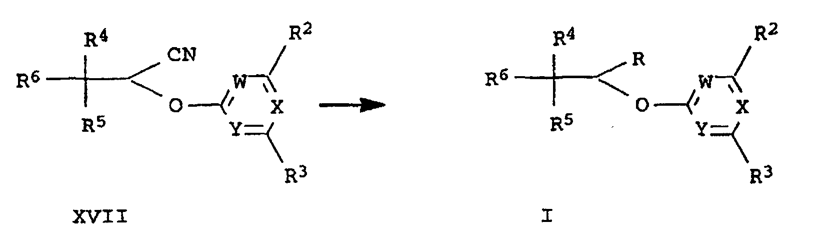

Verbindungen der Formel I können in enantiomerenreiner Form erhalten werden, indem man von enantiomeren Verbindungen II ausgeht, die durch klassische Racematspaltung oder durch enantioselektive Synthesen (wie z.B. Pure Appl.Chem., 1983,55,1799 ff; Helv.Chim.Acta, 1988,71,224 ff; J. Am. Chem. Soc, 1988, 110, 1547-1557; Chem.Eng.News, 1989,25-27) in enantiomerenreiner und gegebenenfalls diastereomerenreiner Form hergestellt werden können, und diese Verbindungen II mit III umsetzt, wie oben beschrieben. Eine andere Möglichkeit, enantiomerenreine Verbindungen der Formel I zu erhalten, ist die klassische Racematspaltung racemischer oder diastereomerer Verbindungen I mit geeigneten enantiomerenreinen Basen wie z.B. Brucin, Strychnin. Chinin, Chinidin, Chinchonidin, Chinchonin, Yohimbin, Morphin, Dehydroabietylamin, Ephedrin (-), (+), Deoxyephedrin (+), (-), threo-2-Amino-1-(p-nitrophenyl)-1,3-propandiol (+), (-), threo-2-(N,N-Dimethylamino)-1-(p-nitrophenyl)-1,3-propandiol (+), (-) threo-2-Amino-1-phenyl-1,3-propandiol (+), (-), α-Methylbenzylamin (+), (-), α-(1-Naphthyl)ethylamin (+), (-), α-(2-Naphthyl)ethylamin (+), (-), Aminomethylpinon, N,N-Dimethyl-1-phenylethylamin, N-Methyl-1-phenylethylamin, 4-Nitrophenylethylamin, 4-Chlorphenylethylamin, Pseudoephedrin, Norephedrin, Norpseudoephedrin, Aminosäurederivate und Peptidderivate.

Bevorzugt sind Verbindungen der Formel I - sowohl als reine Enantiomere bzw. reine Diastereomere oder als deren Mischung - in denen die Substituenten folgende Bedeutung haben:
- R: eine Carbonsäure, ein Carbonsäuresalz oder eine zu einer Carbonsäure hydrolysierbare Gruppe, wie oben beschrieben.
- W: Stickstoff oder C-NO₂;
- X: Stickstoff oder CR¹⁵, worin R¹⁵ Wasserstoff, Nitro C₁-C₅-Alkyl oder C₁-C₅-Alkenyl, optional substituiert mit Hydroxy, Carboxy oder Phenyl, das wiederum substituiert sein kann mit C₁-C₃-Alkyl, Hydroxy oder Carboxy; C₁-C₄-Alkoxy, C₁-C₄-Alkylthio, Hydroxy, Nitro, Amino, Cyano oder Carboxy bedeutet oder CR¹⁵ mit R² und dem benachbarten Kohlenstoffatom einen 5- oder 6-gliedrigen Alkylen- oder Alkylidenring bildet, worin ein oder zwei Kohlenstoffatome durch ein Heteroatom wie Stickstoff, Sauerstoff oder Schwefel ersetzt sein können und der ein- oder zweifach durch eine C₁-C₃-Alkyl oder C₁-C₃-Alkoxygruppe substituiert sein kann; Stickstoff im 5-gliedrigen Ring kann außerdem durch eine CHO- oder COCH₃-Gruppe substituiert sein;
- R²: Wasserstoff, Halogen, C₁-C₄-Alkyl, C₁-C₄-Halogenalkyl, C₁-C₄-Alkoxy, C₁-C₄-Alkylthio, Nitro, Amino, Methylamino, Dimethylamino oder Cyano; ferner kann R² mit dem benachbarten Kohlenstoffatom und X einen 5- oder 6-gliedrigen Ring bilden, wie oben beschrieben;
- R³: Wasserstoff, Halogen, C₁-C₄-Alkyl, C₁-C₄-Alkoxy, C₁-C₄-Halogenalkyl, C₁-C₄-Alkylthio, Nitro, Amino, Methylamino, Dimethylamino oder Cyano; ferner kann R³ mit dem benachbarten Kohlenstoffatom und Y einen 5- oder 6-gliedrigen Alkylen- oder Alkylidenring bilden, worin ein oder zwei Kohlenstoffatome durch Stickstoff, Sauerstoff oder Schwefel ersetzt sein können und der ein- oder zweifach durch eine C₁-C₃-Alkyl- oder C₁-C₃-Alkoxygruppe substituiert sein kann; Stickstoff im 5-gliedrigen Ring kann außerdem durch eine Formyl- oder Acetylgruppe substituiert sein;
- Y: Stickstoff oder CR¹⁶, worin R¹⁶ Wasserstoff, C₁-C₃-Alkyl, C₁-C₃-Alkoxy, C₁-C₃-Alkylthio, Nitro, Halogen, Cyano, Amino, Methylamino, Dimethylamino oder Carboxy bedeutet, oder CR¹⁶ zusammen mit R³ und dessen benachbarten Kohlenstoffatom einen 5- oder 6-gliedrigen Ring bildet, wie oben beschrieben;
- R⁴: steht für Phenyl oder Naphthyl, das durch einen oder mehrere der folgenden Reste substituiert sein kann:
Halogen, Hydroxy, C₁-C₄-Alkyl, C₁-C₄-Halogenalkyl, C₁-C₄-Alkoxy, C₁-C₄-Alkylthio oder Phenyl, ferner kann der Aromat ausschließlich oder zusätzlich zu den obengenannten Resten durch zwei Reste an benachbarten Kohlenstoffatomen substituiert sein, die zusammen eine 1,3-Dioxomethylen- oder eine 1,4-Dioxoethylengruppe darstellen und mit den benachbarten Kohlenstoffatomen einen 5- bzw. 6-gliedrigen Ring bilden;
außerdem können R⁴ und R⁵ Phenylgruppen sein, die orthoständig über eine direkte Bindung, eine Methylen-, Ethylen- oder Ethenylengruppe, ein Sauerstoff- oder Schwefelatom miteinander verbunden sind;
- R⁵: kann dieselbe Bedeutung haben wie R⁴, wobei R⁴ und R⁵ gleich oder verschieden sein können;
- R⁶: steht für Wasserstoff, C₁-C₆-Alkyl, C₂-C₆-Alkenyl oder C₃-C₆-Alkinyl, wobei diese Reste jeweils ein- bis dreifach substituiert sein können durch:
Halogen, Cyano, C₁-C₃-Alkoxy, Hydroxy, C₁-C₃-Alkylthio, Mercapto, C₁-C₃-Halogenalkoxy, Carboxy, C₁-C₃-Alkylcarboxy oder Phenyl bzw. Naphthyl, das ebenfalls ein- bis dreifach durch folgende Reste substituiert sein kann:
Halogen, Cyano, Hydroxy, C₁-C₃-Alkoxy, C₁-C₄-Alkyl, C₁-C₄-Halogenalkyl, C₁-C₃-Halogenalkoxy, Mercapto, C₁-C₃-Alkylthio, Phenyl oder Phenoxy, oder ausschließlich oder zusätzlich zu den obengenannten Resten können zwei Reste an benachbarten Kohlenstoffatomen zusammen eine 1,3-Dioxomethylen- oder eine 1,4-Dioxoethylengruppe darstellen, ferner steht R⁶ für C₁-C₃ Alkyl, das durch Phenylmethoxy oder Phenoxy substituiert ist, worin die Phenylgruppe durch Methyl, Methoxy oder Halogen substituiert ist.

Besonders bevorzugt sind Verbindungen der Formel I - sowohl als reine Enantiomere bzw. reine Diastereomere oder als deren Mischung - in denen die Substituenten folgende Bedeutung haben:
- R: eine Carbonsäure, ein Carbonsäuresalz oder eine zu einer Carbonsäure hydrolysierbare Gruppe, wie oben beschrieben;
- W: Stickstoff;
- X: Stickstoff oder CR¹⁵, worin R¹⁵ Wasserstoff, C₁-C₅-Alkyl oder C₁-C₅-Alkenyl, optional substituiert mit Hydroxy, Carboxy oder Phenyl, das wiederum substituiert sein kann mit C₁-C₃-Alkyl, Hydroxy oder Carboxy, C₁-C₃-Alkoxy, C₁-C₃-Alkylthio, Hydroxy, Cyano oder Carboxy bedeutet oder CR¹⁵ mitR³ und dem benachbarten Kohlenstoffatom einen 5- oder 6-gliedrigen Alkylen- oder Alkylidenring bildet, worin ein Kohlenstoffatom durch Sauerstoff ersetzt sein kann und der durch eine Methoxy- oder Methylgruppe substituiert sein kann;
beispielsweise kann der 5- oder 6-gliedrige Alkylen- und Alkylidenring folgende Strukturen bedeuten: 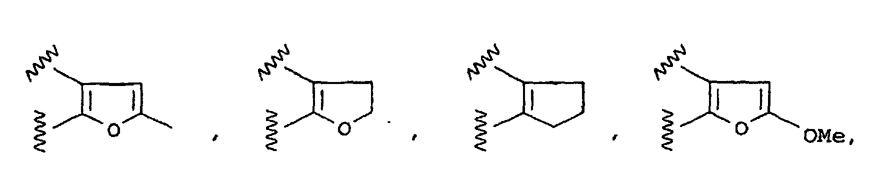 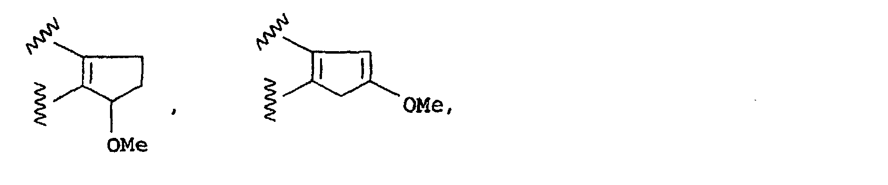
- R²: Wasserstoff, Chlor, Methyl, Ethyl, Trifluormethyl, Nitro, Methoxy, Ethoxy, Methylmercapto, Amino, Dimethylamino, Methylamino; ferner kann R² mit dem benachbarten Kohlenstoffatom und X einen 5- oder 6-gliedrigen Ring bilden, wie oben beschrieben;
- R³: Wasserstoff, Chlor, Methyl, Ethyl, Trifluormethyl, Nitro, Methoxy, Ethoxy, Methylmercapto, Amino, Methylamino oder Dimethylamino;
ferner kann R³ mit Y einen 5- oder 6-gliedrigen Alkylen- oder Alkylidenring bilden, worin ein bis zwei Kohlenstoffatome durch Stickstoff oder Sauerstoff ersetzt sein können und der durch eine Methyl- oder Methoxygruppe substituiert sein kann; Beispiele für derartige Alkylen- oder Alkylidenringe sind: 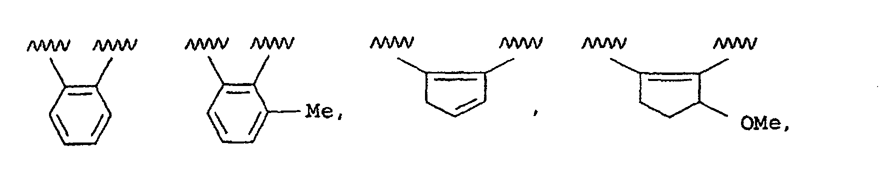 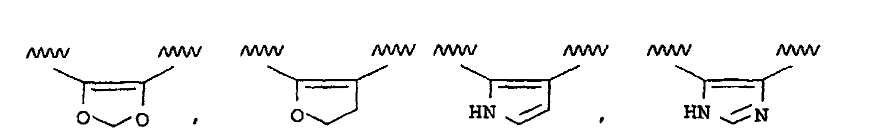
- Y: Stickstoff oder CR¹⁶, worin R¹⁶ Wasserstoff, Nitro, Methyl, Ethyl, Chlor oder Cyano bedeutet, oder CR¹⁶ mit R³ und dessen benachbarten Kohlenstoffatom einen 5- oder 6-gliedrigen Ring bildet, wie oben beschrieben;
- R⁴: steht für Phenyl, das einen oder zwei der folgenden Reste trägt: Halogen, Hydroxy, Methoxy, Ethoxy, C₁-C₃-Alkyl, Trifluormethyl, Methylmercapto, Ethylmercapto oder Phenyl; ferner können zwei Substituenten Dioxomethylengruppe darstellen, ausschließlich oder zusätzlich zu anderen Substituenten; Beispiele für derartige Gruppen, die R⁴ repräsentieren, sind: 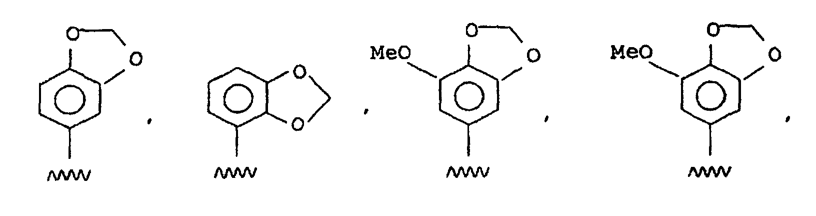 außerdem können R⁴ und R⁵ Phenylgruppe sein, die orthoständig über eine direkte Bindung, eine Methylen- oder Ethylengruppe miteinander verbunden sind;
- R⁵: kann dieselbe Bedeutung haben wie R⁴, R⁴ und R⁵ können gleich oder verschieden sein;
- R⁶: steht für Wasserstoff, C₁-C₆-Alkyl oder C₃-C₆-Alkenyl, wobei diese Reste jeweils ein- oder zweifach substituiert sein können durch:
Chlor, Cyano, Hydroxy, Carboxy, Methoxy, Ethoxy, Methylmercapto, Methylcarboxy, Phenylmethoxy, p-Methylphenylmethoxy, p-Methoxyphenylmethoxy, p-Fluorphenylmethoxy oder Phenyl, das ein- bis zweifach durch folgende Reste substituiert sein kann:
Chlor, Fluor, Cyano, Hydroxy, Methoxy, Ethoxy, Trifluormethyl, Trifluormethoxy, Methylmercapto, Phenyl oder Phenoxy, oder ausschließlich oder zusätzlich zu den obengenannten Resten können zwei Reste an benachbarten Kohlenstoffatomen eine 1,3-Dioxomethylengruppe darstellen.

Die Verbindungen der vorliegenden Erfindung bieten ein neues therapeutisches Potential für die Behandlung von Hypertonie, pulmonalem Hochdruck, Myokardinfarkt, Angina Pectoris, akutem Nierenversagen, Niereninsuffizienz, zerebralen Vasospasmen, zerebraler Ischämie, Subarachnoidalblutungen, Migräne, Asthma, Atherosklerose, endotoxischem Schock, Endotoxin-induziertem Organversagen, intravaskulärer Koagulation, Restenose nach Angioplastie, benigne Prostata-Hyperplasie, ischämisches und durch Intoxikation verursachtes Nierenversagen bzw. Hypertonie sowie von Krebserkrankungen, insbesondere Prostatakrebs und Hautkrebs.

Die gute Wirkung der Verbindungen läßt sich in folgenden Versuchen zeigen:

### Rezeptorbindungsstudien

Für Bindungsstudien wurden klonierte humane ET_{A}-Rezeptorexprimierende CHO-Zellen und Meerschweinchen-Kleinhirnmembranen mit > 60 % ET_{B}- im Vergleich zu ET_{A}-Rezeptoren eingesetzt.

### Membranpräparation

Die ET_{A}-Rezeptor-exprimierenden CHO-Zellen wurden in F₁₂-Medium mit 10 % fötalem Kälberserum, 1 % Glutamin, 100 E/ml Penicillin und 0,2 % Streptomycin (Gibco BRL, Gaithersburg, MD, USA) vermehrt. Nach 48 h wurden die Zellen mit PBS gewaschen und mit 0,05 % trypsinhaltiger PBS 5 min inkubiert. Danach wurde mit F₁₂-Medium neutralisiert und die Zellen durch Zentrifugation bei 300 x g gesammelt. Zur Lyse der Zellen wurde kurz das Pellet mit Lysispuffer (5 mM Tris-HCl, pH 7,4 mit 10 % Glycerin) gewaschen und danach in einer Konzentration von 10⁷-Zellen/ml Lysispuffer 30 min bei 4°C inkubiert. Die Membranen wurden bei 20.000 x g 10 min zentrifugiert und das Pellet in flüssigem Stickstoff gelagert.

Meerschweinchenkleinhirne wurden im Potter-Elvejhem-Homogenisator homogenisiert und durch differentielle Zentrifugation 10 min bei 1.000 x g und wiederholte Zentrifugation des Überstandes 10 min bei 20.000 x g gewonnen.

### Bindungstests

Für den ET_{A}- und ET_{B}-Rezeptorbindungstest wurden die Membranen in Inkubationspuffer (50 mM Tris-HCl, pH 7,4 mit 5 mM MnCl₂, 40 µg/ml Bacitracin und 0,2 % BSA) in einer Konzentration von 50 µg Protein pro Testansatz suspendiert und bei 25°C mit 25 pM [125J] -ET₁ (ET_{A}-Rezeptortest) oder 25 pM [125J]-RZ₃ (ET_{B}-Rezeptortest) in Anwesenheit und Abwesenheit von Testsubstanz inkubiert. Die unspezifische Bindung wurde mit 10⁻⁷ M ET₁ bestimmt. Nach 30 min wurde der freie und der gebundene Radioligand durch Filtration über GF/B Glasfaserfilter (Whatman, England) an einem Skatron-Zellsammler (Skatron, Lier, Norwegen) getrennt und die Filter mit eiskaltem Tris-HCl-Puffer, pH 7,4 mit 0,2 % BSA gewaschen. Die auf den Filtern gesammelte Radioaktivität wurde mit einem Packard 2200 CA Flüssigkeitszintillationszähler quantifiziert.

### Funktionelles in vitro-Testsystem für die Suche nach Endothelinrezeptor (Subtyp A)-Antagonisten

Dieses Testsystem ist ein funktioneller, auf Zellen basierender Test für Endothelinrezeptoren. Bestimmte Zellen zeigen, wenn sie mit Endothelin 1 (ET1) stimuliert werden, einen Anstieg der intrazellulären Calciumkonzentration. Dieser Anstieg kann in intakten Zellen, die mit Calcium-sensitiven Farbstoffen beladen wurden, gemessen werden.

Aus Ratten isolierte 1-Fibroblasten, bei denen ein endogener Endothelinrezeptor vom A-Subtyp nachgewiesen wurde, wurden mit dem Fluoreszenzfarbstoff Fura 2-an wie folgt beladen: Nach Trypsinierung wurden die Zellen in Puffer A (120 mM NaCl, 5 mM KCl, 1,5 mM MgCl₂, 1 mM CaCl₂, 25 mM HEPES, 10 mM Glucose, pH 7,4) bis zu einer Dichte von 2 x 10⁶/ml resuspendiert und in 30 min bei 37°C im Dunkeln mit Fura 2-am (2 µM), Pluronics F-127 (0,04 %) und DMSO (0,2 %) inkubiert. Danach wurden die Zellen zweimal mit Puffer A gewaschen und zu 2 x 10⁶/ml resuspendiert.

Das Fluoreszenzsignal von 2 x 10⁵ Zellen pro ml bei Ex/Em 380/510 wurde bei 30°C kontinuierlich registriert. Zu den Zellen wurden die Testsubstanzen und nach einer Inkubationszeit von 3 min ET1 wurde die maximale Änderung der Fluoreszenz bestimmt. Die Antwort der Zellen auf ET1 ohne vorherige Zugabe einer Testsubstanz diente als Kontrolle und wurde gleich 100 % gesetzt.

### Testung der ET-Antagonisten in vivo

Männliche 250 - 300 g schwere SD-Ratten wurden mit Amobarbital narkotisiert, künstlich beatmet, vagotomisiert und despinalisiert. Die Arteria carotis und Vena jugularis wurden kathetisiert.

In Kontrolltieren führt die intravenöse Gabe von 1 µg/kg ET1 zu einem deutlichen Blutdruckanstieg, der über einen längeren Zeitraum anhält.

Den Testtieren wurde 5 min vor der ET1 Gabe die Testverbindungen i.v. injiziert (1 ml/kg). Zur Bestimmung der ET-antagonistischen Eigenschaften wurde der Blutdruckanstieg in den Testtieren mit dem in den Kontrolltieren verglichen.

### Endothelin-1 induzierter "sudden death" an Mäusen

Das Testprinzip besteht in der Hemmung des durch Endothelin verursachten plötzlichen Herztodes der Maus, der wahrscheinlich durch Verengung der Herzkranzgefäße bedingt ist, durch Vorbehandlung mit Endothelin-Rezeptorantagonisten. Nach intravenöser Injektion von 10 nmol/kg Endothelin im Volumen von 5 ml/kg Körpergewicht kommt es innerhalb weniger Minuten zum Tod der Tiere.

Die letale Endothelin-1 Dosis wird jeweils an einem kleinen Tierkollektiv überprüft. Wird die Prüfsubstanz intravenös appliziert, erfolgt meist 5 min danach die im Referenzkollektiv letale Endothelin-1 Injektion. Bei anderen Applikationsarten verlängern sich die Vorgabezeiten, gegebenenfalls bis zu mehreren Stunden.

Die Überlebensrate wird dokumentiert und effektive Dosen, die 50 % der Tiere 24 h oder länger gegen den Endothelin-Herztod schützen (ED 50) werden ermittelt.

### Funktioneller Gefäßtest für Endothelin-Rezeptorantagonisten

An Aortensegmenten des Kaninchens wird nach einer Vorspannung von 2 g und einer Relaxationszeit von 1 h in Krebs-Henseleitlösung bei 37°C und einem pH-Wert zwischen 7,3 und 7,4 zunächst eine K⁺-Kontraktur ausgelöst. Nach Auswaschen wird eine Endothelin-Dosiswirkungskurve bis zum Maximum erstellt.

Potentielle Endothelin-Antagonisten werden an anderen Präparaten des gleichen Gefäßes 15 min vor Beginn der Endothelin-Dosiswirkungskurve appliziert. Die Effekte des Endothelins werden in % der K⁺-Kontraktur berechnet. Bei wirksamen Endothelin-Antagonisten kommt es zur Rechtsverschiebung der Endothelin-Dosiswirkungskurve.

Die erfindungsgemäßen Verbindungen können in üblicher Weise oral oder parenteral (subkutan, intravenös, intramuskulär, intraperotoneal) verabfolgt werden. Die Applikation kann auch mit Dämpfen oder Sprays durch den Nasen-Rachenraum erfolgen.

Die Dosierung hängt vom Alter, Zustand und Gewicht des Patienten sowie von der Applikationsart ab. In der Regel beträgt die tägliche Wirkstoffdosis zwischen etwa 0,5 und 50 mg/kg Körpergewicht bei oraler Gabe und zwischen etwa 0,1 und 10 mg/kg Körpergewicht bei parenteraler Gabe.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al.: Pharmazeutische Technologie, Thieme-Verlag, Stuttgart, 1991). Die so erhaltenen Applikationsformen enthalten den Wirkstoff normalerweise in einer Menge von 0,1 bis 90 Gew.-%.

Ein weiterer Gegenstand der Erfindung ist die Kombination von Verbindungen der Formel I mit Hemmstoffen des Renin-Angiotensin-Systems (RAS). RAS-Hemmstoffe sind beispielsweise aus EP 634 175 bekannt.

Die erfindungsgemäßen Kombinationen eignen sich zur Behandlung von solchen Krankheiten, für die auch die Verbindungen der Formel I allein Wirksamkeit zeigen, insbesondere zur Behandlung von Hypertonie und chronischer Herzinsuffizienz.

### Synthesebeispiele

### Beispiel 1

### 2,2-Diphenylpropional

50,0 g (0,241 mol) 2,2-Diphenylpropionitril wurden in 200 ml absolutem Diethylether gelöst und 74,2 ml einer einmolaren Lösung von LiAlH₄ in Ether zugetropft. Anschließend wurde eine Stunde unter Rückfluß erhitzt und 16 Stunden bei Raumtemperatur nachgerührt.

Danach wurden 29 ml Wasser zugegeben, die organische Phase abgetrennt und die wäßrige Phase mit Ether extrahiert. Die vereinigten organischen Phasen wurden mit MgSO₄ getrocknet und das Lösungsmittel im Vakuum abgezogen. Man erhielt 52,1 g öliges Rohprodukt, das direkt weiter umgesetzt wurde.

### Beispiel 2

### 2-Hydroxy-3,3-diphenylbutyronitril

42,3 g (0,201 mol 2,2-Diphenylpropionitril wurden in 230 ml THF gelöst und 41,4 g (0,217 mol) p-Toluolsulfonsäure zugegeben. Danach wurden 14,09 g (0,217 mol) KCN in 60 ml Wasser zugetropft. Anschließend wurde noch 3 Stunden auf 40°C erwärmt. Die Reaktionsmischung wurde auf ca. 30 % i.Vak. eingeengt, in Wasser aufgenommen und dreimal mit Essigester extrahiert. Die vereinigten organischen Phasen wurden zweimal mit Natriumdisulfitlösung gewaschen, mit MgSO₄ getrocknet und i.Vak. eingeengt. Das Rohprodukt wurde über Kieselgel mit n-Heptan/Essigester (20:1) chromatographiert. Man erhielt 41,2 g (86 %) 2-Hydroxy-3,3-diphenylbutyronitril.
¹H-NMR [CDCl₃],
- δ =: 1,9 (s, 3H); 2.7 (d, 1H); 5,1 (d, 1H);
7,2 - 7,4 (m, 10H)

### Beispiel 3

### 2-Hydroxy-3,3-diphenylbuttersäureethylester

1,0 g (4,2 mmol) 2-Hydroxy-3,3-diphenylbutyronitril wurden in 10 ml Ethanol gelöst und 10 ml konz, HCI zugegeben. Die Mischung wurde 24 Stunden unter Rückfluß erhitzt. Danach wurde das Solvens im Vak. abgezogen, der Rückstand in Wasser aufgenommen und zweimal mit Essigester extrahiert. Die vereinigten organsichen Phasen wurden mit 10 %iger NaOH gewaschen, mit MgSO₄ getrocknet und i.Vak. eingeengt. Man erhielt 0,8 g (67 %) Produkt.
¹H-NMR [CDCl₃],
- δ =: 0,9 (t, 3H); 1,8 (s, 3H); 3,0 (d, 1H);
3,9 (q, 2H); 5,0 (d, 1H); 7,1 - 7,4 (m, 10H)

### Beispiel 4

### 2-Hydroxy-3,3-diphenylbuttersäureamid

10,0 g (4,22 mmol) 2-Hydroxy-3,3-diphenylbutyronitril wurden in 500 ml Methanol (abs.) gelöst und bei 5 - 10°C 3 Stunden HCl eingeleitet. Anschließend wurde noch 3 Stunden bei 5°C und 16 Stunden bei Raumtemperatur gerührt. Dann wurden 200 ml 6 molare HCl zugegeben und die Mischung i.Vak. zur Trockene eingeengt. Das Rohprodukt wurde aus Essigester-Heptan-Gemisch umkristallisiert.
Man erhielt 2,5 g (23%) 2-Hydroxy-3,3-diphenylbuttersäureamid als weißen Feststoff.

### Beispiel 5

### 2-Hydroxy-3,3-diphenylbuttersäureethylester

2,15 g (8,4 mmol) 2-Hydroxy-3,3-diphenylbuttersäureamid wurden in 15 ml Ethanol gelöst, 15 ml konz. HCI zugegeben und 40 Stunden unter Rückfluß erhitzt. Das Solvens wurde i.Vak. abgezogen und der Rückstand in Wasser aufgenommen. Die wäßrige Phase wurde dreimal mit Essigester extrahiert und die vereinigten organischen Phasen zweimal mit 10%iger Natronlauge gewasceh. Nach dem Trocknen mit MgSO₄ und Abziehen des Solvens i.Vak. erhielt man 1,75 g (73 %) 2-Hydroxy-3,3-diphenylbuttersäureethylester.

### Beispiel 6

### 2-Hydroxy-3,3-diphenylbuttersäure

1,75 g (6.2 mmol) 2-Hydroxy-3,3-diphenylbuttersäureethylester wurden in 10 ml THF gelöst und eine Lösung von 0,23 g (9,3 mmol) LiOH in 6 ml Wasser zugegeben. Man ließ 16 Stunden bei Raumtemperatur und 4 Stunden bei 40°C rühren. Anschließend wurde die Mischung i.Vak. eingeengt, in Wasser aufgenommen und mit Essigester gewaschen. Danach wurde mit HCl angesäuert und dreimal mit Essigester extrahiert. Die vereinigten Essigesterphasen wurden mit MgSO₄ getrocknet und das Lösungsmittel i. Vak. abgezogen. Der Rückstand wurde über Kieselgel mit CH₂Cl₂/MeOH (2:1) chromatographiert. Man erhielt 0,80 g (50 %) 2-Hydroxy-3,3-diphenylbuttersäure.
¹H-NMR [DMSO-d₆],
- δ =: 1,75 (s, 3H); 4,85 (s, 1H); 5,5 (s, breit, 1H));
7,1 - 7,4 (m, 10H), 12,2 (s, breit, 1H)

### Beispiel 7

### 2-(4,6-Dimethyl-pyrimidin-2-yloxy)-3,3-diphenylbuttersäure

0,29 g (9,5 mmol) NaH wurden in DMF vorgelegt und unter Stickstoff 0,80 g (3,15 mmol) 2-Hydroxy-3,3-diphenylbuttersäure in 3 ml DMF zugegeben. Nach 30 Minuten Rühren bei Raumtemperatur wurden 0,45 g (3,15 mmol) 4,6-Dimethyl-2-chlorpyrimidin in 4 ml DMF zugegeben und noch 16 Stunden bei RT gerührt. Nach Abziehen des Solvens i.Vak. wurde der Rückstand in Wasser aufgenommen, mit HCl sauergestellt und zweimal mit Essigester extrahiert. Die vereinigten organischen Phasen wurden mit MgSO₄ getrocknet und das Lösungsmittel i.Vak. abgezogen. Der Rückstand wurde über Kieselgel mit CH₂Cl₂/Methanol (10:1) chromatographiert.

Man erhielt 0,37 g (32%) 2-(4,6-Dimethyl-pyridin-2-yloxy)-3,3-diphenylbuttersäure vom Schmp. 225-230°C.
¹H-NMR [DMSO-d₆],
- δ =: 1,95 (s, 3H); 2,3 (s, 6H); 5,95 (s, 1H));
6,8 (s, 1H); 7,0 - 7,45 (m, 10 H)

Die Verbindung wurde durch Racematspaltung in ihre beiden Enantiomeren aufgetrennt (siehe Tab. 2).

### Beispiel 8

### 2-Hydroxy-3,3-diphenylbuttersäuremethylester

15,0 g (63,3 mmol) 2-Hydroxy-3,3-diphenylbutyronitril wurden in 250 ml absolutem Methanol gelöst und bei 30 - 50°C HCl eingeleitet bis zur Sättigung. Anschließend wurde 72 Stunden unter Rückfluß erhitzt, danach i.Vak. eingeengt und der Rückstand in Wasser aufgenommen. Die Wasserphase wurde dreimal mit Essigester extrahiert; die vereinigten organischen Phasen wurden über MgSO₄ getrocknet und das Solvens i.Vak. abgezogen und der Rückstand mit n-Heptan/Essigester (20:1) über Kieselgel chromatographiert.

Man erhielt 1,2 g (7%) 2-Hydroxy-3,3-diphenylbuttersäuremethylester.
¹H-NMR [CDCl₃]
- δ =: 1,8 (s, 3H); 2,95 (d, 1H); 3,45 (s, 3H));
5,0 (d, 1H); 7,1 - 7,4 (m, 10 H)

### Beispiel 9

### 2-(4,6-Dimethoxypyrimidin-2-yloxy)-3,3-diphenylbuttersäuremethylester

1,2 g (4,4 mmol) 2-Hydroxy-3,3-diphenylbuttersäuremethylester wurden in 10 ml absolutem DMF gelöst und unter Stickstoff 1,2 g (8,8 mmol) K₂CO₃ und 0,97 g (4,4 mmol) 2-Methansulfonyl-4,6-dimethoxypyrimidin zugegeben. Die Mischung wurde 16 Stunden bei Raumtemperatur gerührt. Danach wurde eingedampft und der Rückstand in Wasser aufgenommen und dreimal mit Essigester extrahiert. Die vereinigten organischen Phasen wurden über MgSO₄ getrocknet und eingeengt. Man erhielt 1,8 g Rohprodukt, das ohne Aufreinigung weiter umgesetzt wurde.
¹H-NMR [CDCl₃]
- δ =: 2,0 (s, 3H); 3,25 (s, 3H); 3,9 (s, 6H));
5,75 (d, 1H); 5,8 (s, 1H); 7,1 - 7,3 (m, 10H)

### Beispiel 10

### 2-(4,6-Dimethoxypyrimidin-2-yloxy)-3,3-diphenylbuttersäure

1,8 g (4,4 mmol) 2-(4,6-Dimethoxypyrimidin-2-yloxy)-3,3-diphenylbuttersäuremethylester wurden in 25 ml Dioxan gelöst und 26,5 ml (26,5 mmol) einer 1M KOH-Lösung zugegeben und 6 Stunden bei 90°C gerührt. Die Mischung wurde dann eingeengt, in Wasser aufgenommen und dreimal mit Essigester extrahiert. Die vereinigten organischen Phasen wurden über MgSO₄ getrocknet und eingedampft. Der Rückstand wurde aus Ethanol umkristallisiert. Man erhielt 1,12 g (65 %) 2-(4,6-Dimethoxypyrimidin-2-yloxy)-3,3-diphenylbuttersäure vom Schmp. 229-234°C.
¹H-NMR [DMSO-d₆]
- δ =: 1,9 (s, 3H); 3,85 (s, 6H); 5,85 (s, 1H));
5,95 (s, 1H); 7,1 - 7,4 (m, 10H); 12,5 (s, breit, 1H)

### Beispiel 11

### 2,2-Diphenylbutyronitril

Zur Lösung von 50,0 g (0,259 mol) Diphenylacetonitril in 500 ml THF (abs.) wurden bei -78°C unter Argon 173 ml (0,259 mol) einer 1,5 M-Lösung von LDA in THF zugetropft und anschließend eine Stunde bei -30°C gerührt. Dann wurden bei -78°C 28,23 g (0,259 mol) Ethylbromid zugegeben. Man ließ auf Raumtemperatur kommen und noch 16 Stunden rühren. anschließend wurden 80 ml Phosphatpuffer (pH 7) zugegeben und die Mischung eingedampft. Der Rückstand wurde in Wasser aufgenommen und dreimal mit Essigester extrahiert. Die vereinigten organischen Phasen wurden über MgSO₄ getrocknet und das Solvens i.Vak. abgezogen. Das Rohprodukt wurde über Kieselgel mit n-Heptan/Essigsäure (20:1) chromatographiert. Man erhielt 38,2 g (67%) 2,2-Diphenylbutyronitril.

### Beispiel 12

### 2,2-Diphenylbutyraldehyd

21,1 g (95 mmol) 2,2-Diphenylbutyronitril wurden in 100 ml Toluol gelöst und bei -78°C unter Stickstoff 95 ml (95 mmol) einer 1 M-Lösung von Diisobutylaluminiumhydrid zugetropft. Anschließend wurde noch 16 Stunden bei Raumtemperatur gerührt, danach 60 ml einer Mischung aus gesättigter Ammoniumchloridlösung und 2 NH₂SO₄ im Verhältnis 2:1 zugegeben und 30 Minuten nachgerührt. Die Phasen wurden getrennt und die wäßrige Phase dreimal mit Essigester extrahiert. Die vereinigten organischen Phasen wurden über MgSO₄ getrocknet und i.Vak. eingeengt. Das Rohprodukt wurde über Kieselgel mit Dichlormethan chromatographiert. Man erhielt 19,0 g (89 %) 2,2-Diphenylbutyraldehyd als helles Öl.

### Beispiel 13

### 2-Hydroxy-3,3-diphenylvaleronitril

Zu einer Lösung von 19,09 g (84,8 mmol) 2,2-Diphenylbutyraldehyd in 97 ml THF (abs.) wurden 17,4 g (91,6 mmol) p-Toluolsulfonsäure · H₂O und danach 5,9 g (91,6 mmol) KCN in 25 ml Wasser bei 35°C zugegeben. Anschließend wurde noch 4 Stunden bei 40°C und 16 Stunden bei Raumtemperatur gerührt. Die Mischung wurde auf 1/3 des Volumens eingedampft, mit Wasser versetzt und die Phasen getrennt. Die wäßrige Phase wurde noch dreimal mit Essigester extrahiert und die vereinigten organischen Phasen mit 10%iger Natriumdisulfitlösung gewaschen, über MgSO₄ getrocknet und i.Vak. eingeengt. Das Rohprodukt wurde über Kieselgel nut n-Heptan/Essigsäure (20:1) chromatographiert. Man erhielt 19,5 g (92 %) 2-Hydroxy-3,3-diphenylvaleronitril als helles Öl.
¹H-NMR [CDCl₃]
- δ =: 0,7 (t, 3H); 2,2 - 2,5 (m, 3H); 5,25 (d, 1H); 7,1 - 7,4 (m, 10 H)

### Beispiel 14

### 2-(4,6-Dimethoxypyrimidin-2-yloxy)-3,3-diphenylvaleronitril

7,0 g (27,9 mmol) 2-Hydroxy-3,3-diphenylvaleronitril wurden in 100 ml DMF (abs.) gelöst und unter Stickstoff 7,57 g (55,7 mmol) Kaliumcarbonat und 6,1 g (27,9 mmol) 2-Methansulfonyl-4,6-dimethoxypyrimidin zugegeben und 72 Stunden bei Raumtemperatur gerührt. Die Mischung wurde i.Vak. eingeengt, der Rückstand in Wasser aufgenommen und dreimal mit Essigester extrahiert. Die vereinigten organischen Phasen wurden über MgSO₄ getrocknet und das Solvens abgedampft. Das Rohprodukt wurde mit n-Heptan/Essigester (20:1) über Kieselgel chromatographiert. Man erhielt 8,8 g (81%) Produkt.
¹H-NMR [CDCl₃]
- δ =: 0,8 (t, 3H); 2,45 (dq, 2H); 3,95 (s, 6H), 5,8 (s, 1H); 6,25 (s, 1H); 7,2-7,4 (m, 10 H)

### Beispiel 15

### 2-(4,6-Dimethoxypyrimidin-2-yloxy)-3,3-diphenylvaleriansäure

0,5 g (1,3 mmol) 2-(4,6-Dimethoxypyrimidin-2-yloxy) -3,3-diphenylvaleronitril wurden in 5 ml Ethanol gelöst und 5 ml konz. HCI zugegeben und die Mischung 3 Stunden unter Rückfluß erhitzt. Anschließend wurde die Mischung i.Vak. eingeengt, der Rückstand in Wasser aufgenommen und zweimal mit Essigester extrahiert. Die vereinigten Essigesterphasen wurden über MgSO₄ getrocknet und eingedampft. Der Rückstand wurde mit Acetonitril/Wasser als Elnent mit MPLC über Reversed-Phase-Material chromatographiert. Man erhielt 0,17 g (32 %) 2-(4,6-Dimethoxypyrimidin-2-yloxyl-3,3-diphenylvaleriansäure vom Schmp. 78-87°C.
¹H-NMR [DMSO-d₆]
- δ =: 0,7 (t, 3H); 2,2-2,55 (m, 2H); 3,85 (s, 6H); 5,9 (s, 2H), 70-74 (m, 10H); 12,7 (s, breit, 1H)

Die in der folgenden Tabelle 1 aufgezeigten Beispiele können nach den eingangs beschriebenen Methoden hergestellt werden:

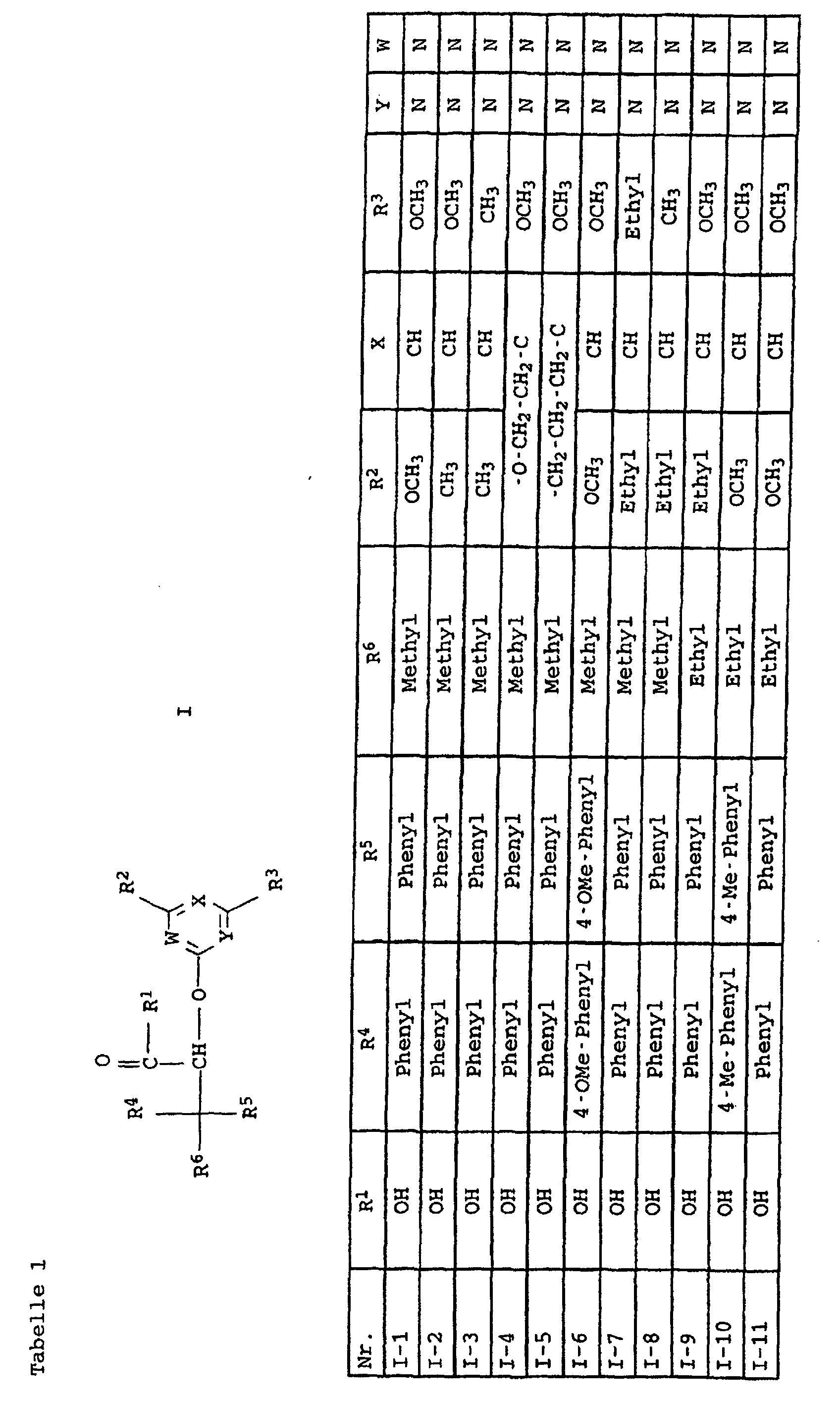

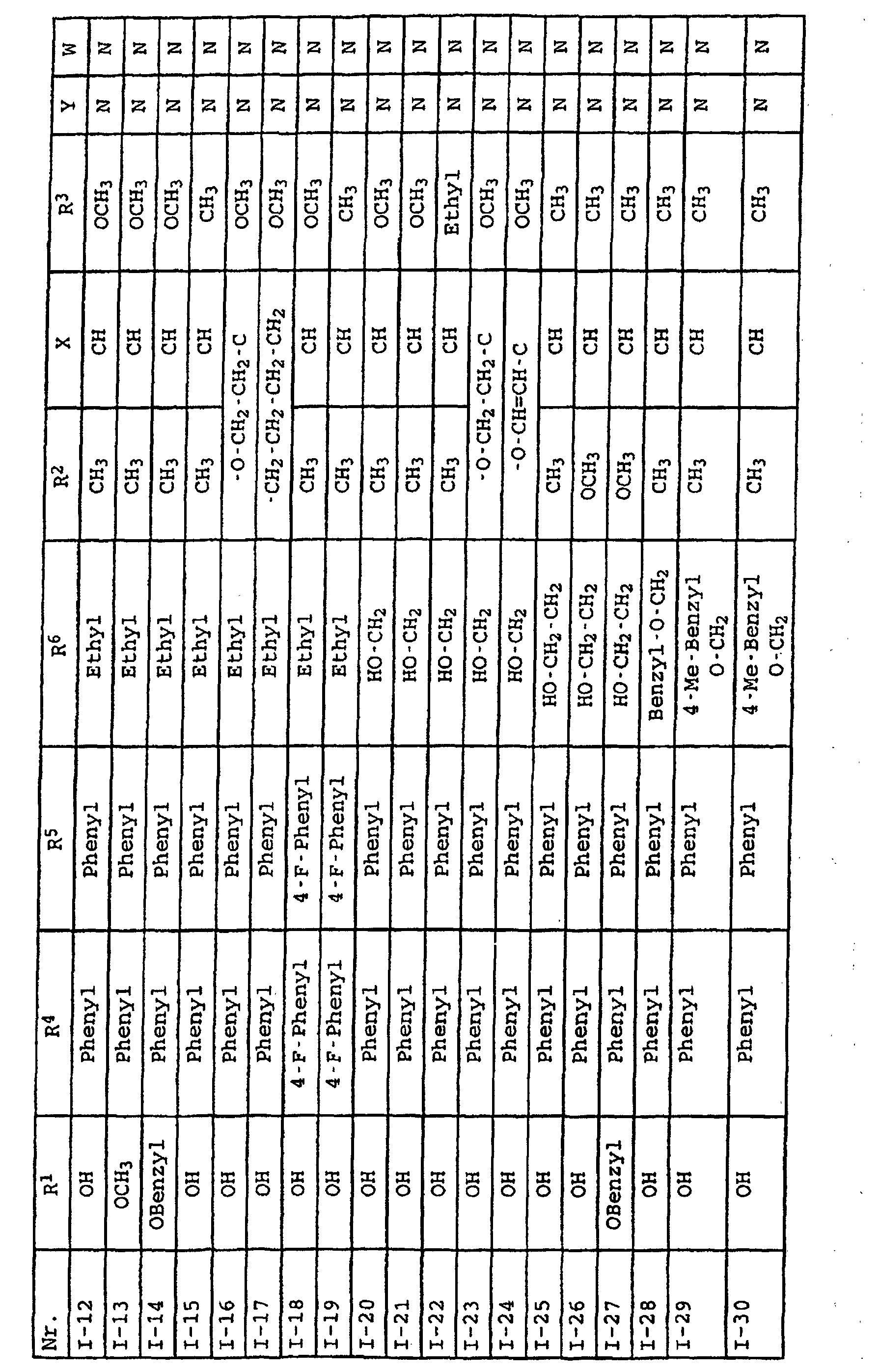

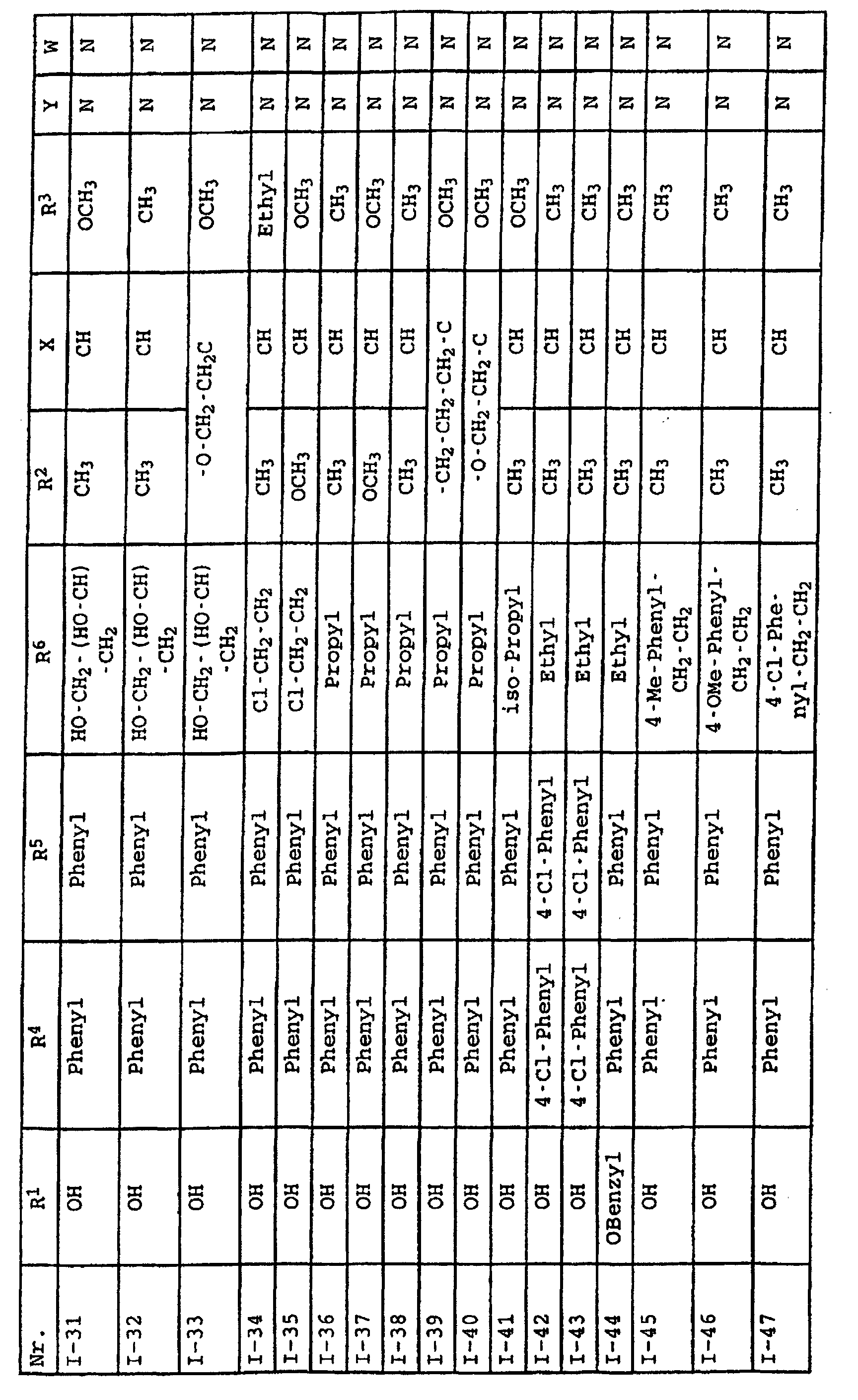

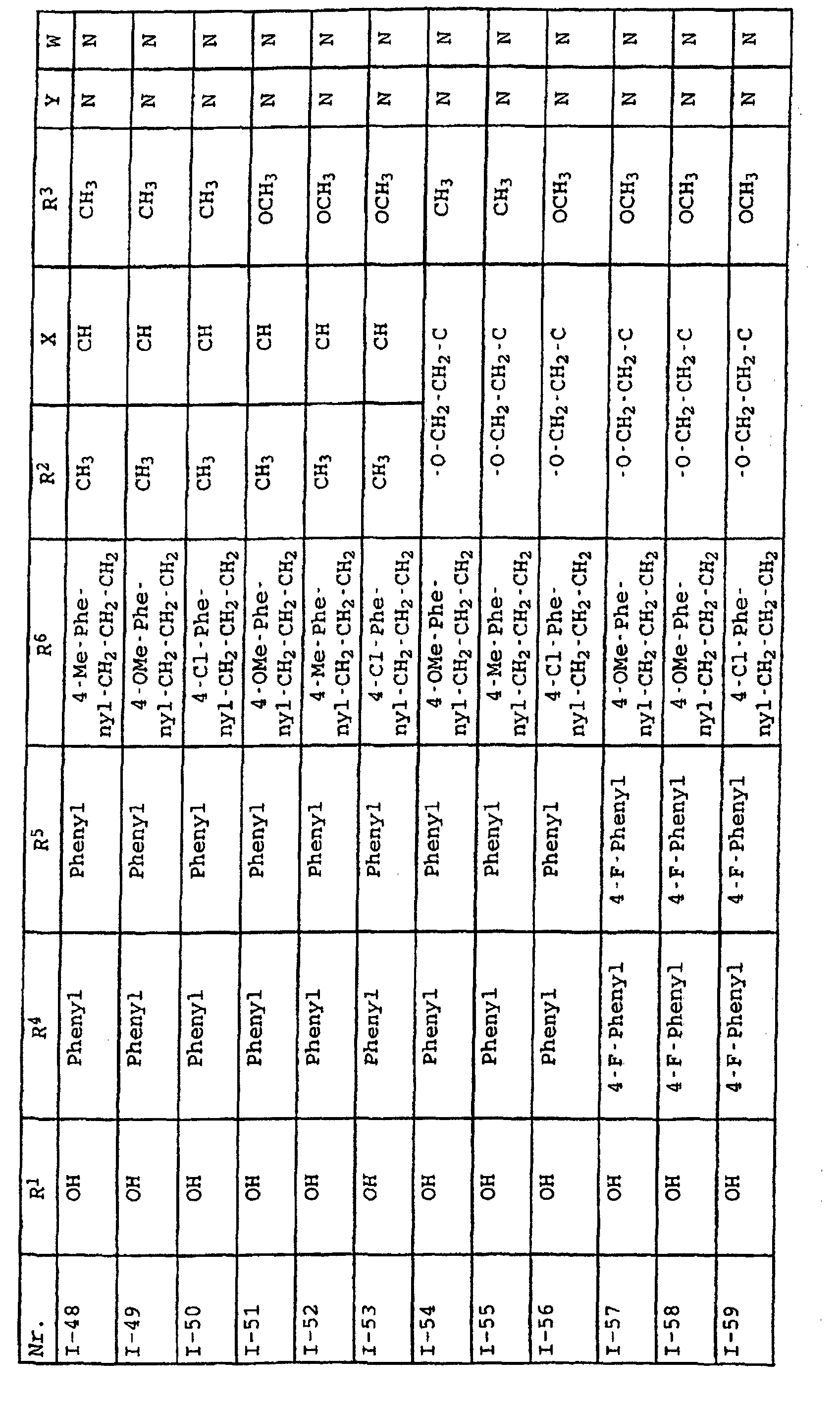

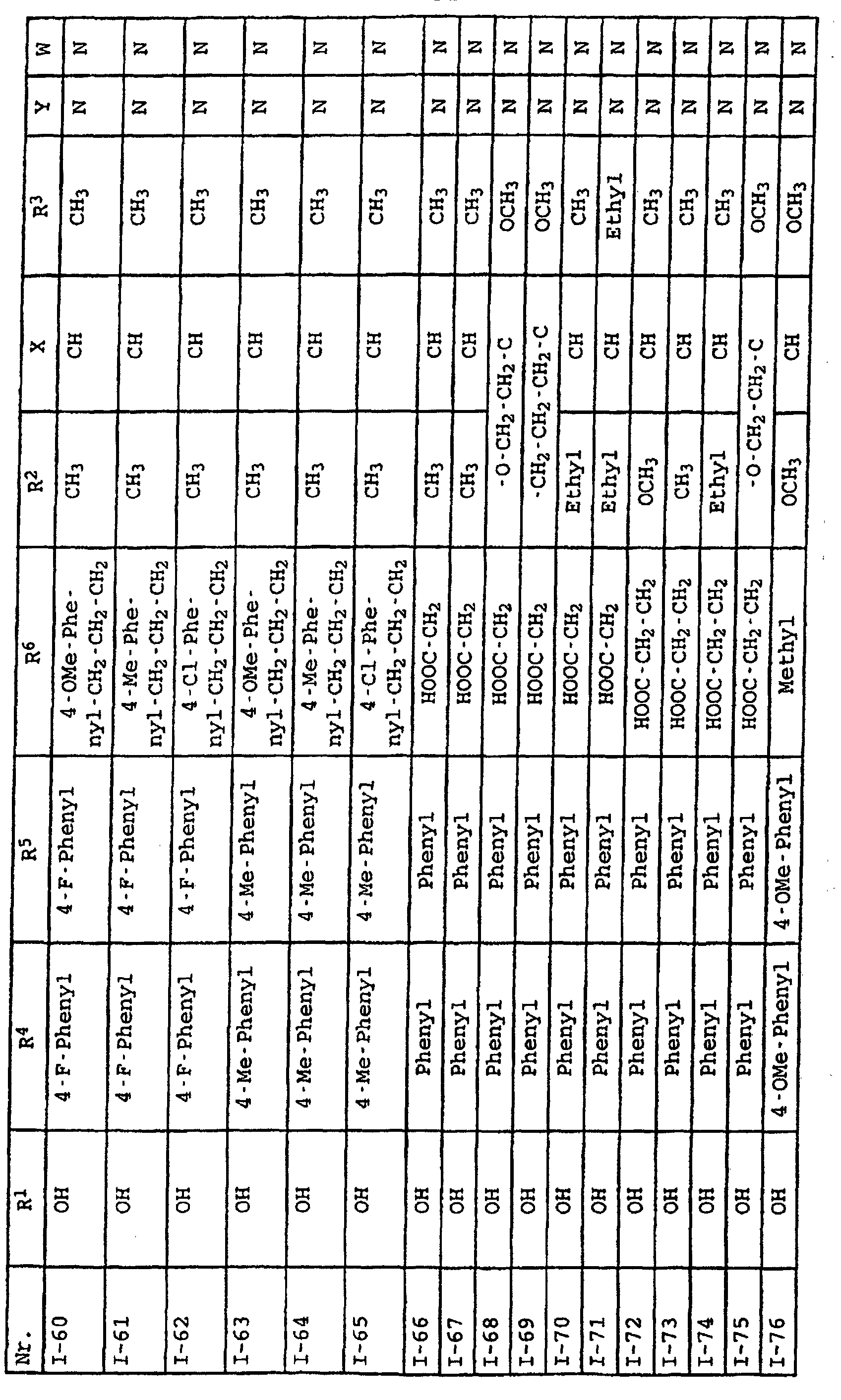

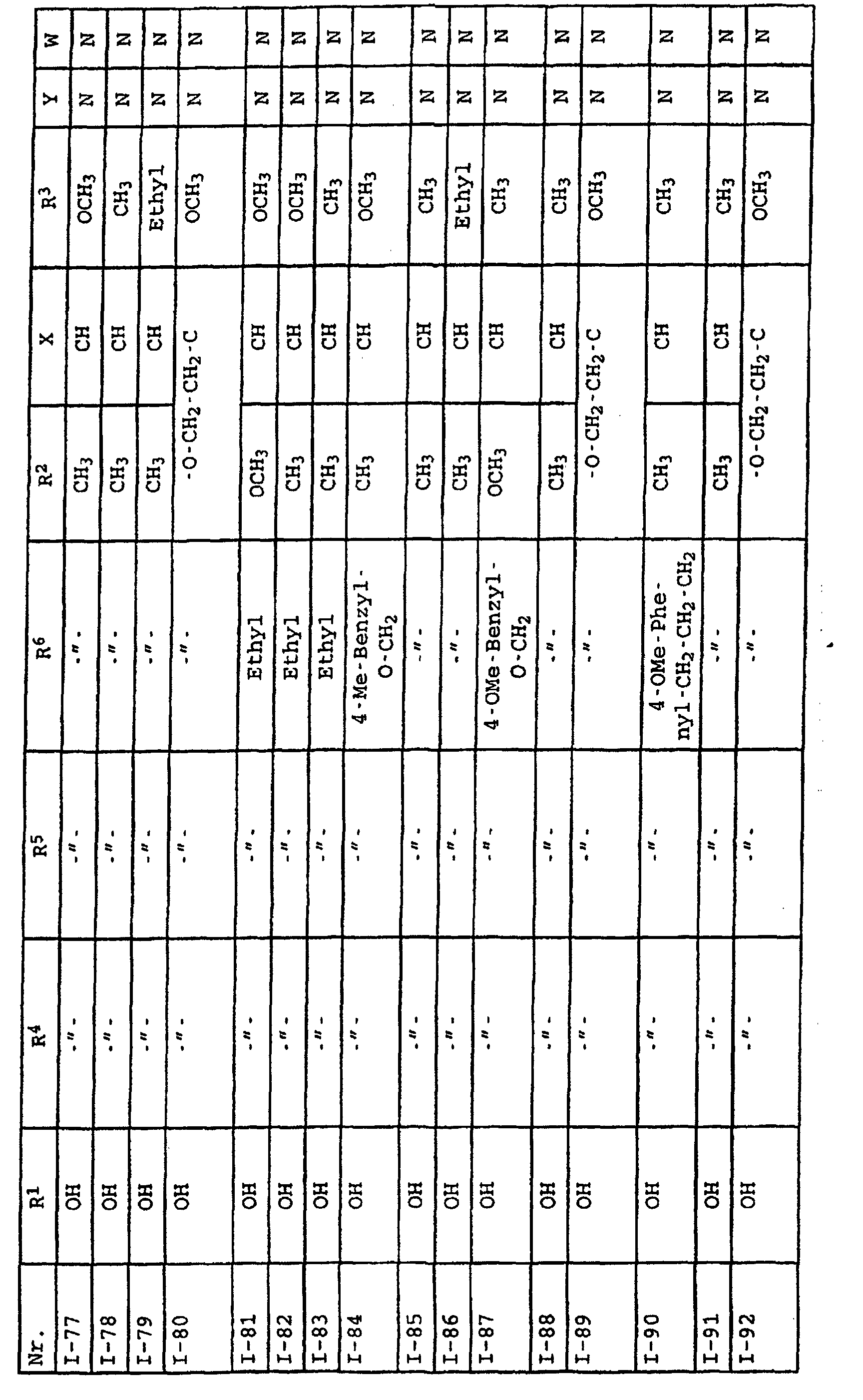

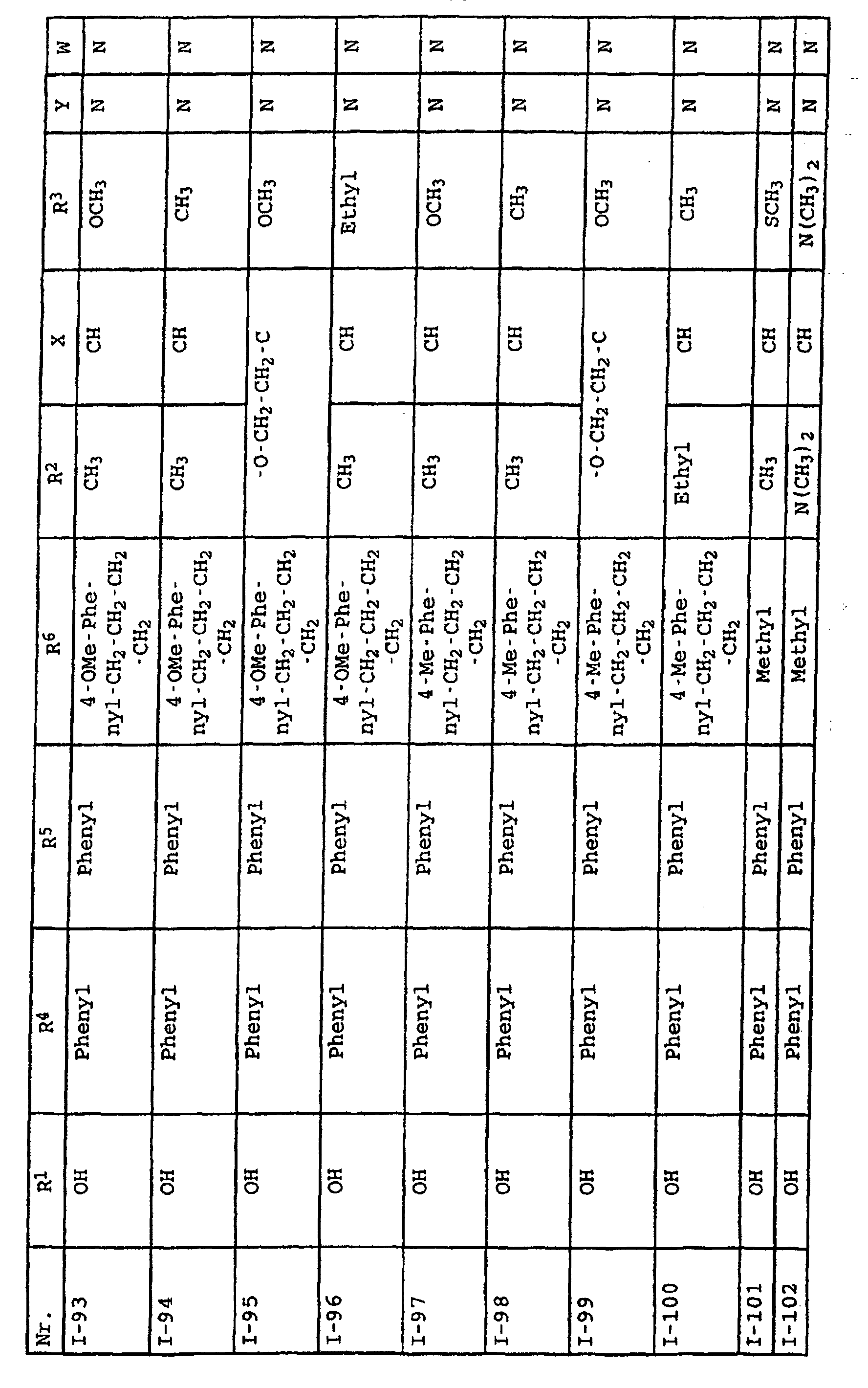

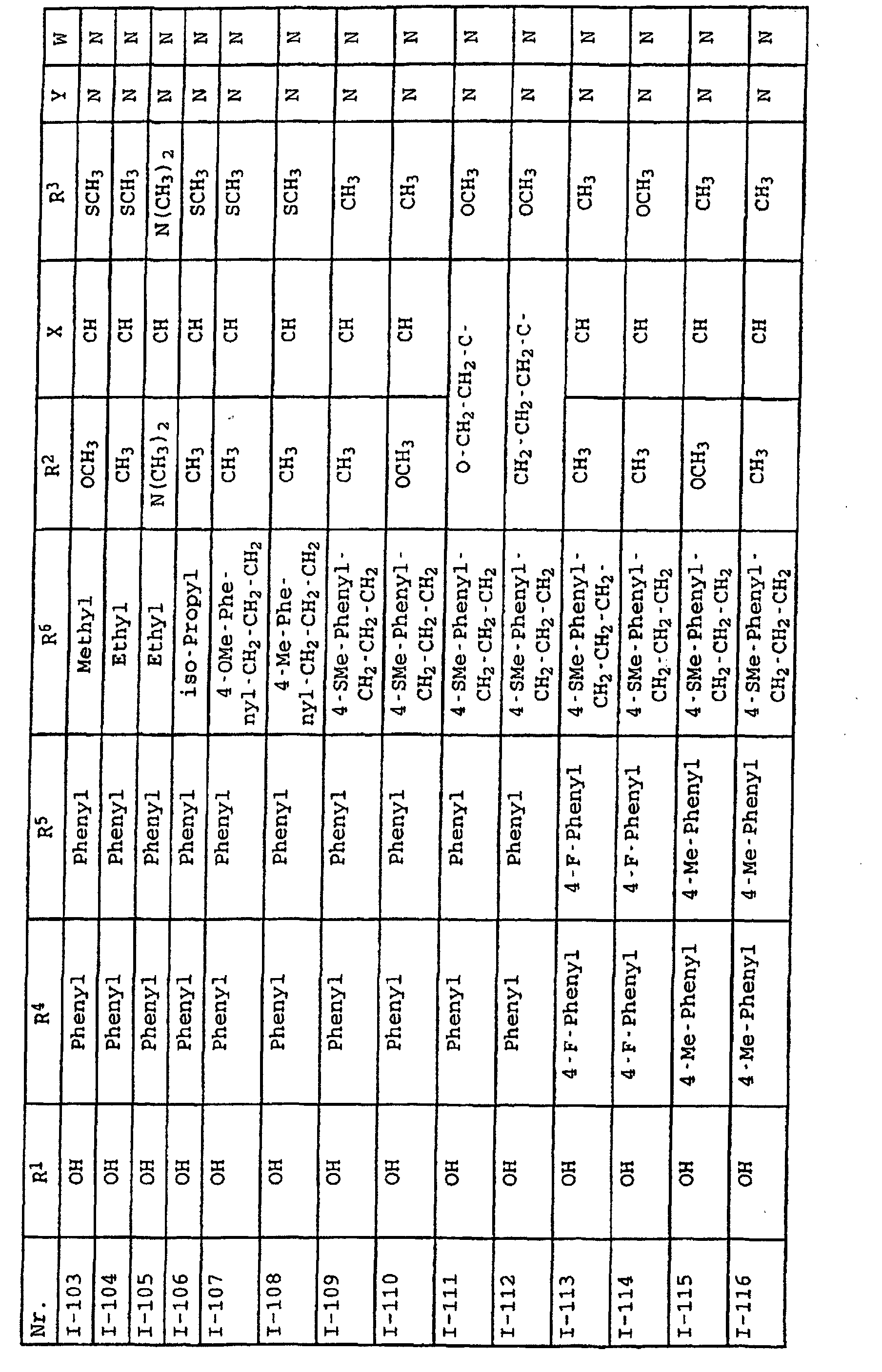

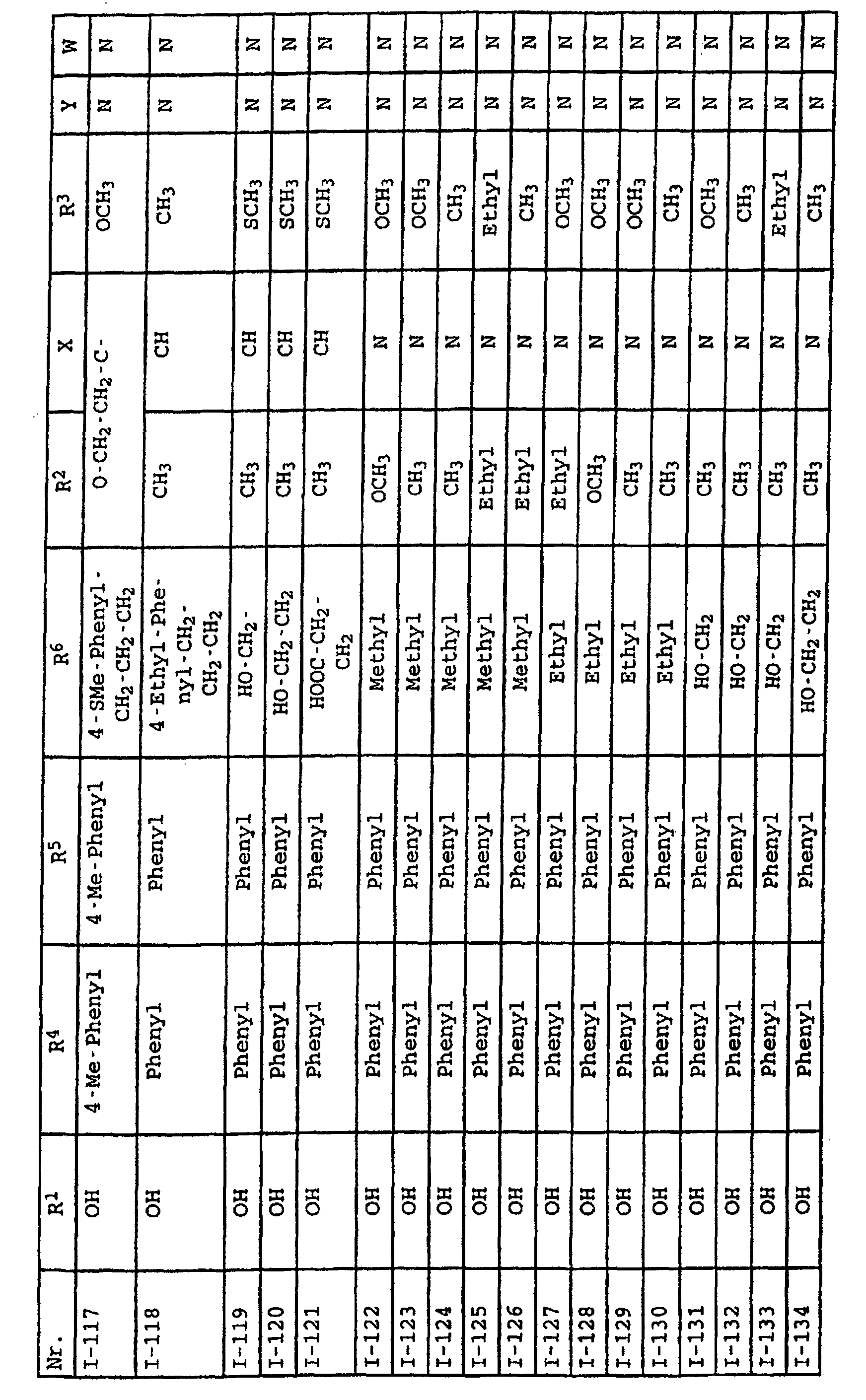

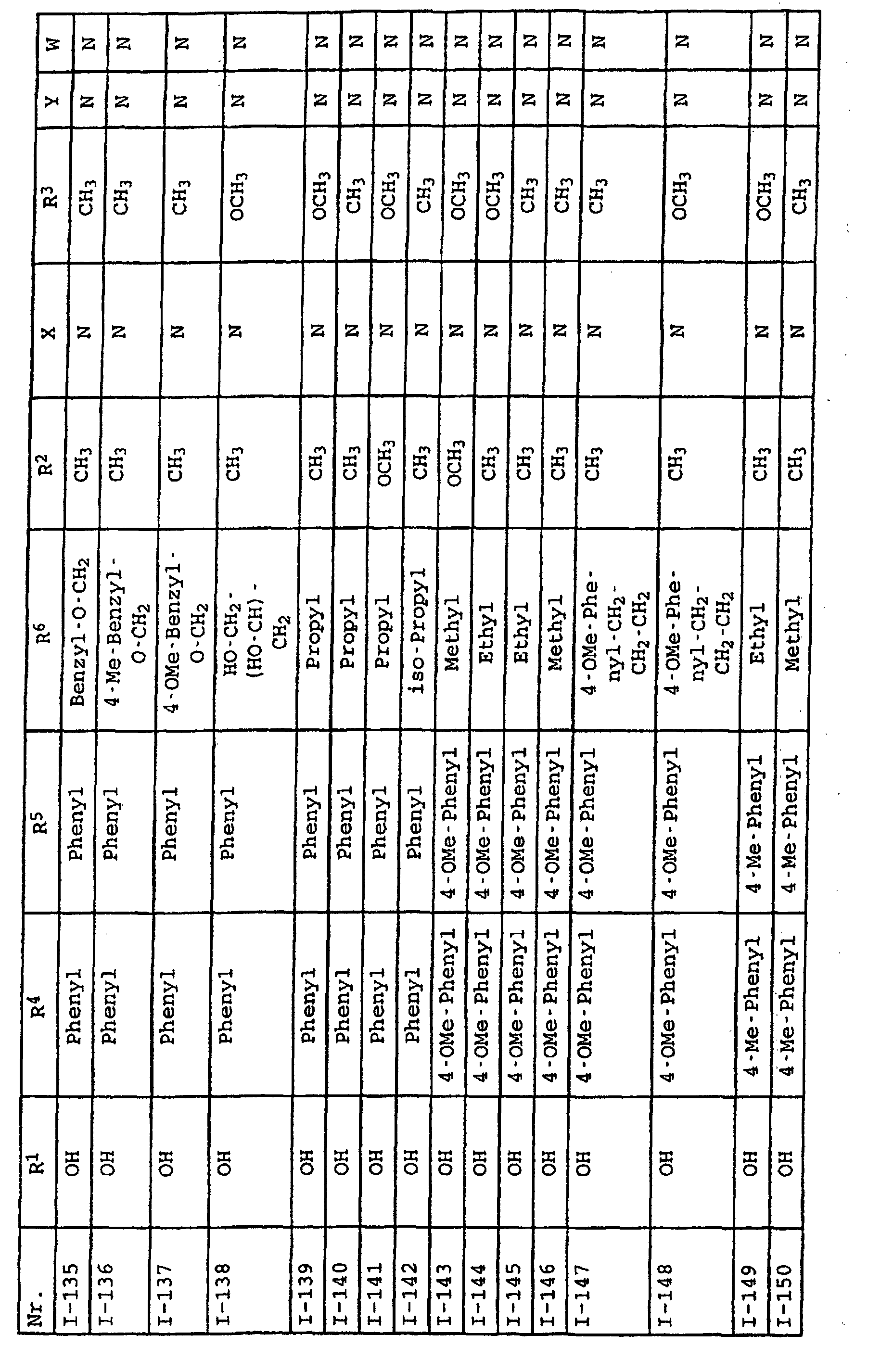

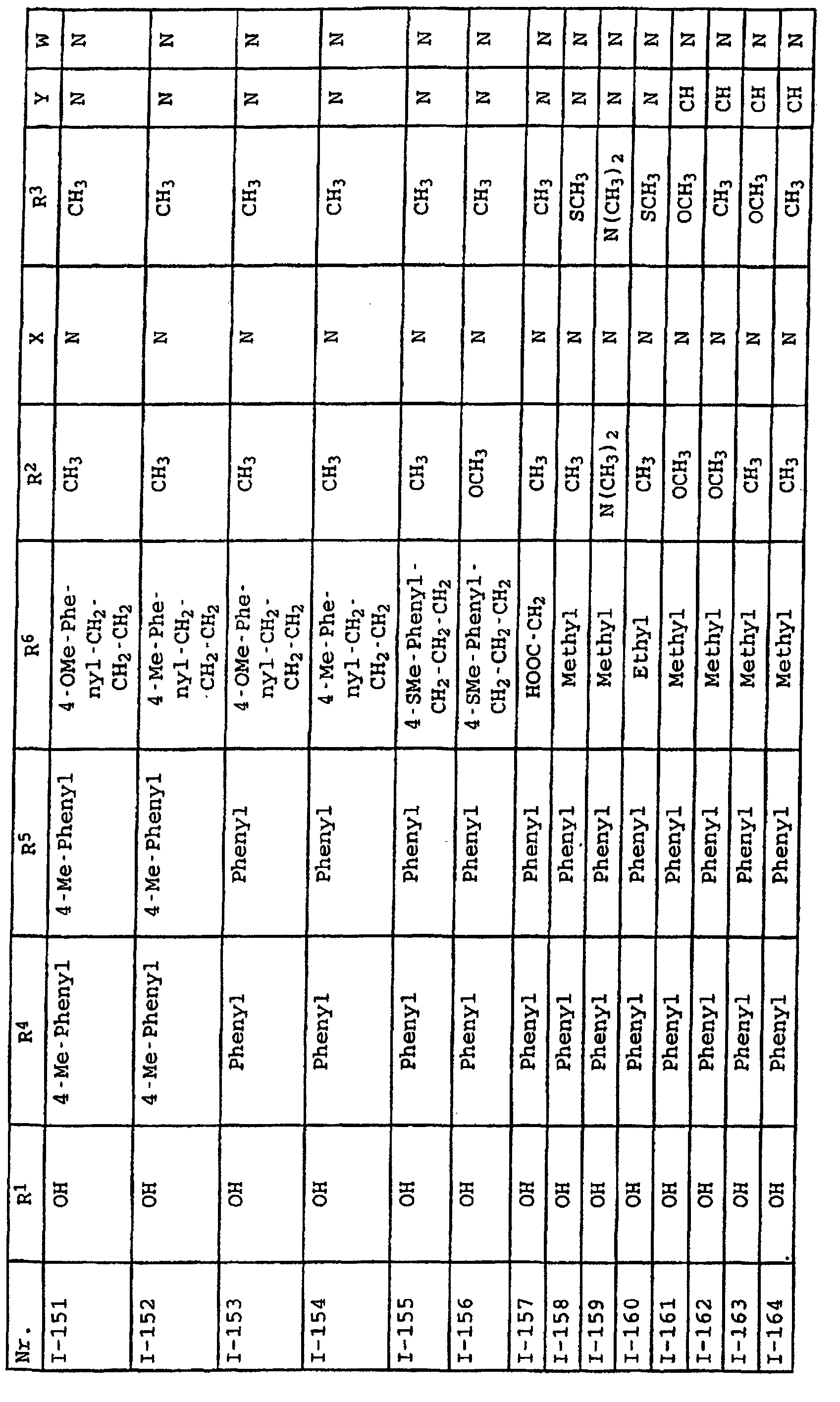

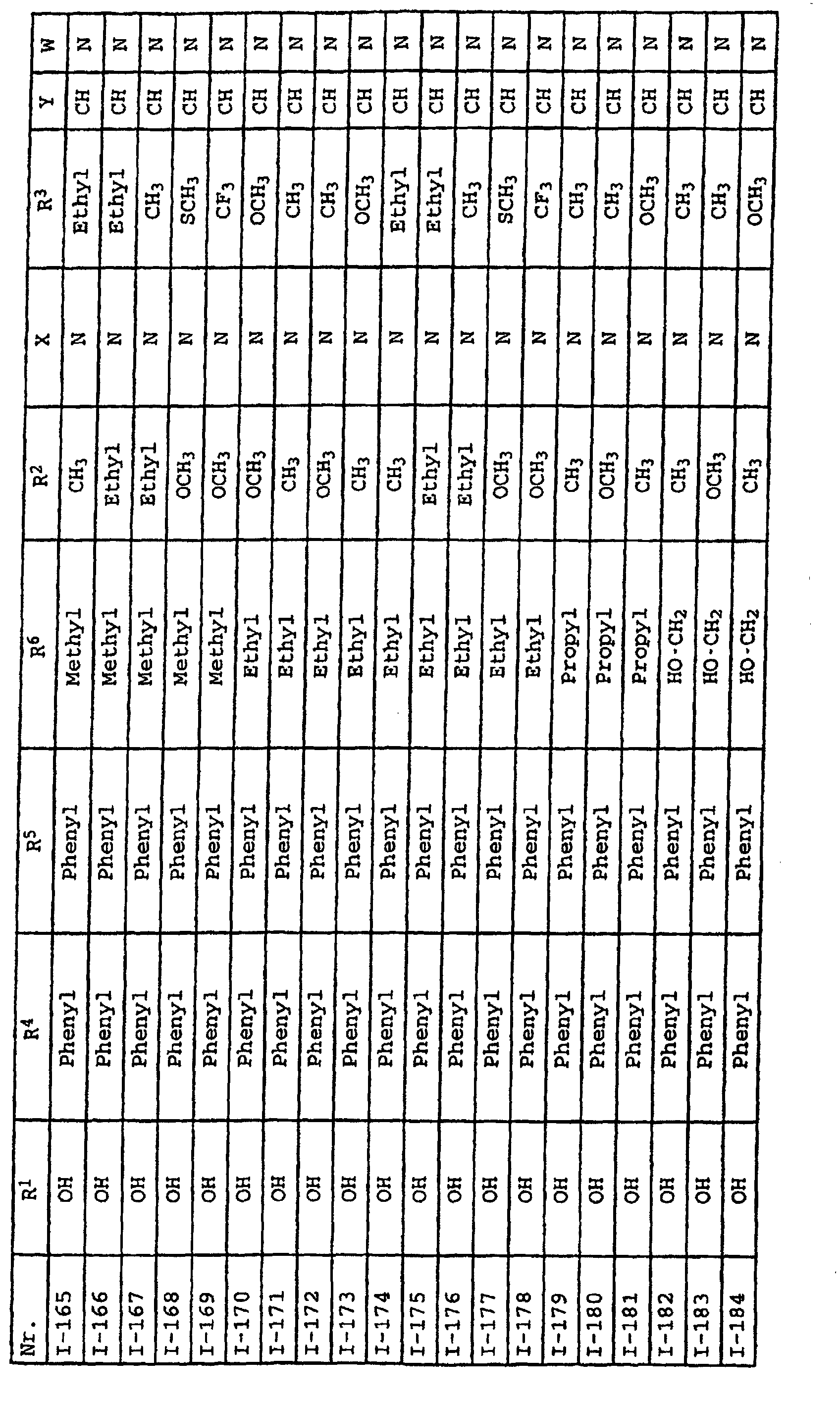

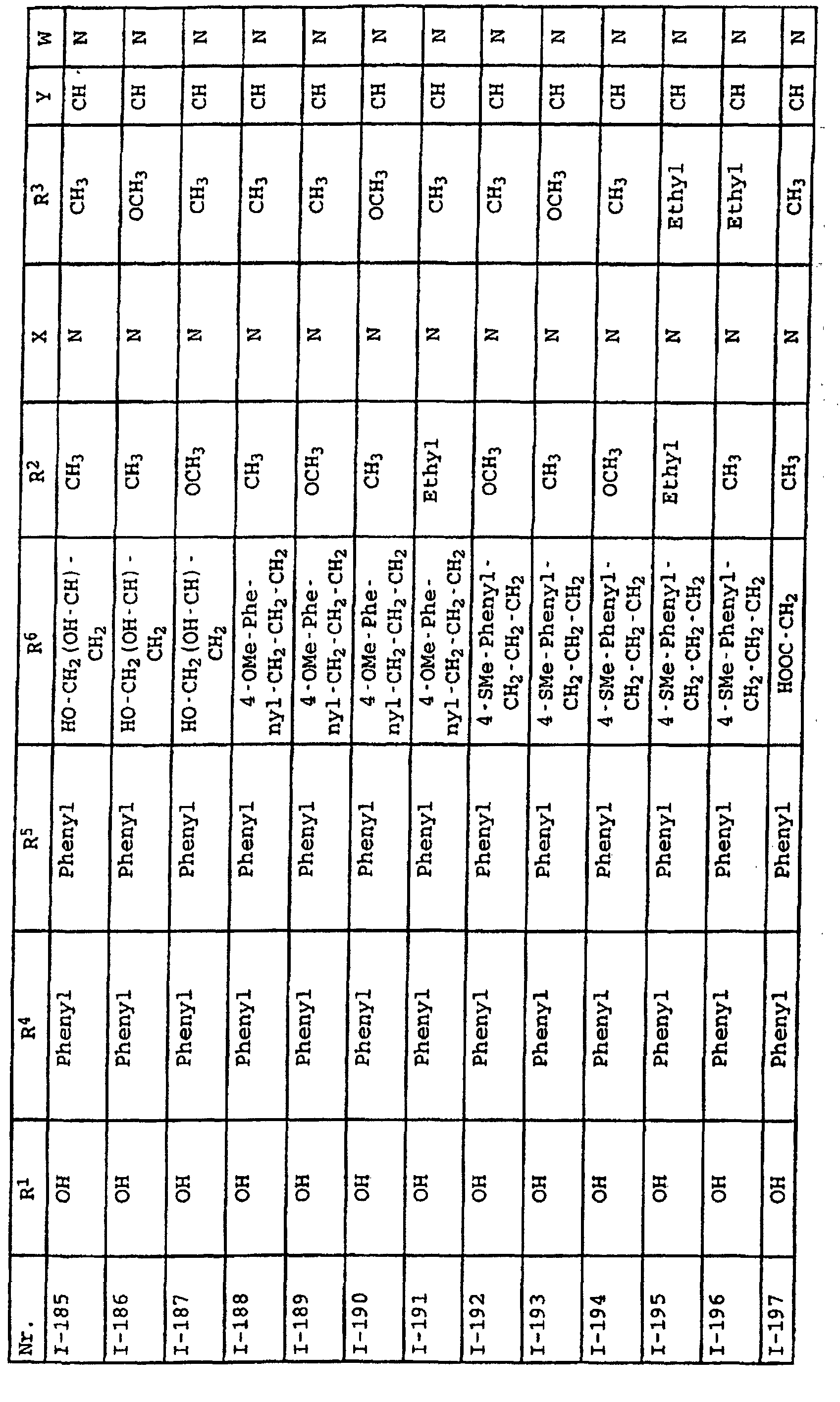

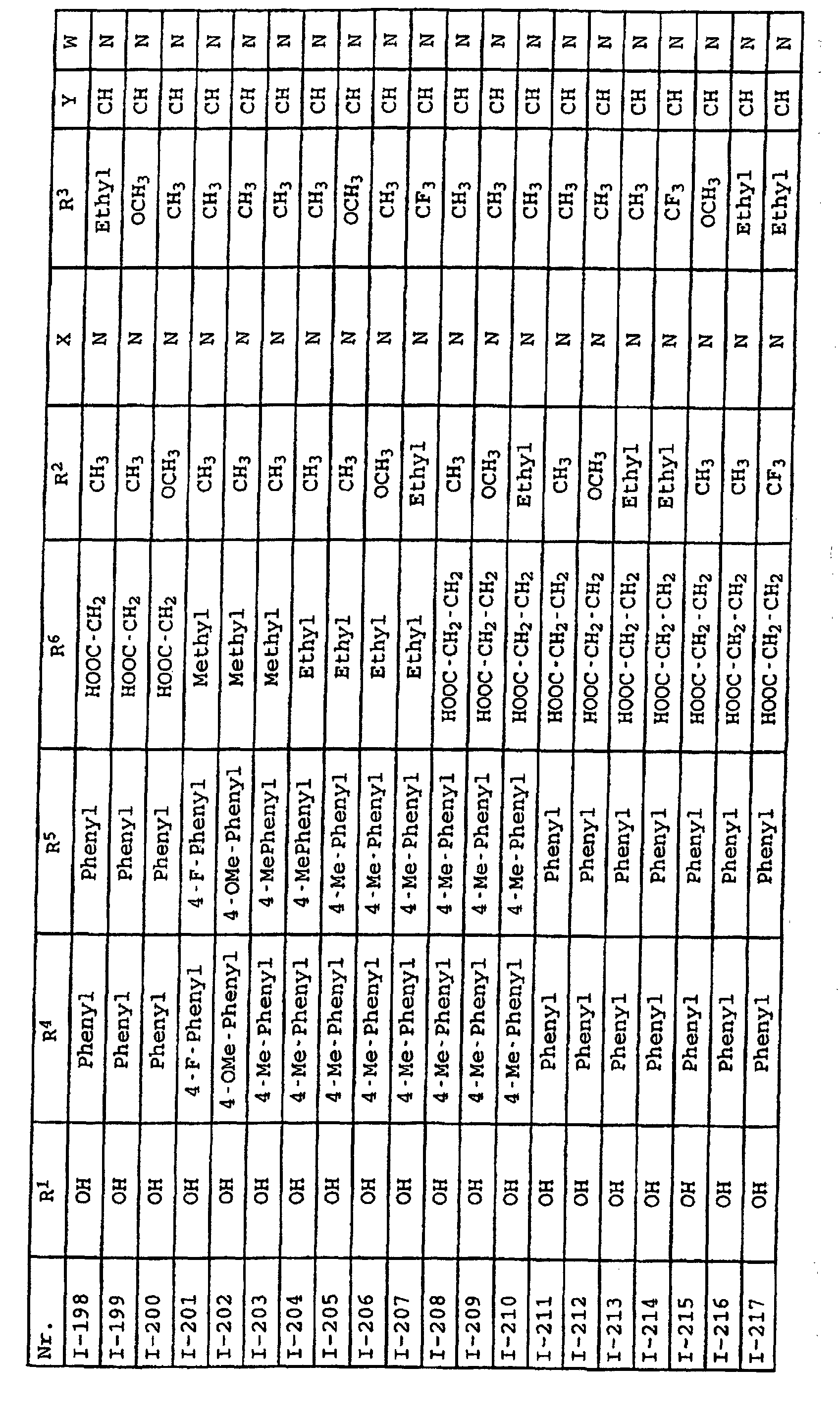

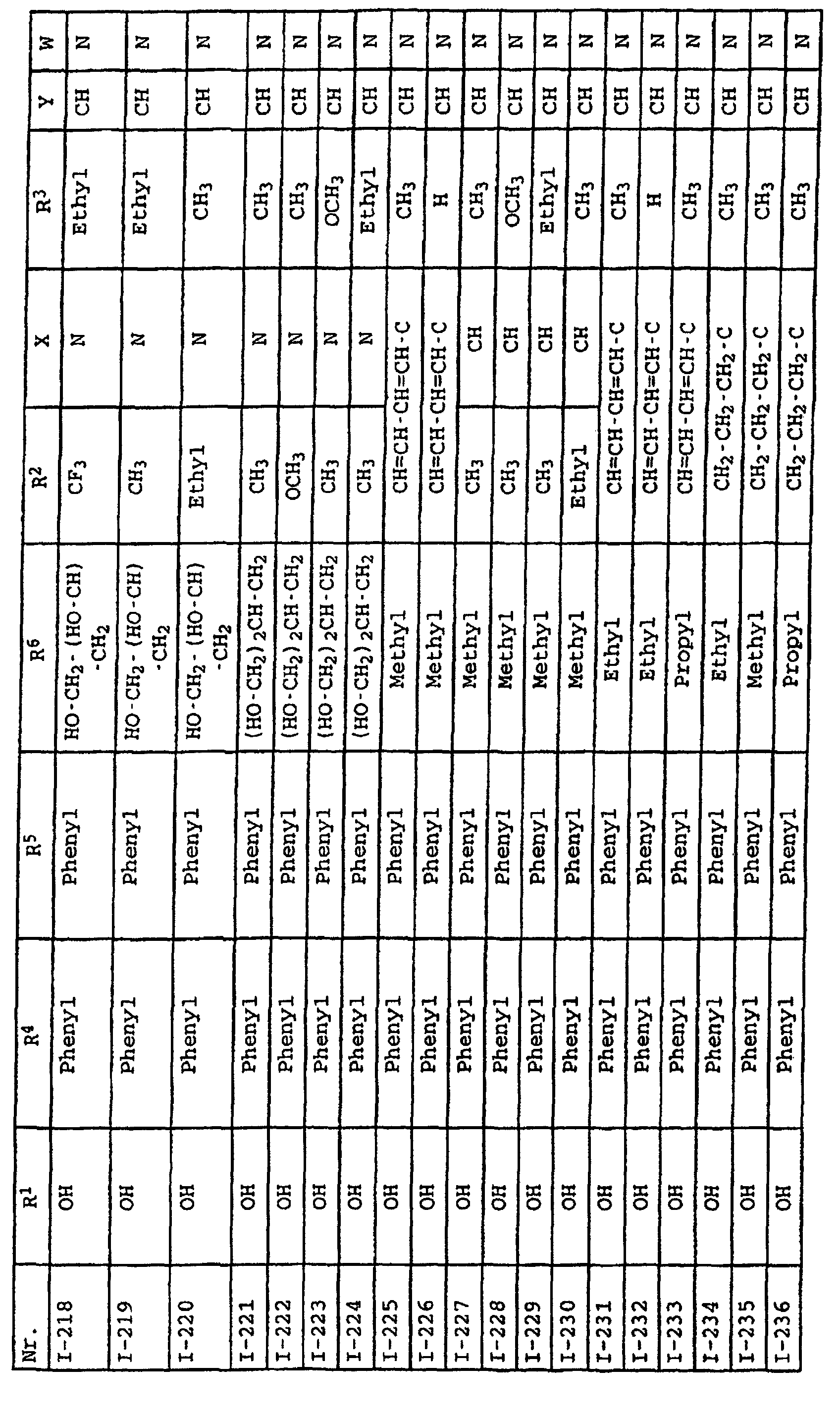

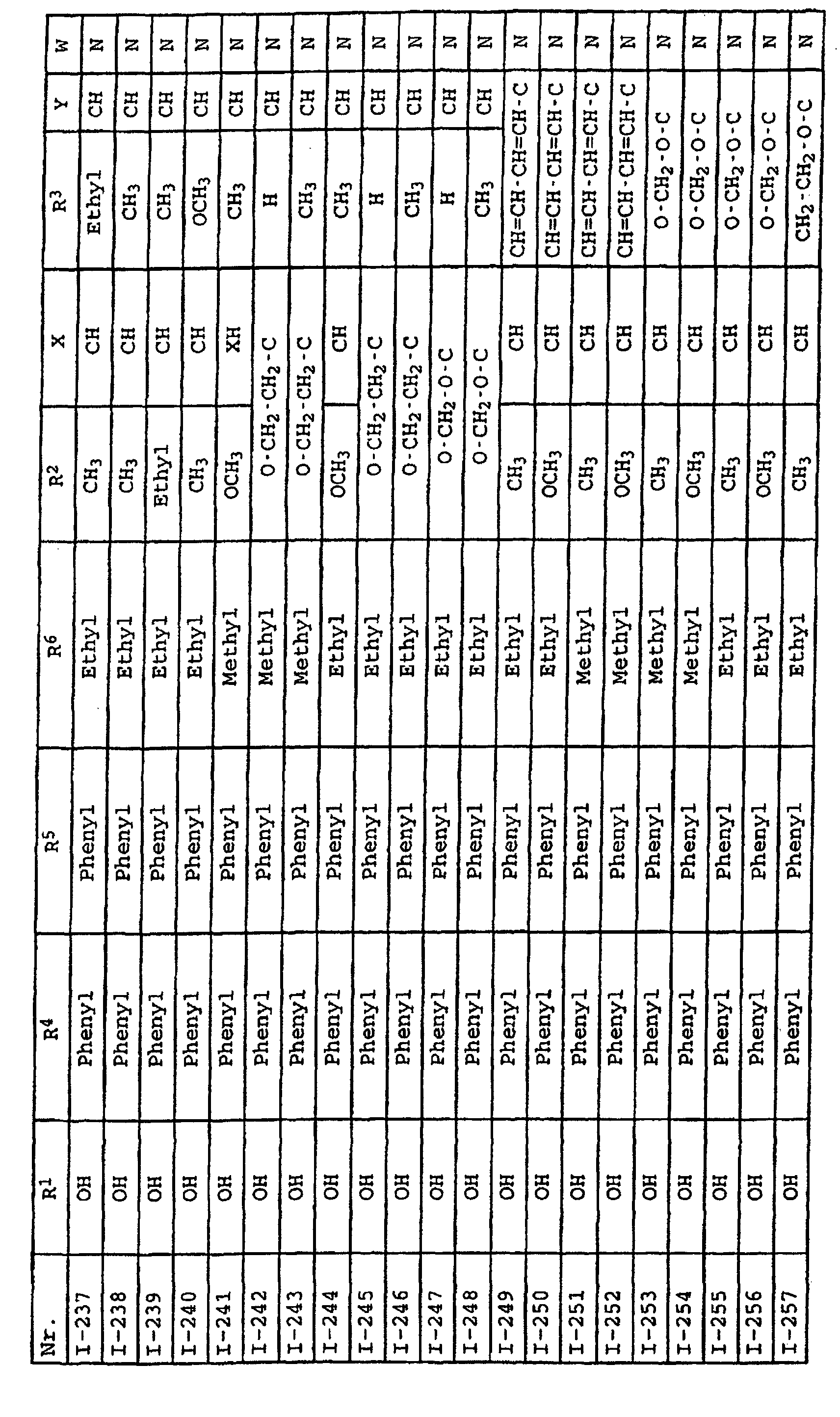

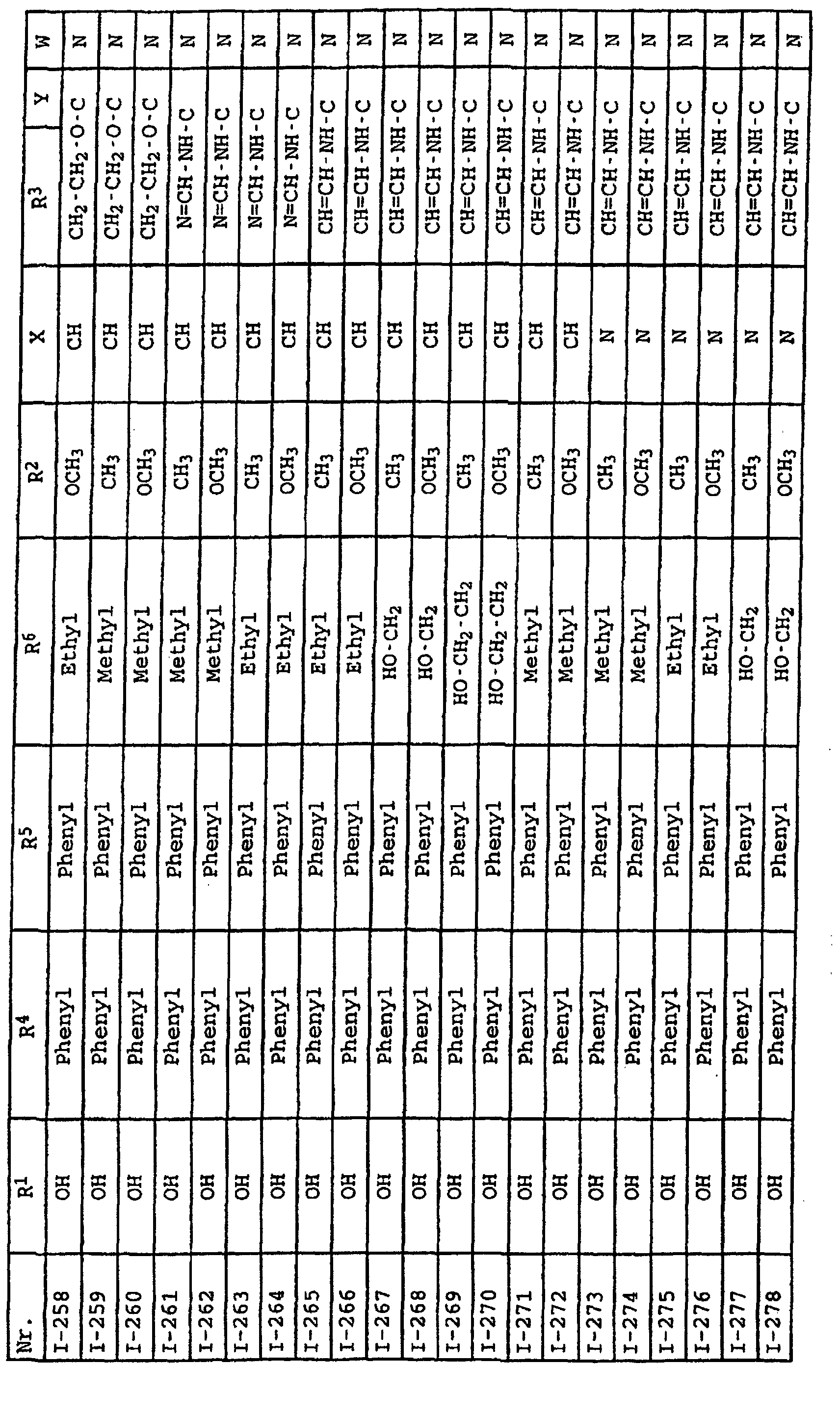

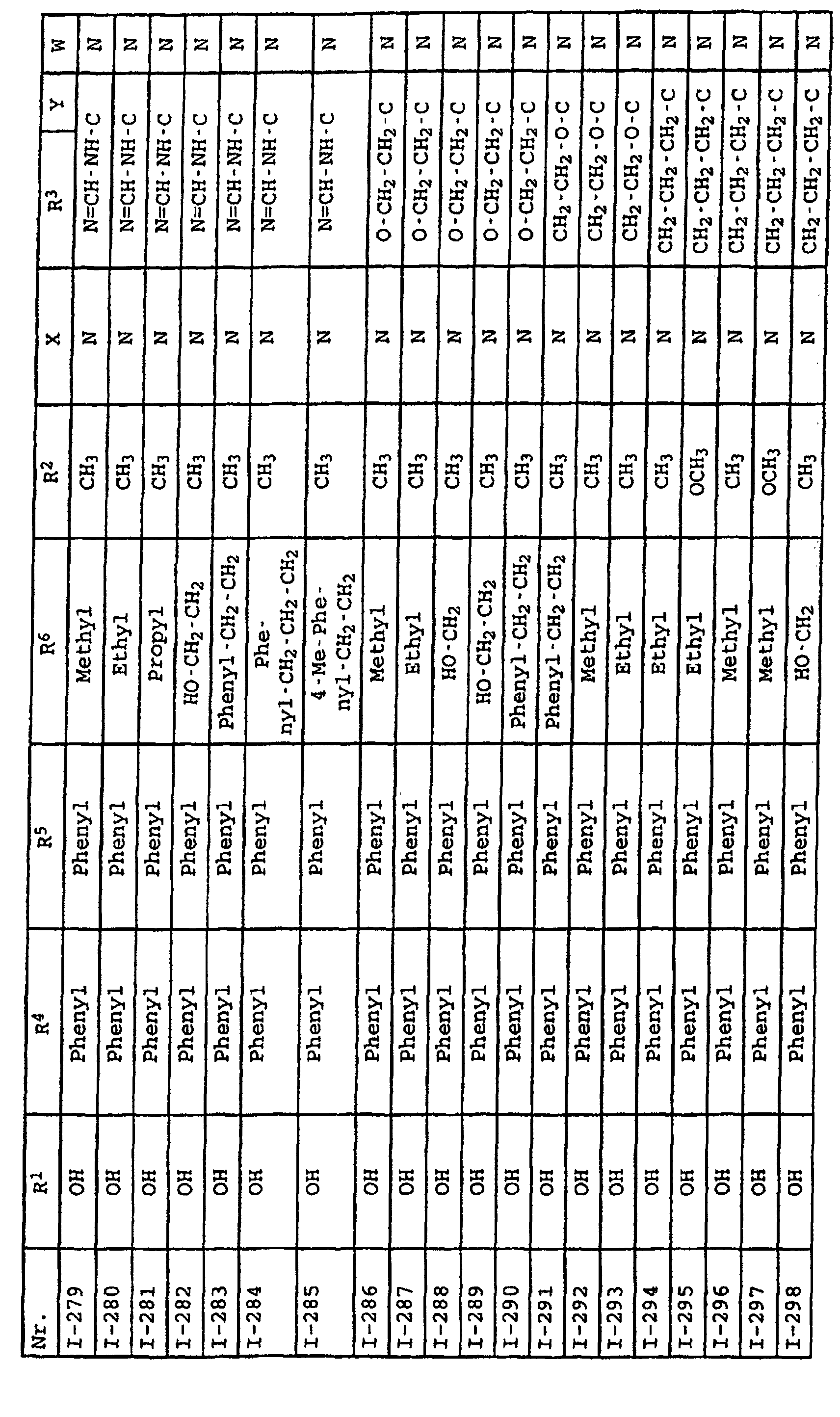

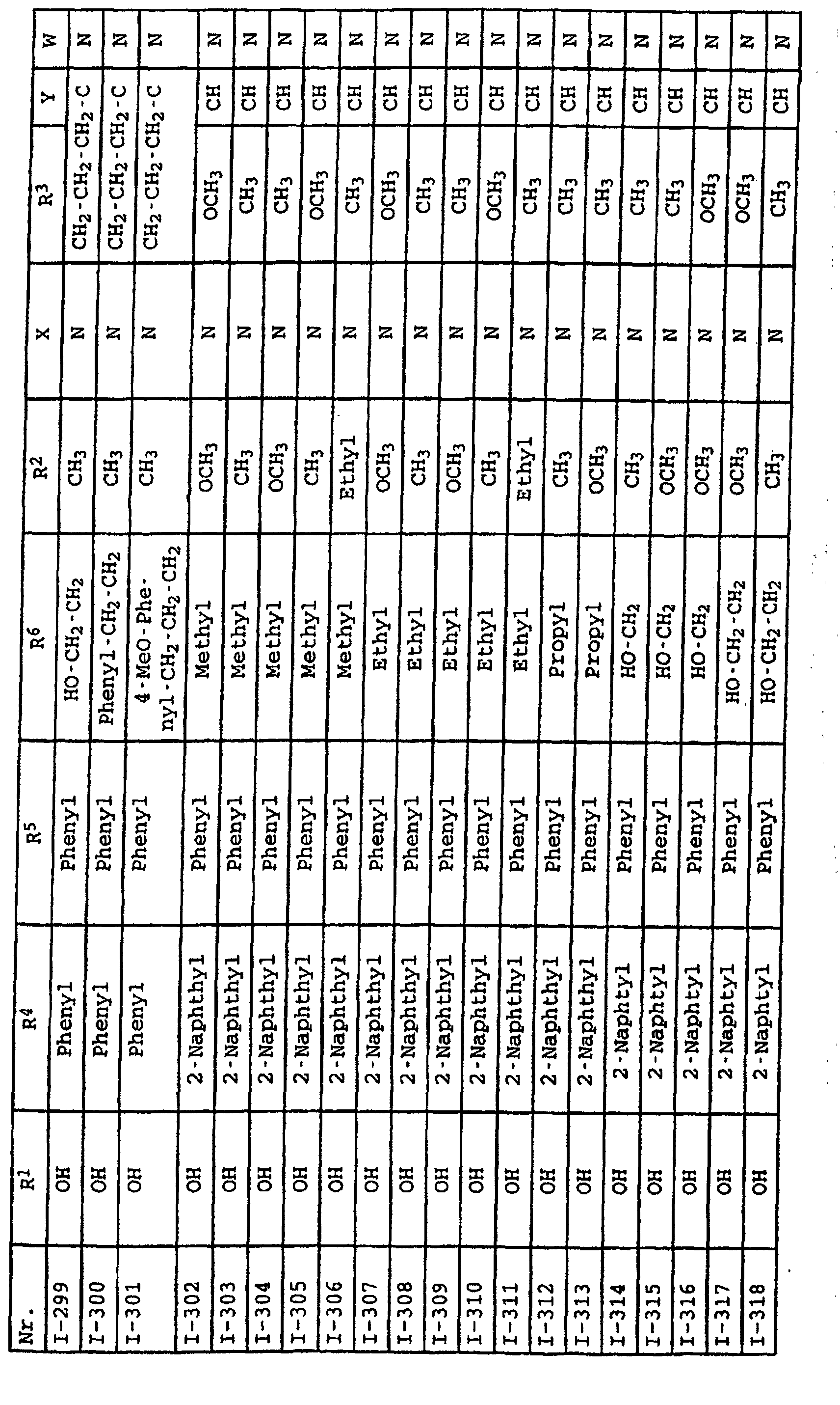

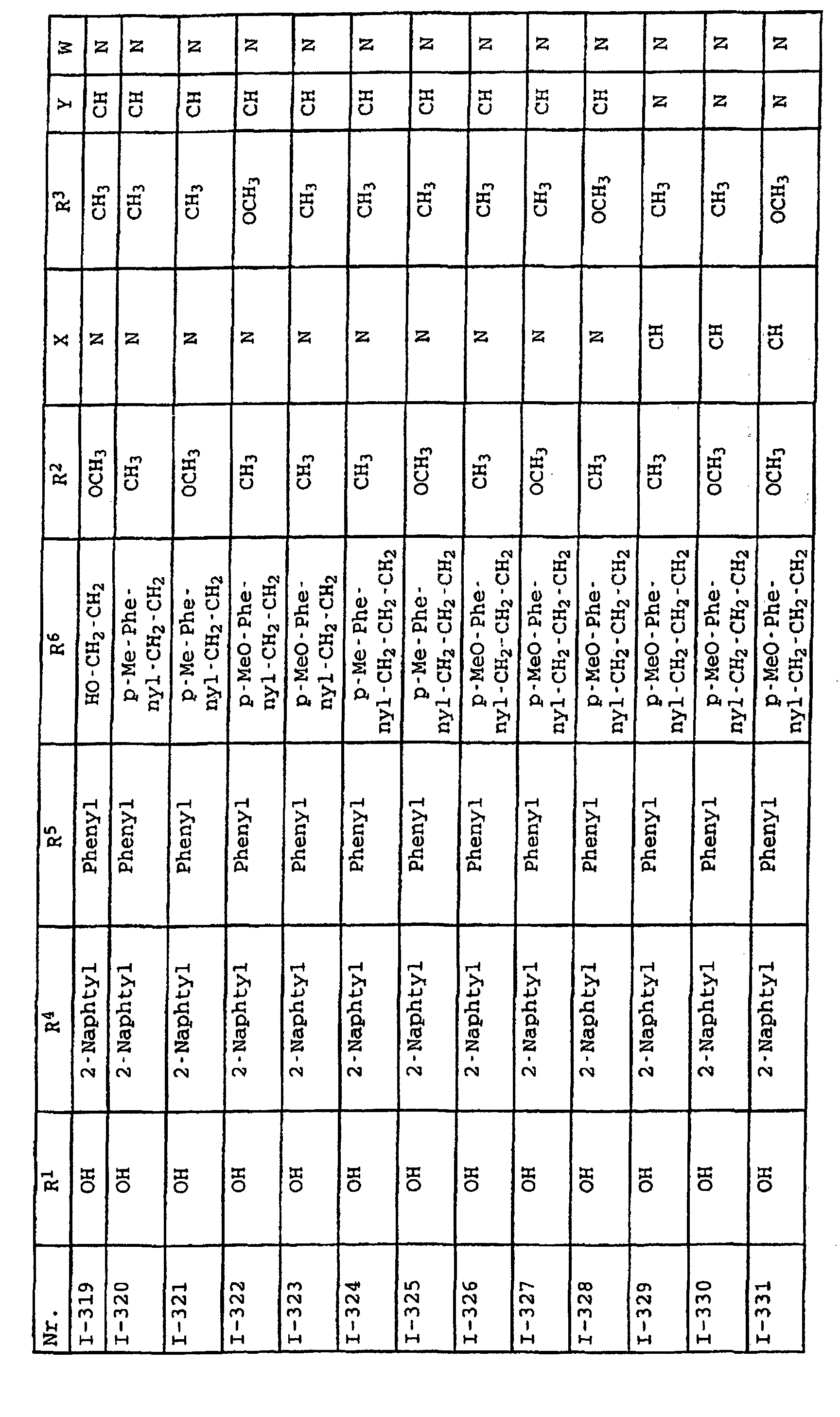

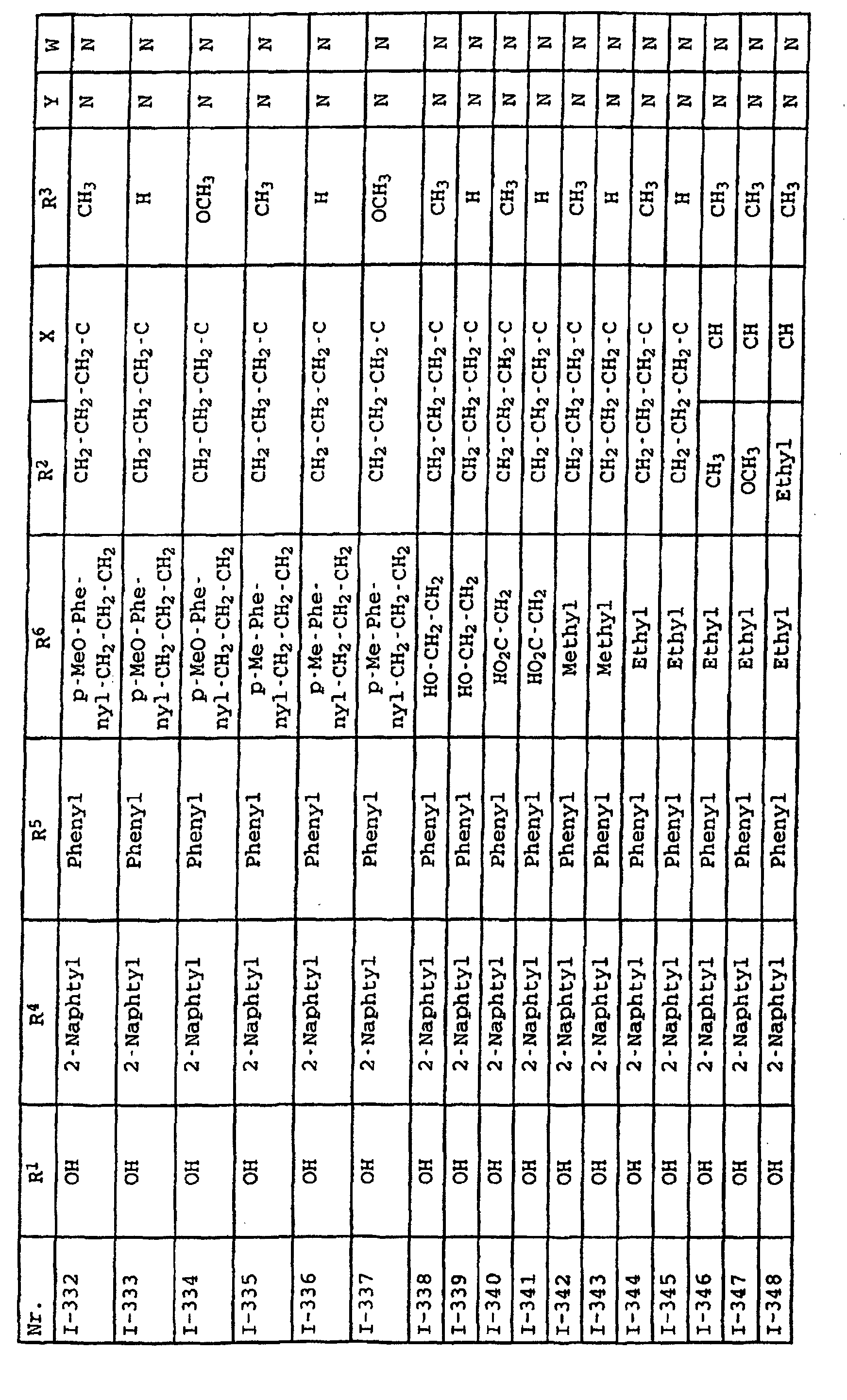

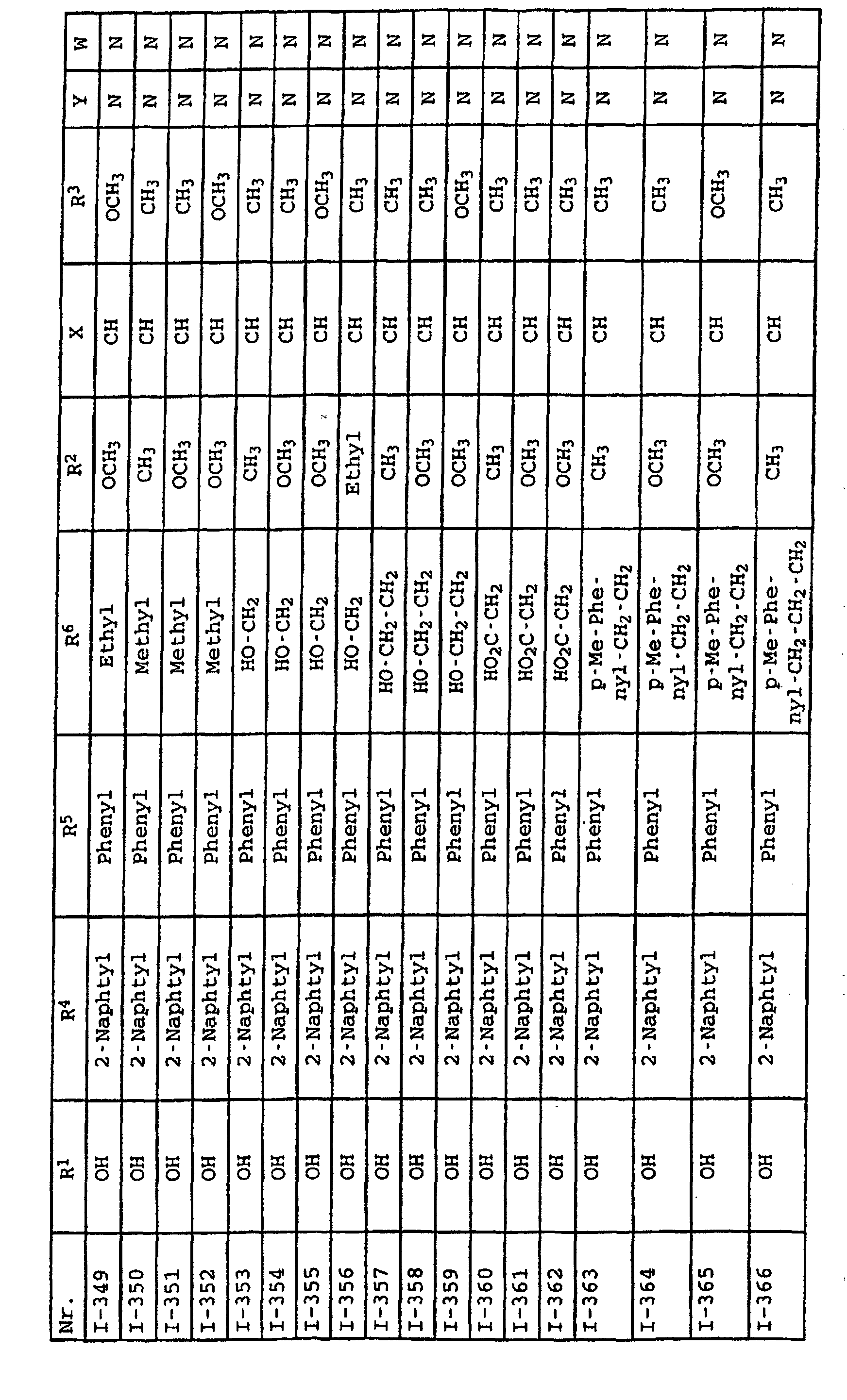

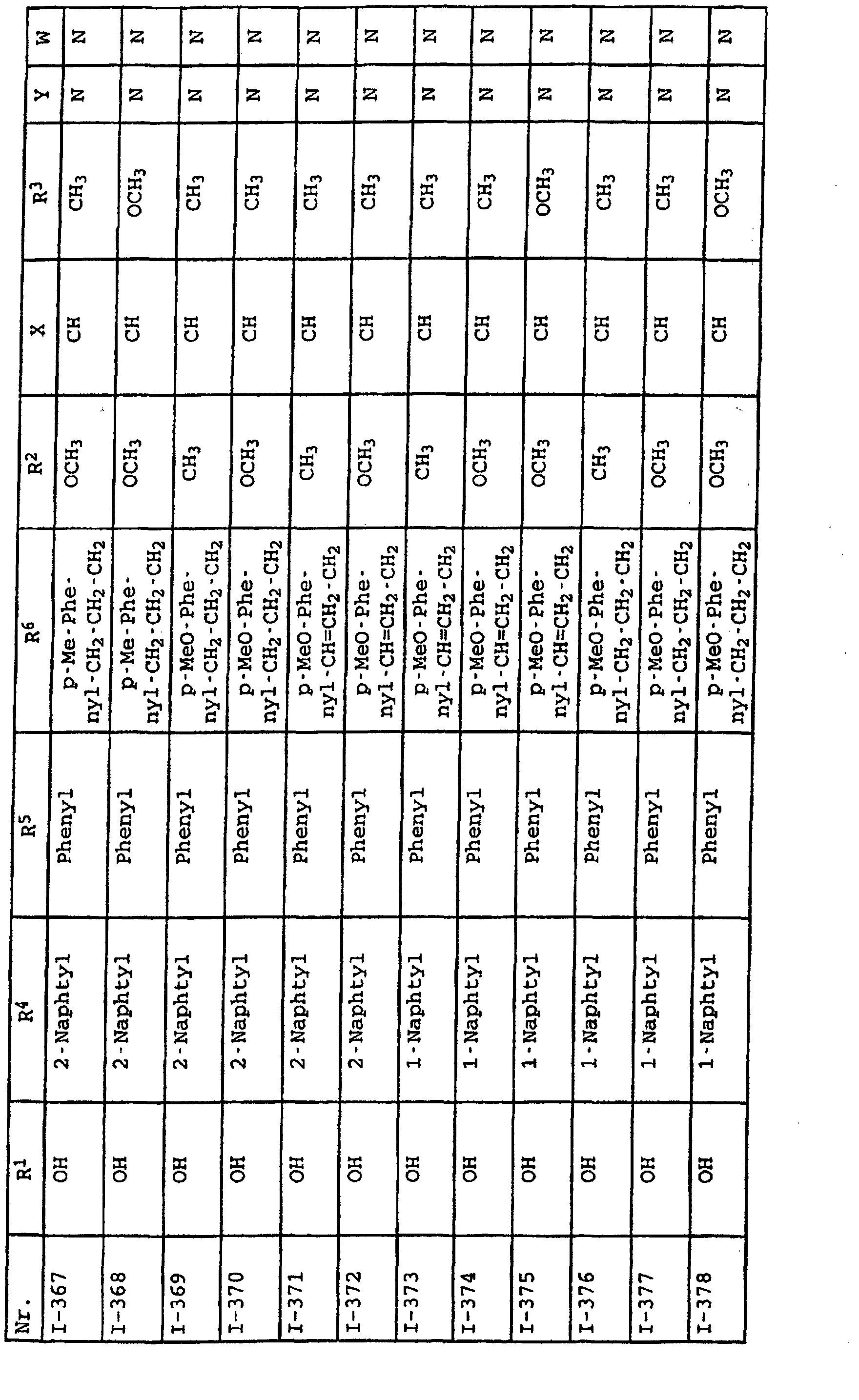

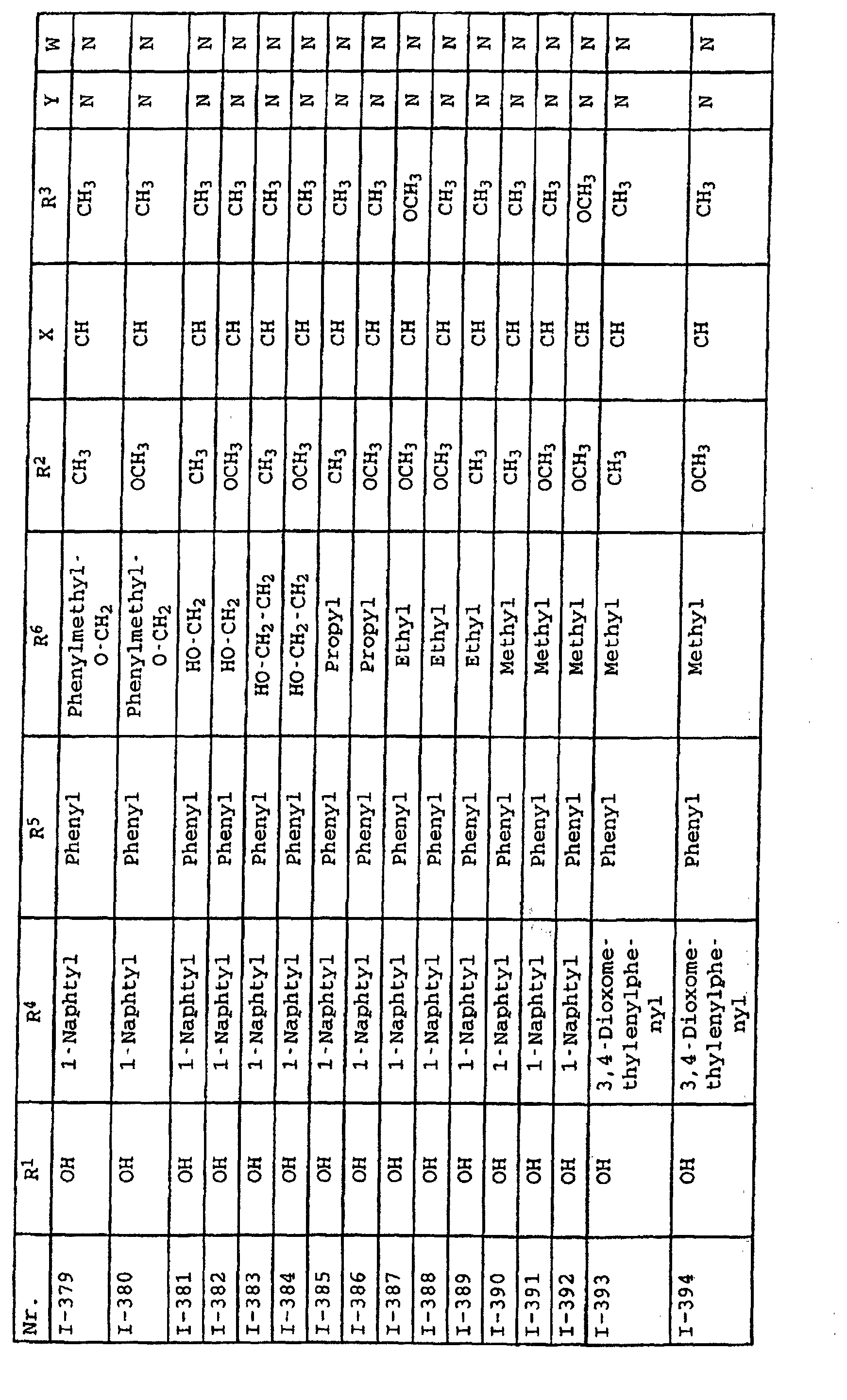

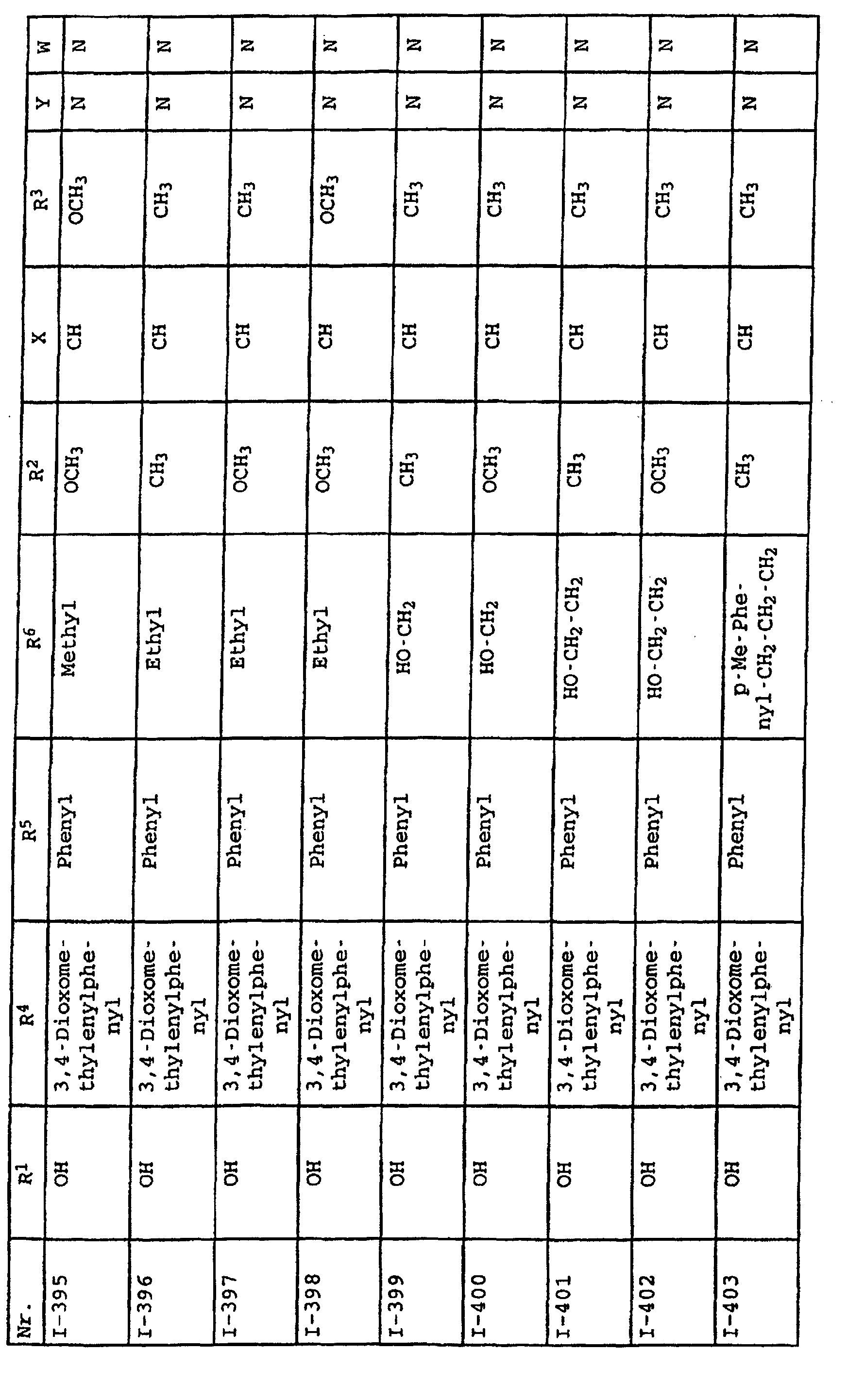

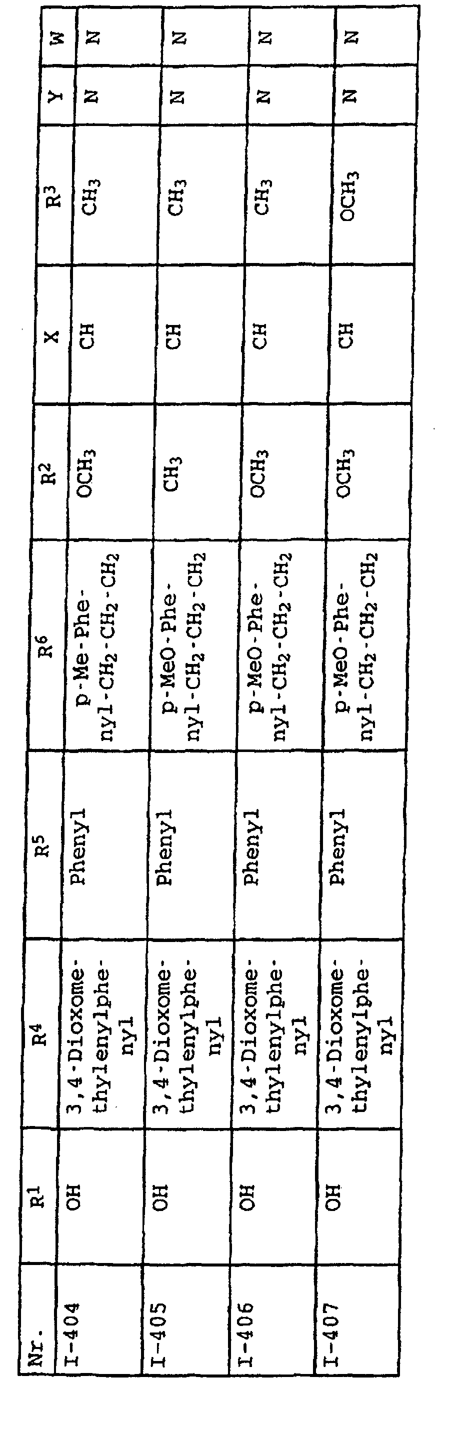

### Beispiel 16

Gemäß dem oben beschriebenen Bindungstest wurden für die nachfolgend aufgeführten Verbindungen Rezeptorbindungsdaten gemessen.

Die Ergebnisse sind in Tabelle 2 dargestellt.

**Tabelle 2**

| Rezeptorbindungsdaten (Kᵢ-Werte) | | |
|---|---|---|
| Verbindung | ET_{A} [nM/1] | ET_{B} [nM/1] |
| I-3 | 4 | 325 |
| I-3 Enantiomer I | 0,85 | 73 |
| I-3 Enantiomer II | 450 | >720 |
| I-1 | 25 | 950 |
| I-2 | 3.5 | 290 |
| I-11 | 20 | 1400 |
| I-12 | 4 | 250 |
| I-15 | 4 | 540 |
| I-20 | 5 | 445 |

## Patentansprüche

1. α-Hydroxycarbonsäurederivate der Formel I 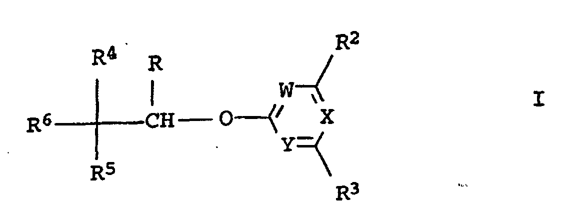 in der R für eine Gruppe 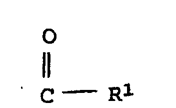 steht, in der R¹ folgende Bedeutung hat:
a) Wasserstoff
b) eine Succinylimidoxygruppe
c) ein über ein Stickstoffatom verknüpfter 5-gliedriger Heteroaromat wie Pyrrolyl, Pyrazolyl-, Imidazolyl und Triazolyl, welcher ein bis zwei Halogenatome oder ein bis zwei C₁-C₄-Alkyl- oder ein bis zwei C₁-C₄-Alkoxygruppen tragen kann;
d) eine Gruppe 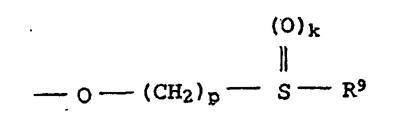 in der k die Werte 0,1 und 2, p die Werte 1,2,3 und 4 annehmen und R⁹ für
C₁-C₄-Alkyl, C₃-C₇-Cycloalkyl, C₃-C₆-Alkenyl,
C₃-C₆-Alkinyl oder gegebenenfalls substituiertes Phenyl steht, das durch einen oder mehrere, der folgenden Reste substituiert sein kann:
Halogen, Nitro, Cyano, C₁-C₄-Alkyl, C₁-C₄-Halogenalkyl, Hydroxy, C₁-C₄-Alkoxy, C₁-C₄-Alkylthio, Mercapto, Amino, C₁-C₄-Alkylamino, C₁-C₄-Dialkylamino;
e) ein Rest OR¹⁰, worin R¹⁰ bedeutet:
Wasserstoff, das Kation eines Alkalimetalls oder das Kation eines Erdalkalimetalls sowie physiologisch verträgliches Alkylammoniumion oder das Ammoniumion;
C₃-C₈-Cycloalkyl,
C₁-C₈-Alkyl,
CH₂-Phenyl, das durch einen oder mehrere der folgenden Reste substituiert sein kann: Halogen, Nitro, Cyano, C₁-C₄-Alkyl, C₁-C₄-Halogenalkyl, Hydroxy, C₁-C₄-Alkoxy, Mercapto, C₁-C₄-Alkylthio, Amino, C₁-C₄-Alkylamino, C₁-C₄-Dialkylamino,
eine C₃-C₆-Alkenyl, eine C₃-C₆-Alkinylgruppe, wobei diese Gruppe ihrerseits ein bis fünf Halogenatome tragen können;
R¹⁰ kann weiterhin ein Phenylrest sein, welcher ein bis fünf Halogenatome und/oder ein bis drei der folgende Reste tragen kann: Nitro, Cyano, C₁-C₄-Alkyl, C₁-C₄-Halogenalkyl, Hydroxy, C₁-C₄-Alkoxy, Mercapto, C₁-C₄-Alkylthio, Amino, C₁-C₄-Alkylamino, C₁-C₄-Dialkylamino;
ein über ein Stickstoffatom verknüpfter 5-gliedriger Heteroaromat, enthaltend ein bis drei Stickstoffatome, welcher ein bis zwei Halogenatome und/oder ein bis zwei der folgenden Reste tragen kann: C₁-C₄-Alkyl, C₁-C₄-Halogenalkyl, C₁-C₄-Alkoxy, Phenyl, C₁-C₄-Halogenalkoxy und/oder C₁-C₄-Alkylthio,
f) ein Rest 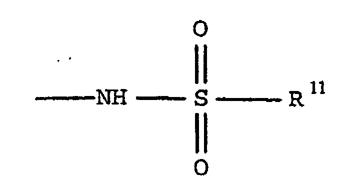 worin R¹¹ bedeutet:
C₁-C₄-Alkyl, C₃-C₆-Alkenyl, C₃-C₆-Alkinyl, C₃-C₈-Cycloalkyl, wobei diese Reste einen C₁-C₄-Alkoxy-, C₁-C₄-Alkylthio- und/oder einen Phenylrest tragen können;
Phenyl, gegebenenfalls substituiert;
g) ein Rest 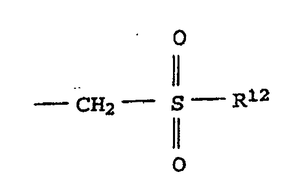 worin R¹² die gleiche Bedeutung hat wie R¹¹;
h) ein Rest 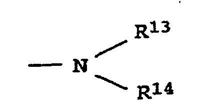
wobei R¹³ und R¹⁴ gleich oder verschieden sein können und folgende Bedeutung haben:
Wasserstoff, C₁-C₇-Alkyl, C₃-C₇-Cycloalkyl, C₃-C₇-Alkenyl, C₃-C₇-Alkinyl, Benzyl, Phenyl, gegebenenfalls substituiert,
oder R¹³ und R¹⁴ bilden gemeinsam eine zu einem Ring geschlossene, gegebenenfalls durch C₁-C₄-Alkyl substituierte C₄-C₇-Alkylenkette, die ein Heteroatom enthalten kann;
oder R ein Tetrazol oder ein Nitril sein;
W Stickstoff, C-NO₂, eine CH-Gruppe, wenn ein oder mehrere der Substituenten R², R³, R¹⁵ und/oder R¹⁶ eine Nitrogruppe bedeuten, oder wenn X und/oder Y Stickstoff bedeuten;
R² Wasserstoff, Halogen, C₁-C₄-Alkyl, C₁-C₄-Halogenalkyl, C₁-C₄-Alkoxy, C₁-C₄-Halogen-alkoxy, Hydroxy, Mercapto, C₁-C₄-Alkylthio, Nitro, Amino, C₁-C₄-Alkylamino oder C₁-C₄-Dialkylamino, Cyano, Phenyl, optional ein- bis dreifach substituiert mit Halogen, Hydroxy, Amino, Mono- oder Dialkyl (C₁-C₃)-Amino, C₁-C₃-Alkyl, C₁-C₃-Alkoxy, Mercapto oder C₁-C₃-Alkylthio, Carboxyl, C₁-C₃-Alkylcarboxyl;
ein fünf- oder sechsgliedriger Heteroaromat, enthaltend ein bis drei Stickstoffatome und/oder ein Schwefel- oder Sauerstoffatom;
R² kann mit dem benachbarten Kohlenstoffatom und X einen 5- oder 6-gliedrigen Alkylen- oder Alkylidenring bilden, worin jeweils ein oder zwei Kohlenstoffatome durch ein Heteroatom wie Stickstoff, Schwefel oder Sauerstoff ersetzt sein kann und der ein- bis dreifach durch folgende Reste substituiert sein kann: Halogen, Nitro, Cyano, Hydroxy, Mercapto, C₁-C₃-Alkyl, C₁-C₃-Halogenalkyl, C₁-C₃-Alkoxy, C₁-C₃-Alkylthio, Amino, C₁-C₃-Alkylamino, C₁-C₃-Dialkylamino;
X Stickstoff oder CR¹⁵, worin R¹⁵ Wasserstoff oder C₁-C₅-Alkyl, optional ein- bis zweimal substituiert mit Hydroxy oder Carboxy; C₁-C₆-Alkoxy, C₁-C₆-Alkylthio, Phenyl, Benzyl, Hydroxy, Mercapto, Nitro, Amino, C₁-C₄-Alkylamino, C₁-C₄-Dialkylamino, Cyano oder Carboxy bedeutet;
CR¹⁵ kann mit R² zu einem 5- oder 6-gliedrigen Ring verknüpft sein, wie oben beschrieben, oder CR¹⁵ kann mit R³ und dessen benachbartem Kohlenstoffatom einen 5- oder 6-gliedrigen Alkylen- oder Alkylidenring bilden, worin jeweils ein oder zwei Kohlenstoffatome durch Stickstoff, Sauerstoff oder Schwefel ersetzt sein kann, der 5- oder 6-gliedrige Ring kann optional ein- bis dreifach mit folgenden Resten substituiert sein:
Halogen, Nitro, Cyano, Hydroxy, Mercapto, C₁-C₃-Alkyl, C₁-C₃-Halogenalkyl, C₁-C₃-Alkoxy, C₁-C₃-Alkylthio, Amino, C₁-C₃-Alkylamino, C₁-C₃-Dialkylamino oder Carboxy; Stickstoff im 5-Ring kann auch durch eine Formyl- oder Acetylgruppe substituiert sein;
R³ kann dieselbe Bedeutung haben wie R², R² und R³ können gleich oder verschieden sein; ferner kann R³ mit dem benachbarten Kohlenstoffatom und mit X einen 5- oder 6-gliedrigen Ring bilden, wie oben beschrieben; ferner kann R³ mit dem benachbarten Kohlenstoffatom und Y zusammen einen 5- oder 6-gliedrigen Alkylen- oder Alkylidenring bilden, worin jeweils ein oder zwei Kohlenstoffatome durch Stickstoff, Sauerstoff oder Schwefel ersetzt sein kann; der 5- oder 6-gliedrige Ring kann optional ein- bis dreifach mit folgenden Resten substituiert sein:
Halogen, Nitro, Cyano, Hydroxy, Mercapto, C₁-C₃-Alkyl, C₁-C₃-Halogenalkyl, C₁-C₃-Alkoxy, C₁-C₃-Alkylthio, Amino, C₁-C₃-Alkylamino, C₁-C₃-Dialkylamino oder Carboxy; Stickstoff im 5-Ring kann auch durch eine Formyl- oder Acetylgruppe substituiert sein;
Y Stickstoff oder CR¹⁶, worin R¹⁶ Wasserstoff, C₁-C₅-Alkyl, C₁-C₅-Alkoxy, C₁-C₅-Alkylthio, Nitro, Phenyl, Hydroxy, Halogen, Cyano, Amino, C₁-C₄-Alkylamino, C₁-C₄-Dialkylamino, Mercapto oder Carboxy bedeutet oder CR¹⁶ zusammen mit R³ und dessen benachbarten Kohlenstoffatom einen 5- oder 6-gliedrigen Ring bilden, wie oben beschrieben;
R⁴ Phenyl, Naphthyl, Dihydro- oder Tetrahydronaphthyl, das durch einen oder mehrere der folgenden Reste substituiert sein kann:
Halogen, Nitro, Cyano, Hydroxy, C₁-C₄-Alkyl, C₁-C₄-Halogenalkyl, C₁-C₄-Alkoxy, C₁-C₄-Halogenalkoxy, Phenoxy, Phenyl, C₁-C₄-Alkylthio, Amino, C₁-C₄-Alkylamino oder C₁-C₄-Dialkylamino, wobei zwei Reste an benachbarten Kohlenstoffatomen zusammen mit diesem über eine Alkylen- oder Alkylidengruppe verbundenen fünf- oder sechsgliedrigen Ring bilden können, bei dem ein oder mehrere Methylen oder Methylidengruppen durch Sauerstoff ersetzt sein können;
R⁵ kann dieselbe Bedeutung haben wie R⁴, wobei R⁴ und R⁵ gleich oder verschieden sein können;
R⁶ Wasserstoff, C₁-C₈-Alkyl, C₂-C₈-Alkenyl oder C₃-C₈-Alkinyl, wobei diese Reste jeweils ein- oder mehrfach substituiert sein können durch: Halogen, Nitro, Cyano, C₁-C₄-Alkoxy, Hydroxy, C₁-C₄-Alkylthio, Mercapto, C₁-C₄-Halogenalkoxy, Carboxy, C₁-C₄-Alkylcarboxy, C₁-C₄-Alkylcarbonyl, Amino, C₁-C₄-Alkylamino, C₁-C₄-Dialkylamino oder Phenyl, bzw. Naphthyl, das seinerseits ein- oder mehrfach substituiert sein kann durch: Halogen, Nitro, Cyano, Hydroxy, C₁-C₄-Alkoxy, C₁-C₄-Alkyl, C₁-C₄-Halogenalkyl, C₁-C₄-Halogenalkoxy, Mercapto, C₁-C₄-Alkylthio, Amino, C₁-C₄-Alkylamino, C₁-C₄-Dialkylamino oder Phenoxy.
C₁-C₄-Alkyl, das durch Phenylmethoxy oder Phenoxy substituiert ist, worin die Phenylgruppe ein- oder zweifach durch Halogen, Methyl oder Methoxy substituiert sein kann;
C₁-C₈-Alkyl, C₃-C₈-Alkenyl- oder C₃-C₈-Alkinylkette, die durch einen der folgenden Reste substituiert ist:
Heteroaryl oder Heteroaryloxy, fünf- oder sechsgliedriger, enthaltend ein bis drei Stickstoffatome und/oder ein Schwefel- oder Sauerstoffatom, welcher ein bis vier Halogenatome und/oder einen bis zwei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
Phenyl, Naphthyl, das seinerseits ein- oder mehrfach substituiert sein kann durch: Halogen, Nitro, Cyano, Hydroxy, C₁-C₄-Alkoxy, C₁-C₄-Alkyl, C₁-C₄-Halogenalkyl, C₁-C₄-Halogenalkoxy, Mercapto, C₁-C₄-Alkylthio, Amino, C₁-C₄-Alkylamino, C₁-C₄-Dialkylamino oder Phenoxy;
oder einen der folgenden Reste: 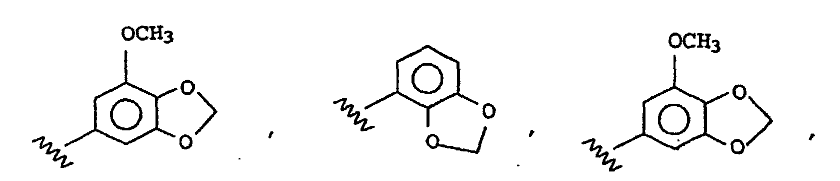 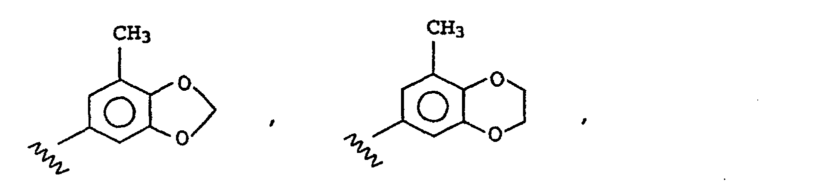 bedeuten.

2. α-Hydroxycarbonsäurederivate nach Anspruch 1, **dadurch gekennzeichnet, daß** R=COOH bedeutet.

3. α-Hydroxycarbonsäurederivate nach einem der obenstehenden Ansprüche, **dadurch gekennzeichnet, daß** mindestens einer der Reste R⁴ und R⁵ Phenyl bedeutet.

4. α-Hydroxycarbonsäurederivate nach Anspruch 3, **dadurch gekennzeichnet, daß** R⁴ und R⁵ beide Phenyl bedeuten.

5. α-Hydroxycarbonsäurederivate nach einem der obenstehenden Ansprüche, **dadurch gekennzeichnet, daß** R⁶=C₁-C₈-Alkyl, gegebenenfalls substituiert durch OH, C₁-C₄-Alkoxy oder gegebenenfalls substituiertes Phenyl bedeutet.

6. α-Hydroxycarbonsäurederivate nach einem der obenstehenden Ansprüche, **dadurch gekennzeichnet, daß** X=CH bedeutet.

7. α-Hydroxycarbonsäurederivate nach einem der obenstehenden Ansprüche, **dadurch gekennzeichnet, daß** mindestens einer der Reste W, Y Stickstoff (N) bedeutet.

8. α-Hydroxycarbonsäurederivate nach einem der obenstehenden Ansprüche, **dadurch gekennzeichnet, daß** mindestens einer der Reste R², R³ C₁-C₄-Alkyl bedeutet.

9. Verwendung von Verbindungen gemäß Anspruch 1 bis 8 zur Herstellung von Medikamenten zur Behandlung von Hypertonie, pulmonalem Hochdruck, akutem und chronischem Nierenversagen, chronischer Herzinsuffizienz, zerebraler Ischämie, Restenose nach Angioplastie, Prostatakrebs.

10. Kombination einer Verbindung gemäß Anspruch 1 bis 8 mit einem Inhibitor des Renin-Angiotensin-Systems (RAS).

11. Verwendung einer Kombination einer Verbindung gemäß Anspruch 1 bis 8 mit einem Inhibitor des Renin-Angiotensin-Systems (RAS) zur Herstellung von Medikamenten zur Behandlung von Hypertonie, jpulmonalem Hochdruck, Myokardinfarkt, Angina Pectoris, akutem Nierenversagen, Niereninsuffizienz, zerebralen Vasospasmen, zerebraler Ischämie, Subarachnoidalblutungen, Migräne, Asthma, Atherosklerose, endotoxischem Schock, Endotoxin-induziertem Organversagen, intravaskulärer Koagulation, Restenose nach Angioplastie, benigne Prostata-Hyperplasie, ischämisches und durch Intoxikation verursachtes Nierenversagen bzw. Hypertonie sowie von Krebserkrankungen, Prostatakrebs und Hautkrebs.

## Claims

1. α-Hydroxycarboxylic acid derivatives of the formula I 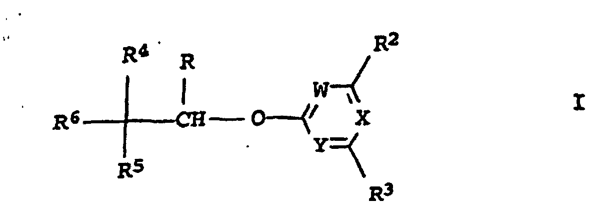 in which R represents a group 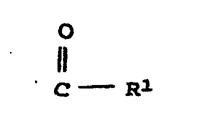 in which R¹ has the following meaning:
a) hydrogen
b) a succinylimidoxy group
c) a 5-membered heteroaromatic which is linked via a nitrogen atom, such as pyrrolyl, pyrazolyl-,imidazolyl and triazolyl, and which can carry one to two halogen atoms or one to two C₁-C₄-alkyl or one to two C₁-C₄-alkoxy groups;
d) a group 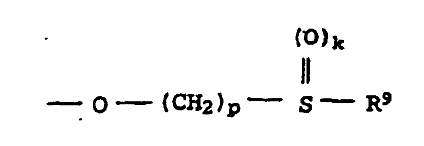 in which k assumes the values 0, 1 and 2, p assumes the values 1, 2, 3 and 4 and R⁹ represents C₁-C₄-alkyl, C₃-C₇-cycloalkyl, C₃-C₆-alkenyl, C₃-C₆-alkinyl or optionally substituted phenyl, which can be substituted by one or more of the following radicals:
halogen, nitro, cyano, C₁-C₄-alkyl, C₁-C₄-halogenoalkyl, hydroxyl, C₁-C₄-alkoxy, C₁-C₄-alkylthio, mercapto, amino, C₁-C₄-alkylamino, C₁-C₄-dialkylamino;
e) a radical OR¹⁰, wherein R¹⁰ denotes:
hydrogen, the cation of an alkali metal or the cation of an alkaline earth metal and a physiologically acceptable alkylammonium ion or the ammonium ion;
C₃-C₈-cycloalkyl,
C₁-C₈-alkyl,
CH₂-phenyl, which can be substituted by one or more of the following radicals: halogen, nitro, cyano, C₁-C₄-alkyl, C₁-C₄-halogenoalkyl, hydroxyl, C₁-C₄-alkoxy, mercapto, C₁-C₄-alkylthio, amino, C₁-C₄-alkylamino, C₁-C₄-dialkylamino,
a C₃-C₆-alkenyl, a C₃-C₆-alkinyl group, wherein these groups in their turn can carry one to five halogen atoms;
R¹⁰ can furthermore be a phenyl radical, which can carry one to five halogen atoms and/or one to three of the following radicals: nitro, cyano, C₁-C₄-alkyl, C₁-C₄-halogenoalkyl, hydroxyl, C₁-C₄-alkoxy, mercapto, C₁-C₄-alkylthio, amino, C₁-C₄-alkylamino, C₁-C₄-dialkylamino;
a 5-membered heteroaromatic containing one to three nitrogen atoms which is linked via a nitrogen atom and can carry one to two halogen atoms and/or one to two of the following radicals: C₁-C₄-alkyl, C₁-C₄-halogenoalkyl, C₁-C₄-alkoxy, phenyl, C₁-C₄-halogenoalkoxy and/or C₁-C₄-alkylthio,
f) a radical 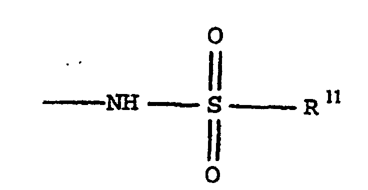 wherein R¹¹ denotes:
C₁-C₄-alkyl, C₃-C₆-alkenyl, C₃-C₆-alkinyl, C₃-C₈-cycloalkyl, wherein these radicals can carry a C₁-C₄-alkoxy, C₁-C₄-alkylthio and/or a phenyl radical;
phenyl, optionally substituted;
g) a radical 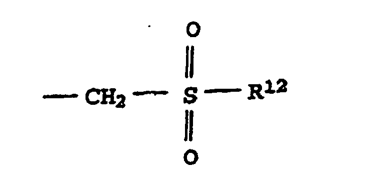 wherein R¹² has the same meaning as R¹¹;
h) a radical 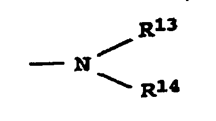 wherein R¹³ and R¹⁴ can be identical or different and have the following meaning:
hydrogen, C₁-C₇-alkyl, C₃-C₇-cycloalkyl, C₃-C₇-alkenyl, C₃-C₇-alkinyl, benzyl, phenyl, optionally substituted,
or R¹³ and R¹⁴ together form a C₄-C₇-alkylene chain which is closed to form a ring, is optionally substituted by C₁-C₄-alkyl and can contain a heteroatom;
or R is a tetrazole or a nitrile;
W denotes nitrogen, C-NO₂, a CH group, if one or more of the substituents R², R³, R¹⁵ and/or R¹⁶ denote a nitro group, or if X and/or Y denote nitrogen;
R² denotes hydrogen, halogen, C₁-C₄-alkyl, C₁-C₄-halogenoalkyl, C₁-C₄-alkoxy, C₁-C₄-halogenoalkoxy, hydroxyl, mercapto, C₁-C₄-alkylthio, nitro, amino, C₁-C₄-alkylamino or C₁-C₄-dialkylamino, cyano, phenyl, optionally mono- to trisubstituted by halogen, hydroxyl, amino, mono- or dialkyl(C₁-C₃)-amino, C₁-C₃-alkyl, C₁-C₃-alkoxy, mercapto or C₁-C₃-alkylthio, carboxyl, C₁-C₃-alkylcarboxyl; a five- or six-membered heteroaromatic, containing one to three nitrogen atoms and/or a sulfur or oxygen atom;
R² with the adjacent carbon atom and X can form a 5- or 6-membered alkylene or alkylidene ring, wherein in each case one or two carbon atoms can be replaced by a heteroatom, such as nitrogen, sulfur or oxygen, and which can be mono- to trisubstituted by the following radicals: halogen, nitro, cyano, hydroxyl, mercapto, C₁-C₃-alkyl, C₁-C₃-halogenoalkyl, C₁-C₃-alkoxy, C₁-C₃-alkylthio, amino, C₁-C₃-alkylamino, C₁-C₃-dialkylamino;
X denotes nitrogen or CR¹⁵, wherein R¹⁵ denotes hydrogen or C₁-C₅-alkyl, optionally mono- to disubstituted by hydroxyl or carboxyl; C₁-C₆-alkoxy, C₁-C₆-alkylthio, phenyl, benzyl, hydroxyl, mercapto, nitro, amino, C₁-C₄-alkylamino, C₁-C₄-dialkylamino, cyano or carboxyl;
CR¹⁵ can be linked to R² to form a 5- or 6- membered ring, as described above, or CR¹⁵ with R³ and its adjacent carbon atom can form a 5- or 6- membered alkylene or alkylidene ring, wherein in each case one or two carbon atoms can be replaced by nitrogen, oxygen or sulfur, the 5- or 6- membered ring can be optionally mono- to trisubstituted by the following radicals:
halogen, nitro, cyano, hydroxyl, mercapto, C₁-C₃-alkyl, C₁-C₃-halogenoalkyl, C₁-C₃-alkoxy, C₁-C₃-alkylthio, amino, C₁-C₃-alkylamino, C₁-C₃-dialkylamino or carboxyl; nitrogen in the 5- membered ring can also be substituted by a formyl or acetyl group;
R³ can have the same meaning as R², R² and R³ can be identical or different; furthermore R³ with the adjacent carbon atom and with X can form a 5- or 6- membered ring, as described above; furthermore R³ with the adjacent carbon atom and Y together form a 5- or 6-membered alkylene or alkylidene ring, wherein in each case one or two carbon atoms can be replaced by nitrogen, oxygen or sulfur; the 5- or 6-membered ring can be optionally mono- to trisubstituted by the following radicals: halogen, nitro, cyano, hydroxyl, mercapto, C₁-C₃-alkyl, C₁-C₃-halogenoalkyl, C₁-C₃-alkoxy, C₁-C₃-alkylthio, amino, C₁-C₃-alkylamino, C₁-C₃-dialkylamino or carboxyl; nitrogen in the 5-membered ring can also be substituted by a formyl or acetyl group;
Y denotes nitrogen or CR¹⁶, wherein R¹⁶ denotes hydrogen, C₁-C₅ alkyl, C₁-C₅-alkoxy, C₁-C₅-alkylthio, nitro, phenyl, hydroxyl, halogen, cyano, amino, C₁-C₄-alkylamino, C₁-C₄-dialkylamino, mercapto or carboxyl or CR¹⁶ together with R³ and its adjacent carbon atom form a 5- or 6-membered ring, as described above;
R⁴ denotes phenyl, naphthyl, dihydro- or tetrahydronaphthyl, which can be substituted by one or more of the following radicals: halogen, nitro, cyano, hydroxyl, C₁-C₄-alkyl, C₁-C₄-halogenoalkyl, C₁-C₄-alkoxy, C₁-C₄-halogenoalkoxy, phenoxy, phenyl, C₁-C₄-alkylthio, amino, C₁-C₄-alkylamino or C₁-C₄-dialkylamino, wherein two radicals on adjacent carbon atoms together with this can form a five- or six- membered ring bonded via an alkylene or alkylidene group, in which one or more methylene or methylidene groups can be replaced by oxygen;
R⁵ can have the same meaning as R⁴, wherein R⁴ and R⁵ can be identical or different;
R⁶ denotes hydrogen, C₁-C₈-alkyl, C₂-C₈-alkenyl or C₃-C₈-alkinyl, wherein these radicals in each case can be mono- or polysubstituted by: halogen, nitro, cyano, C₁-C₄-alkoxy, hydroxyl, C₁-C₄-alkylthio, mercapto, C₁-C₄-halogenoalkoxy, carboxyl, C₁-C₄-alkylcarboxy, C₁-C₄-alkylcarbonyl, amino, C₁-C₄-alkylamino, C₁-C₄-dialkylamino or phenyl, or naphthyl, which in its turn can be mono- or polysubstituted by: halogen, nitro, cyano, hydroxyl, C₁-C₄-alkoxy, C₁-C₄-alkyl, C₁-C₄-halogenoalkyl, C₁-C₄-halogenoalkoxy, mercapto, C₁-C₄-alkylthio, amino, C₁-C₄-alkylamino, C₁-C₄-dialkylamino or phenoxy,
C₁-C₄-alkyl, which is substituted by phenylmethoxy or phenoxy, wherein the phenyl group can be mono- or disubstituted by halogen, methyl or methoxy;
C₁-C₈-alkyl, C₃-C₈-alkenyl or C₃-C₈-alkinyl chain, which is. substituted by one of the following radicals:
heteroaryl or heteroaryloxy, five- or six- membered, containing one to three nitrogen atoms and/or a sulfur or oxygen atom, which can carry one to four halogen atoms and/or one to two of the following radicals: C₁-C₄-alkyl, C₁-C₄-halogenoalkyl, C₁-C₄-alkoxy, C₁-C₄-halogenoalkoxy, C₁-C₄-alkylthio, phenyl, phenoxy or phenylcarbonyl, wherein the phenyl radicals in their turn can carry one to five halogen atoms and/or one to three of the following radicals: C₁-C₄-alkyl, C₁-C₄-halogenoalkyl, C₁-C₄-alkoxy, C₁-C₄-halogenoalkoxy and/or C₁-C₄-alkylthio;
phenyl, naphthyl, which in its turn can be mono- or polysubstituted by: halogen, nitro, cyano, hydroxyl, C₁-C₄-alkoxy, C₁-C₄-alkyl, C₁-C₄-halogenoalkyl, C₁-C₄-halogenoalkoxy, mercapto, C₁-C₄-alkylthio, amino, C₁-C₄-alkylamino, C₁-C₄-dialkylamino or phenoxy;
or one of the following radicals:

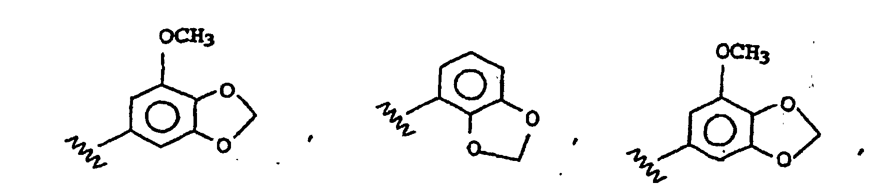

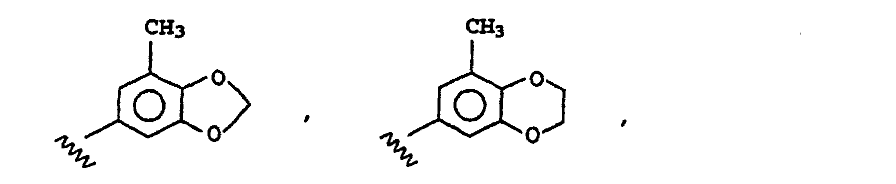

2. α-Hydroxycarboxylic acid derivatives according to claim 1, **characterized in that** R denotes COOH.

3. α-Hydroxycarboxylic acid derivatives according to one of the above claims, **characterized in that** at least one of the radicals R⁴ and R⁵ denotes phenyl.

4. α-Hydroxycarboxylic acid derivatives according to claim 3, **characterized in that** R⁴ and R⁵ both denote phenyl.

5. α-Hydroxycarboxylic acid derivatives according to one of the above claims, **characterized in that** R⁶ denotes C₁-C₈-alkyl, optionally substituted by OH, C₁-C₄-alkoxy or optionally substituted phenyl.

6. α-Hydroxycarboxylic acid derivatives according to one of the above claims, **characterized in that** X denotes CH.

7. α-Hydroxycarboxylic acid derivatives according to one of the above claims, **characterized in that** at least one of the radicals W, Y denotes nitrogen (N).

8. α-Hydroxycarboxylic acid derivatives according to one of the above claims, **characterized in that** at least one of the radicals R², R³ denotes C₁-C₄-alkyl.

9. Use of compounds according to claim 1 to 8 for the preparation of medicaments for treatment of hypertension, pulmonary hypertension, acute and chronic renal failure, chronic cardiac insufficiency, cerebral ischaemia, restenosis following angioplasty, prostate cancer.

10. Combination of a compound according to claim 1 to 8 with an inhibitor of the renin-angiotensin system (RAS).

11. Use of a combination of a compound according to claim 1 to 8 with an inhibitor of the renin-angiotensin system (RAS) for the preparation of medicaments for treatment of hypertension, pulmonary hypertension, myocardial infarction, angina pectoris, acute renal failure, renal insufficiency, cerebral vasospasms, cerebral ischaemia, subarachnoidal haemorrhages, migraine, asthma, atherosclerosis, endotoxic shock, endotoxin-induced organ failure, intravascular coagulation, restenosis following angioplasty, benign prostate hyperplasia, ischaemic and intoxication-induced renal failure or hypertension, and cancer diseases, prostate cancer and skin cancer.

## Revendications

1. Dérivés d'acides α-hydroxycarboxyliques de formule I 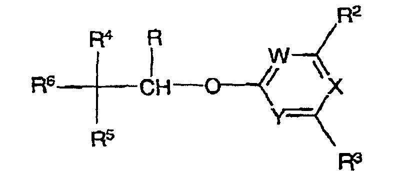 dans laquelle R représente un groupe 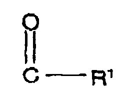 dans lequel R¹ a les significations suivantes :
a) l'hydrogène
b) un groupe succinylimidoxy
c) un cycle hétéroaromatique à 5 chaînons relié par l'intermédiaire d'un atome d'azote, tel que pyrrolyle, pyrazolyle, imidazolyle et triazolyle, qui peut porter 1 à 2 atomes d'halogènes ou 1 à 2 groupes alkyle en C₁ à C₄ ou 1 à 2 groupes alcoxy en C₁ à C₄ ;
d) un groupe 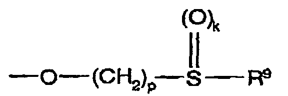 dans lequel k est égal à 0, 1 ou 2, p est égal à 1, 2, 3 ou 4 et R⁹ représente
un groupe alkyle en C₁ à C₄, cycloalkyle en C₃ à C₇, alcényle en C₃ à C₆, alcynyle en C₃ à C₆ ou phényle éventuellement substitué, qui peut porter un ou plusieurs des substituants suivants :
halogéno, nitro, cyano, alkyle en C₁ à C₄, halogénoalkyle en C₁ à C₄, l'hydroxy, alcoxy en C₁ à C₄, alkylthio en C₁ à C₄, mercapto, amino, alkylamino en C₁ à C₄, di(alkyle en C₁ à C₄)amino ;
e) un groupe OR¹⁰ dans lequel R¹⁰ représente :
l'hydrogène, le cation d'un métal alcalin ou le cation d'un métal alcalino-terreux ou un ion alkylammonium acceptable pour les usages pharmaceutiques, ou l'ion ammonium ;
un groupe cycloalkyle en C₃ à C₈,
un groupe alkyle en C₁ à C₈,
un groupe CH₂-phényle qui peut porter un ou plusieurs des substituants suivants: halogéno, nitro, cyano, alkyle en C₁ à C₄, halogénoalkyle en C₁ à C₄, hydroxy, alcoxy en C₁ à C₄, mercapto, alkylthio en C₁ à C₄, amino, alkylamino en C₁ à C₄, di(alkyle en C₁ à C₄)amino,
un groupe alcényle en C₃ à C₆, un groupe alcynyle en C₃ à C₆, ces groupes pouvant porter 1 à 5 atomes d'halogènes ;
R¹⁰ peut en outre représenter un groupe phényle qui peut porter 1 à 5 atomes d'halogènes et/ou 1 à 3 des substituants suivants : nitro, cyano, alkyle en C₁ à C₄, halogénoalkyle en C₁ à C₄, hydroxy, alcoxy en C₁ à C₄, mercapto, alkylthio en C₁ à C₄, amino, alkylamino en C₁ à C₄, di(alkyle en C₁ à C₄)amino ;
un cyde héteroaromatique à 5 chaînons, contenant 1 à 3 atomes d'azote, relié par l'intermédiaire d'un atome d'azote, et qui peut porter 1 à 2 atomes d'halogènes et/ou 1 à 2 des substituants suivants : alkyle en C₁ à C₄, halogénoalkyle en C₁ à C₄, alcoxy en C₁ à C₄, phényle, halogénoalcoxy en C₁ à C₄ et/ou alkylthio en C₁ à C₄,
f) un groupe 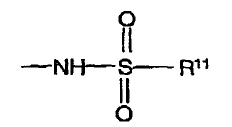 dans lequel R¹¹ représente :
un groupe alkyle en C₁ à C₄, alcényle en C₃ à C₆, alcynyle en C₃ à C₆, cycloalkyle en C₃ à C₈, chacun d'eux pouvant porter un substituant alcoxy en C₁ à C₄, alkylthio en C₁ à C₄ et/ou phényle ;
un groupe phényle, éventuellement substitué ;
g) un groupe 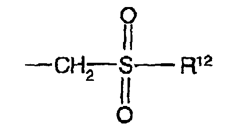 dans lequel R¹² a les mêmes significations que R¹¹ ;
h) un groupe 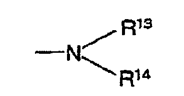 dans lequel R¹³ et R¹⁴, ayant des significations identiques ou différentes, représentent chacun :
l'hydrogène, un groupe alkyle en C₁ à C₇, cycloalkyle en C₃ à C₇, alcényle en C₃ à C₇, alcynyle en C₃ à C₇, benzyle, phényle, éventuellement substitué,
ou bien R¹³ et R¹⁴ forment ensemble une chaîne alkylène en C₄ à C₇ fermée en un cycle, éventuellement substituée par un groupe alkyle en C₁ à C₄ et qui peut contenir un hétéroatome ;
ou bien R représente un tétrazole ou un nitrile ;
W représente l'azote, C-NO₂, un groupe CH, lorsqu'un ou plusieurs des symboles R², R³, R¹⁵ et/ou R¹⁶ représentent un groupe nitro, ou bien lorsque X et/ou Y représentent l'azote ;
R² représente l'hydrogène, un halogène, un groupe alkyle en C₁ à C₄, halogénoalkyle en C₁ à C₄, alcoxy en C₁ à C₄, halogénoalcoxy en C₁ à C₄, hydroxy, mercapto, alkylthio en C₁ à C₄, nitro, amino, alkylamino en C₁ à C₄ ou di(alkyle en C₁ à C₄)amino, cyano, phényle, portant éventuellement un à trois substituants halogéno, hydroxy, amino, mono- ou di-(alkyle en C₁ à C₃)amino, alkyle en C₁ à C₃, alcoxy en C₁ à C₃, mercapto ou alkylthio en C₁ à C₃, carboxyle, (alkyle en C₁ à C₃)carboxyle ;
un cycle hétéroaromatique à 5 ou 6 chaînons contenant 1 à 3 atomes d'azote et/ou un atome de soufre ou d'oxygène ;
R² peut également former, avec l'atome de carbone voisin et X, un cycle alkylène ou alkylidène à 5 ou 6 chaînons dans chacun desquels 1 ou 2 atomes de carbone peuvent être remplacés par un hétéroatome tel que l'azote, le soufre ou l'oxygène, et pouvant porter 1 à 3 des substituants suivants : halogéno, nitro, cyano, hydroxy, mercapto, alkyle en C₁ à C₃, halogénoalkyle en C₁ à C₃, alcoxy en C₁ à C₃, alkylthio en C₁ à C₃, amino, alkylamino en C₁ à C₃, di(alkyle en C₁ à C₃)amino ;
X représente l'azote ou CR¹⁵ dans lequel R¹⁵ représente l'hydrogène ou un groupe alkyle en C₁ à C₅ portant éventuellement 1 à 2 substituants hydroxy ou carboxy ;
un groupe alcoxy en C₁ à C₆, alkylthio en C₁ à C₆, phényle, benzyle, hydroxy, mercapto, nitro, amino, alkylamino en C₁ à C₄, di(alkyle en C₁ à C₄)amino, cyano ou carboxy ;
CR¹⁵ peut être relié à R², formant un cycle à 5 ou 6 chaînons comme décrit ci-dessus, ou bien CR¹⁵ peut former avec R³ et l'atome de carbone voisin de celui-ci un cycle alkylène ou alkylidène à 5 ou 6 chaînons dans chacun desquels 1 ou 2 atomes de carbone peuvent être remplacés par l'azote, l'oxygène ou le soufre, le cycle à 5 ou 6 chaînons pouvant éventuellement porter 1 à 3 des substituants suivants :
halogèno, nitro, cyano, hydroxy, mercapto, alkyle en C₁ à C₃, halogénoalkyle en C₁ à C₃, alcoxy en C₁ à C₃, alkylthio en C₁ à C₃, amino, alkylamino en C₁ à C₃, di(alkyle en C₁ à C₃)amino ou carboxy ;
l'azote du cycle à 5 chaînons peut également porter un substituant formyle ou acétyle ;
R³ a les mêmes significations que R², mais R² et R³ peuvent avoir des significations identiques ou différentes ; en outre, R³ peut former avec l'atome de carbone voisin et avec X un cycle à 5 ou 6 chaînons comme décrit ci-dessus ; en outre, R³ peut former avec l'atome de carbone voisin et Y un cycle alkylène ou alkylidène à 5 ou 6 chaînons, dans chacun desquels 1 ou 2 atomes de carbone peuvent être remplacés par l'azote, l'oxygène ou le soufre ; le cycle à 5 ou 6 chaînons peut éventuellement porter 1 à 3 des substituants suivants :
halogéno, nitro, cyano, hydroxy, mercapto, alkyle en C₁ à C₃, halogénoalkyle en C₁ à C₃, alcoxy en C₁ à C₃, alkylthio en C₁ à C₃, amino, alkylamino en C₁ à C₃, di(alkyle en C₁ à C₃)amino ou carboxy ;
l'azote du cycle à 5 chaînons peut également porter un substituant formyle ou acétyle ;
Y représente l'azote ou CR¹⁶ dans lequel R¹⁶ représente l'hydrogène, un groupe alkyle en C₁ à C₅, alcoxy en C₁ à C₅, alkylthio en C₁ à C₅, nitro, phényle, hydroxy, un halogène, un groupe cyano, amino, alkylamino en C₁ à C₄, di(alkyle en C₁ à C₄)amino, mercapto ou carboxy, ou bien CR¹⁶ forme avec R³ et l'atome de carbone voisin de celui-ci un cycle à 5 ou 6 chaînons comme décrit ci-dessus ;
R⁴ représente un groupe phényle, naphtyle, dihydro- ou tétrahydro-naphtyle qui peut porter un ou plusieurs des substituants suivants :
halogéno, nitro, cyano, hydroxy, alkyle en C₁ à C₄, halogénoalkyle en C₁ à C₄, alcoxy en C₁ à C₄, halogénoalcoxy en C₁ à C₄, phénoxy, phényle, alkylthio en C₁ à C₄, amino, alkylamino en C₁ à C₄, ou di(alkyle en C₁ à C₄)amino, 2 groupes placés sur des atomes de carbone voisins pouvant former avec ceux-ci un cycle à 5 ou 6 chaînons reliés par l'intermédiaire d'un groupe alkylène ou alkylidène, et dans lequel un ou plusieurs groupes méthylène ou méthylidène peuvent être remplacés par l'oxygène ;
R⁵ peut avoir les mêmes significations que R⁴ mats R⁴ et R⁵ peuvent avoir des significations identiques ou différentes ;
R⁶ représente l'hydrogène, un groupe alkyle en C₁ à C₈, alcényle en C₂ à C₈ ou alcynyle en C₃ à C₈, chacun d'eux pouvant porter un ou plusieurs substituants halogéno, nitro, cyano, alcoxy en C₁ à C₄, hydroxy, alkylthio en C₁ à C₄, mercapto, halogénoalcoxy en C₁ à C₄, carboxy, (alkyle en C₁ à C₄)carboxy, (alkyle en C₁ à C₄)carbonyle, amino, alkylamino en C₁ à C₄, di(alkyle en C₁ à C₄)amino ou phényle, ou bien un groupe naphtyle qui lui-même peut porter un ou plusieurs substituants halogéno, nitro, cyano, hydroxy, alcoxy en C₁ à C₄, alkyle en C₁ à C₄, halogénoalkyle en C₁ à C₄, halogénoalcoxy en C₁ à C₄, mercapto, alkylthio en C₁ à C₄, amino, alkylamino en C₁ à C₄, di(alkyle en C₁ à C₄)amino ou phénoxy ;
un groupe alkyle en C₁ à C₄ qui porte un substituant phénylméthoxy ou phénoxy, dans lequel le groupe phényle peut lui-même porter 1 ou 2 substituants halogéno, méthyle, ou méthoxy ;
une chaîne alkyle en C₁ à C₈, alcényle en C₃ à C₈ ou alcynyle en C₃ à C₈, qui porte l'un des substituants suivants :
hétéroaryle ou hétéroaryloxy, à 5 ou 6 chaînons, contenant 1 à 3 atomes d'azote et/ou un atome de soufre ou d'oxygène, et qui peut porter 1 à 4 atomes d'halogènes et/ou 1 à 2 des substituants suivants : alkyle en C₁ à C₄, halogénoalkyle en C₁ à C₄, alcoxy en C₁ à C₄, halogénoalcoxy en C₁ à C₄, alkylthio en C₁ à C₄, phényle, phénoxy ou phénylcarbonyle, les groupes phényle pouvant eux-mêmes porter 1 à 5 atomes d'halogènes et/ou 1 à 3 des substituants suivants : alkyle en C₁ à C₄, halogénoalkyle en C₁ à C₄, alcoxy en C₁ à C₄, halogénoalcoxy en C₁ à C₄ et/ou alkylthio en C₁ à C₄ ;
un groupe phényle, naphtyle, qui peut porter un ou plusieurs substituants halogéno, nitro, cyano, hydroxy, alcoxy en C₁ à C₄, alkyle en C₁ à C₄, halogénoalkyle en C₁ à C₄, halogénoalcoxy en C₁ à C₄, mercapto, alkylthio en C₁ à C₄, amino, alkylamino en C₁ à C₄, di(alkyle en C₁ à C₄)amino ou phénoxy ;
ou bien l'un des groupes suivants :

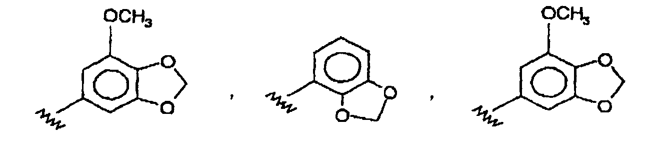

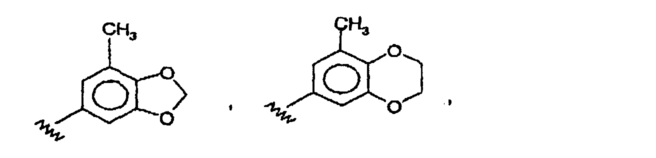

2. Dérivés d'acides α-hydroxycarboxyliques selon la revendication 1, **caractérisés en ce que** R=COOH.

3. Dérivés d'acides α-hydroxycarboxyliques selon l'une des revendications qui précèdent, **caractérisés en ce que** l'un au moins des symboles R⁴ et R⁵ représente un groupe phényle.

4. Dérivés d'acides α-hydroxycarboxyliques selon la revendication 3, **caractérisés en ce que** R⁴ et R⁵ représentent tous deux des groupes phényle.

5. Dérivés d'acides α-hydroxycarboxyliques selon l'une des revendications qui précèdent, **caractérisés en ce que** R⁶=alkyle en C₁ à C₈ éventuellement substitué par OH, alcoxy en C₁ à C₄ ou phényle lui-même éventuellement substitué .

6. Dérivés d'acides α-hydroxycarboxyliques selon l'une des revendications qui précèdent, **caractérisés en ce que** X=CH.

7. Dérivés d'acides α-hydroxycarboxyliques selon l'une des revendications qui précèdent, **caractérisés en ce que** l'un au moins des symboles W et Y représente l'azote (N).

8. Dérivés d'acides α-hydroxycarboxyliques selon l'une des revendications qui précèdent, **caractérisés en ce que** l'un au moins des symboles R² et R³ représente un groupe alkyle en C₁ à C₄.

9. Utilisation de composés selon les revendications 1 à 8 pour la préparation de médicaments prévus pour le traitement de l'hypertonie, l'hypertension pulmonaire, les troubles rénaux aigus et chroniques, l'insuffisance cardiaque chronique, l'ischémie cérébrale, la sténose récidivante après angioplastie, le cancer de la prostate.

10. Combinaison d'un composé selon les revendications 1 à 8 et d'un inhibiteur du système rénine-angiotensine (RAS).

11. Utilisation d'une combinaison d'un composé selon les revendications 1 à 8 et d'un inhibiteur du système rénine-angiotensine (RAS) pour la préparation de médicaments prévus pour le traitement de l'hypertonie, de l'hypertension pulmonaire, de l'infarctus du myocarde, de l'angine de poitrine, des troubles rénaux aigus, de l'insuffisance rénale, des vasospasmes cérébraux, de l'ischémie cérébrale, des saignements subarachnoïdaux, des migraines, de l'asthme, de l'athérosclérose, du choc endotoxique, des troubles d'organes induits par l'endotoxine, de la coagulation intravasculaire, de la sténose récidivante après angioplastie, de l'hyperplasie bénigne de la prostate, des troubles rénaux ou de l'hypertonie causés par ischémie et par intoxication, et des maladies cancéreuses, du cancer de la prostate et du cancer de la peau.
